(19) Europäisches Patentamt
European Patent Office
Office européen des brevets

(11) Veröffentlichungsnummer: **0 505 868 B1**

(12) **EUROPÄISCHE PATENTSCHRIFT**

(45) Veröffentlichungstag der Patentschrift: **13.09.95**

(51) Int. Cl.$^6$: **C07D 401/12**, C07D 211/46, C07D 207/08, A61K 31/445

(21) Anmeldenummer: **92104474.9**

(22) Anmeldetag: **16.03.92**

# (54) N-Acyl-alpha-aminosäurederivate.

(30) Priorität: **26.03.91 CH 910/91**
**22.01.92 CH 176/92**

(43) Veröffentlichungstag der Anmeldung:
**30.09.92 Patentblatt 92/40**

(45) Bekanntmachung des Hinweises auf die Patenterteilung:
**13.09.95 Patentblatt 95/37**

(84) Benannte Vertragsstaaten:
**AT BE CH DE DK ES FR GB GR IT LI LU MC NL PT SE**

(56) Entgegenhaltungen:
**EP-A- 0 372 486**
**EP-A- 0 381 033**
**EP-A- 0 445 796**

**CHEMICAL ABSTRACTS, vol. 92, no. 25, 23. Juni 1980, Columbus, Ohio, US; abstract no. 208785s, LABES D. ET AL. 'Free-Wilson analysis of the inhibitory effect of 4-substituted benzamidines on thrombin, plasmin and trypsin.' Seite 18 ;Spalte 1 ;**

(73) Patentinhaber: **F. HOFFMANN-LA ROCHE AG**
**Postfach 3255**
**CH-4002 Basel (CH)**

(72) Erfinder: **Alig, Leo**
**Liebrütistrasse 32**
**CH-4303 Kaiseraugst (CH)**
Erfinder: **Hadvary, Paul**
**Neumattenweg 8**
**CH-4105 Biel-Benken (CH)**
Erfinder: **Hürzeler, Marianne**
**Grod 12**
**CH-4658 Däniken (CH)**
Erfinder: **Müller, Marcel**
**Ouellenweg 10**
**CH-4402 Frenkendorf (CH)**
Erfinder: **Steiner, Beat**
**Im Brunnacker 18**
**CH-4112 Bättwil (CH)**
Erfinder: **Weller, Thomas**
**Münzgasse 3**
**CH-4051 Basel (CH)**

(74) Vertreter: **Mahé, Jean et al**
**Postfach 3255**
**Grenzacherstrasse 124**
**CH-4002 Basel (CH)**

EP 0 505 868 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue N-Acyl-α-aminosäurederivate, Verfahren zu deren Herstellung, pharmazeutische Präparate, die solche Verbindungen enthalten, sowie die Verwendung dieser Verbindungen bei der Herstellung pharmazeutischer Präparate.

Die Erfindung betrifft insbesondere N-Acyl-α-aminosäurederivate der Formel

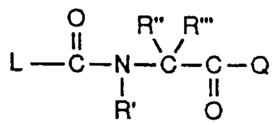

I

worin

L eine Gruppe der Formel

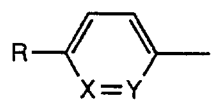

$L^1$

oder

$R^o$-NH$(CH_2)_t$ $L^2$

R Amidino oder Guanidino,

eins von X und Y CH und das andere CH oder N,

$R^o$ Wasserstoff oder Amidino,

t eine ganze Zahl von 2 bis 6,

R', R'' und R''' Wasserstoff oder in α-Aminocarbonsäuren übliche N-Substituenten bzw. Seitenketten sind, wobei in R', R'' und R''' vorkommende Hydroxy- bzw. Carboxygruppen veräthert bzw. verestert oder amidiert, und vorkommende Aminogruppen $C_{1-6}$-alkanoyliert oder aroyliert, sein können,

Q eine Gruppe der Formel

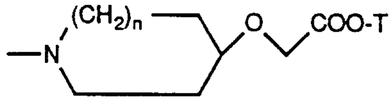

Q[1]

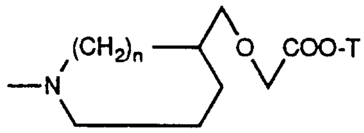

Q[2]

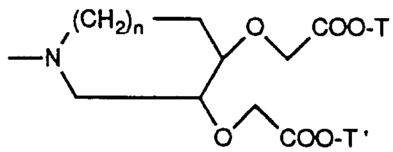

Q[3]

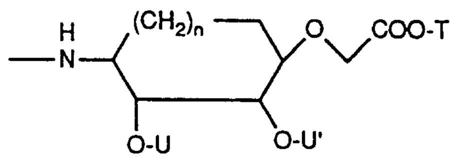

Q[4]

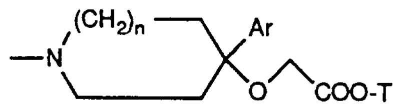

Q[5]

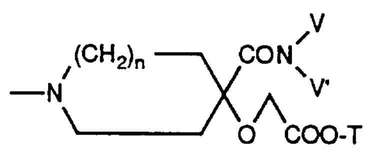

Q[6]

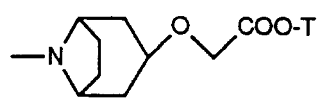

Q[7]

oder

$-N(V')(CH_2)_v-(V'',V''')CH_2OCH_2COO-T$ Q8

ist oder, falls R' und R'' zusammen mit dem N- und C-Atom an das sie gebunden sind, einen Ring bilden, auch eine Gruppe der Formel

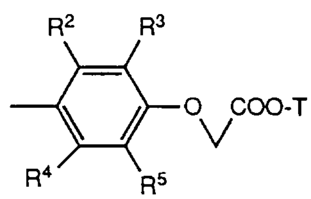

Q[9]

sein kann,

n — die Zahl 0 oder 1,

v — eine ganze Zahl von 0 bis 3,

T und T' — Wasserstoff oder unter physiologischen Bedingungen spaltbares nieder-Alkyl oder Phenyl-nieder-alkyl,

| | |
|---|---|
| V bis V''' | Wasserstoff oder nieder-Alkyl, |
| U und U' | Wasserstoff, $C_{1-6}$-Alkanoyl oder Aroyl, |
| Ar | Aryl, und |
| $R^2$ bis $R^5$ | Wasserstoff, nieder-Alkyl, nieder-Alkoxy, Halogen oder eine Gruppe $-OCH_2COO-T'$ sind, oder |
| $R^2$ und $R^3$ | zusammen mit der Phenylgruppe, an die sie gebunden sind, eine 1-Naphthylgruppe bilden, |

sowie Hydrate oder Solvate und physiologisch verwendbare Salze davon.

Im Rahmen der vorliegenden Erfindung bezeichnet Me Methyl, Ac Acetyl, tBu t-Butyl, Boc t-Butoxycarbonyl, Z Benzyloxycarbonyl, Fmoc 9-Fluorenylmethoxycarbonyl, Val L-Valyl, Phe L-Phenylalanyl, Ser L-Seryl, Gly Glycyl, Ala L-Alanyl, Asp L-α-Aspartyl, Leu L-Leucyl, Tyr L-Tyrosyl, Sar Sarcosyl, Orn L-Ornithyl, Lys L-Lysyl, Phg L-α-Phenylglycyl, Pro L-Prolyl, Glu L-Glutamyl, Trp L-Tryptophan.

Der Ausdruck "nieder" bezeichnet Gruppen mit 1-6, vorzugsweise 1-4, C-Atomen. Beispiele von nieder-Alkylgruppen sind Methyl, Aethyl, Propyl, Isopropyl, n-, s- oder t-Butyl und Hexyl. Beispiele von unter physiologischen Bedingungen spaltbaren nieder-Alkylgruppen sind primäre und sekundäre nieder-Alkylgruppen.

Die Symbole R', R" und R''' in dem α-Aminocarbonsäurerest -N(R')C(R",R''')CO- stellen Wasserstoff oder in offenkettigen oder cyclischen, natürlichen oder synthetischen α-Aminocarbonsäuren übliche N-Substituenten oder Seitenketten dar. Beispiele von solchen N-Substituenten R' und Seitenketten R" und R''' sind gegebenenfalls durch OH, COOH, $NH_2$ oder Aryl, insbesondere durch Phenyl, Hydroxyphenyl, Hydroxyjodphenyl oder Hydroxydijodphenyl, substituiertes nieder-Alkyl. Zwei auf diese Weise gegebenenfalls substituierte nieder-Alkylgruppen R' und R" können zusammen mit dem N- bzw. C-Atom, mit dem sie verbunden sind, einen 4- bis 6-gliedrigen, insbesondere einen 5-gliedrigen Ring bilden. In den N-Substituenten R' und Seitenketten R" und R''' vorkommende Hydroxy bzw. Carboxygruppen können veräthert bzw. verestert oder amidiert und vorkommende Aminogruppen $C_{1-6}$-alkanoyliert oder aroyliert sein. Beispiele von solchen Aether-, Ester- bzw. Amidgruppen sind $-O-T^o$, $-COO-T^o$ bzw. -CON(V, V'), worin V und V' obige Bedeutung haben und $T^o$ nieder-Alkyl, insbesondere Methyl, Hexyl und tBu, oder Aralkyl, insbesondere Benzyl ist.

Beispiele von offenkettigen α-Aminocarbonsäuren sind H-Gly-OH, H-Ala-OH, H-Orn-OH und H-Tyr-OH; Beispiele von cyclischen α-Aminocarbonsäuren, d.h. solche worin R' und R" zusammen mit dem N- bzw. dem C-Atom, an dem sie gebunden sind, einen Ring bilden, sind H-Pro-OH, H-Pro(4-OH)-OH und 2-Piperidincarbonsäure.

Beispiele von $C_{1-6}$-Alkanoylgruppen U und U' sind Formyl, Acetyl und Propionyl. Aryl bezeichnet Phenyl gegebenenfalls mit bis zu 3 Substituenten, wie Alkyl, OH, nieder-Alkoxy, Halogen oder Halo-nieder-alkyl, insbesondere $CF_3$. Aroyl bezeichnet die entsprechenden Benzoylgruppen.

Die Verbindungen der Formel I können solvatisiert, insbesondere hydratisiert sein. Die Hydratisierung kann im Zuge des Herstellungsverfahrens erfolgen oder allmählich als Folge hygroskopischer Eigenschaften einer zunächst wasserfreien Verbindung der Formel I auftreten.

Beispiele von physiologisch verwendbaren Salzen der Verbindungen der Formel I sind Salze mit physiologisch verträglichen Mineralsäuren, wie Salzsäure, Schwefelsäure oder Phosphorsäure; oder mit organischen Säuren, wie Methansulfonsäure, Essigsäure, Trifluoressigsäure, Zitronensäure, Fumarsäure, Maleinsäure, Weinsäure, Bernsteinsäure oder Salicylsäure. Die Verbindungen der Formel I mit freier Carboxygruppe können auch Salze mit physiologisch verträglichen Basen bilden. Beispiele solcher Salze sind Alkalimetall-, Erdalkalimetall-, Ammonium- und Alkylammoniumsalze, wie das Na-, K-, Ca- oder Tetramethylammoniumsalz. Die Verbindungen der Formel I können auch in Form von Zwitterionen vorliegen.

Die Verbindungen der Formel I, die ein oder mehrere asymmetrische C-Atome enthalten, können als Enantiomere, als Diastereomere oder als Gemische davon, z.B. als Racemate, vorliegen.

Die in der EP-A-372486 beschriebenen Verbindungen enthalten keine der vorliegenden Gruppe Q entsprechende Gruppe. Die eine substituierte Phenylgruppe $Q^9$ enthaltenden Verbindungen unterscheiden sich von den in der EP-A-381033 beschriebenen Verbindungen dadurch, dass in ersteren R' und R" zusammen mit dem N- und C-Atom, an das sie gebunden sind, einen Ring bilden.

In der Formel I ist R vorzugsweise Amidino, X CH, Y CH oder N, und Q eine Gruppe $Q^1$, $Q^2$, $Q^4$, $Q^5$ oder $Q^9$.

In den Verbindungen der Formel I, worin Q = $Q^1$ ist, ist n vorzugsweise 1, T Wasserstoff oder Methyl, und -N(R')C(R",R''')CO- einer der Reste Gly, Ala, D-Ala, Val, Leu, Sar, Orn, Lys, Phg, 2-Methyl-Pro, Phe, Tyr, 3-Jod-Tyr, 3,5-Dijod-TH, Ser(Ac), Ser, Asp, Glu, Pro, 4-Benzyloxy-Pro, 4-Hydroxy-Pro, 2-Piperidylencarbonyl, $NHCH(CH_2CH_2NH_2)CO$, Trp, Tyr(Me), Tyr(Hexyl), Tyr(O-tBu), $O,N(Me)_2$-Tyr und $N(MeOCH_2CH_2)$-

Gly.

Bevorzugte Verbindungen mit Q = $Q^2$, $Q^4$ oder $Q^5$ sind diejenigen mit n = 1, T = H; U und U' = H oder Ac; Ar = α,α,α-Trifluor-m-tolyl, und -N(R')C(R",R"')CO- = Ala.

Falls Q = $Q^9$ ist, sind $R^2$ bis $R^5$ vorzugsweise H, oder $R^2$ Carboxymethoxy oder Methoxycarbonylmethoxy, T = H oder $CH_3$, und -N(R')C(R",R"')CO- = Pro.

Beispiele von bevorzugten Verbindungen sind die aus der Gruppe von:
[[1-[N-(p-Amidinobenzoyl)-L-alanyl]-4-piperidinyl]oxy]essigsäure,
[[1-[N-[(5-Amidino-2-pyridyl)carbonyl]-L-alanyl]-4-piperidinyl]oxy]essigsäure,
[[1-[N-(p-Amidinobenzoyl)-3-(4-hydroxy-3-jodphenyl)-L-alanyl]-4-piperidinyl]oxy]essigsäure,
[[1-[3-Acetoxy-N-(p-amidinobenzoyl)-L-alanyl]-4-piperidinyl]oxy]essigsäure,
[p-[[1-(p-Amidinobenzoyl)-2-pyrrolidinyl]carbonyl]phenoxy]essigsäure,
[[1-[N-[(5-Amidino-2-pyridyl)carbonyl]-L-tyrosyl]-4-piperidinyl]oxy]essigsäure und insbesondere
[[1-[N-(p-Amidinobenzoyl)-L-tyrosyl]-4-piperidinyl]oxy]essigsäure.

Weitere bevorzugte Verbindungen der Formel I sind diejenigen worin Q eine Gruppe $Q^3$, insbesondere worin n = 0 und T Wasserstoff, oder eine Gruppe $Q^7$, insbesondere worin T Wasserstoff ist,
sowie diejenigen, worin Q eine Gruppe $Q^8$, insbesondere worin v = 1, T Wasserstoff oder Butyl und V' bis V"' Wasserstoff sind.

Beispiele von solchen Verbindungen sind:
(S)-1-[2-(5-Amidinopyridin-2-ylcarbonylamino)-3-(4-methoxyphenyl)propionyl]piperidin-4-yloxyessigsäure,
(S)-1-[2-(4-Amidinobenzamido)-3-(4-methoxyphenyl)propionyl]piperidin-4-yloxyessigsäureäthylester,
(S)-1-[2-(4-Amidinobenzamido)-3-(4-methoxyphenyl)propionyl]piperidin-4-yloxyessigsäure,
[1-[N-(4-Amidinobenzoyl)-4'-hexyloxy-L-phenylalanyl]piperidin-4-yloxy]essigsäure.

Die obigen N-Acyl-α-aminosäuren lassen sich erfindungsgemäss dadurch herstellen, dass man

a) in einer Verbindung der Formel

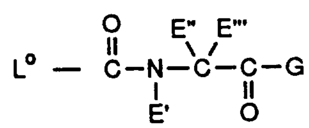

II

worin

$L^o$ eine Gruppe der Formel

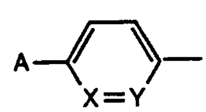

$L^{o1}$

oder

$R^{o1}$-$(CH_2)_t$ $L^{o2}$

ist, in der A eine gegebenenfalls geschützte Amidino- oder Guanidinogruppe,

$R^{o1}$ eine gegebenenfalls geschützte Amino- oder Guanidinogruppe,

E', E", E"' und G die gleiche Bedeutung wie R', R", R"' bzw. Q in der Formel I haben, wobei, falls $R^{o1}$ Amino oder Guanidino, oder falls A Amidino oder Guanidino ist, zumindest eins von E', E", E"' und G zumindest eine Carbonsäureestergruppe und/oder Aethergruppe und/oder geschützte Aminogruppe enthält,

eine Aethergruppe bzw. eine geschützte Amino-, Amidino- bzw. Guanidinogruppe bzw. einen Carbonsäureester spaltet, oder

b) in einem Nitril der Formel

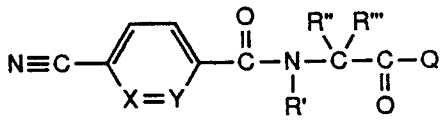

III

die Cyangruppe in die Amidinogruppe überführt, oder

c) ein Amin der Formel

R'-NHC(R'',R''')CO-Q IV

mit einer Säure der Formel $L^1$-COOH oder einem reaktionsfähigen Derivat davon umsetzt, und

d) gewünsthtenfalls eine in einer Verbindung der Formel I enthaltene reaktionsfähige Gruppe funktionell abwandelt, und

e) gewünschtenfalls eine Verbindung der Formel I in ein physiologisch verträgliches Salz überführt oder ein Salz einer Verbindung der Formel I in die freie Säure oder Base überführt.

Beispiele von spaltbaren Carbonsäureestergruppen sind Benzyl-OCO- und nieder-Alky-OCO, wie tBu-OCO-. Beispiele von spaltbaren geschützten Amino-, Amidino- und Guanidinogruppen sind -NH-Z, -NH-Boc und $-N_3$; -C(NH)NH-Z, -C(NH)NH-Boc, C(N-Boc)N$(Boc)_2$ und -C(N-Boc)NH-Boc; -NHC(NH)$NHNO_2$ und -NHC(N-Boc)NH-Boc. Ein Beispiel einer spaltbaren Aethergruppe ist tBu-O-.

Estergruppen lassen sich in an sich bekannter Weise, beispielsweise mit einer Base, wie einem Alkalimetallhydroxid, z.B. Natriumhydroxid, in einem Lösungsmittel, wie Methanol oder Wasser; oder mit einer Säure, wie Salzsäure, hydrolysieren. Benzylester können durch Hydrierung in Gegenwart eines Edelmetallkatalysators, wie Palladium auf Aktivkohle (Pd/C) in einem Lösungsmittel, wie Methanol, Aethanol, Ameisensäure oder Essigsäure, bei einer Temperatur bis zu etwa 40 °C, vorzugsweise bei Raumtemperatur gespalten werden. Dabei wird gleichzeitig eine in der Gruppe A enthaltene Amidinoschutzgruppe, wie Z, abgespalten.

Estergruppen, wie tBu-OCO-, sowie Amino- bzw. Amidinoschutzgruppen, wie Boc, und Aethergruppen, wie tBu-O-, lassen sich z.B. mit einer Säure, wie Ameisensäure oder Trifluoressigsäure, gewünschtenfalls in einem Lösungsmittel, wie Dichlormethan, bzw. mit HCl gesättigtem Eisessig, bei einer Temperatur bis zu 40 °C, vorzugsweise bei Raumtemperatur spalten.

Die Variante b) kann man dadurch bewerkstelligen, dass man ein Nitril III durch Umsetzung mit Schwefelwasserstoff und Triäthylamin in Pyridin zum Thioamid, dieses durch Methylierung mit Methyljodid in Aceton und anschliessende Ammonolyse mit Ammoniumacetat in Methanol in eine Verbindung I überführt.

Die Kupplung c) des Amins IV mit der Säure $L^1$-COOH oder einem reaktionsfähigen Derivat davon, wie dem Säurechlorid, wird in Gegenwart einer Base, wie Picolin, in einem Lösungsmittel, wie Dichlormethan, bei einer Temperatur bis zu 40 °C, vorzugsweise bei Raumtemperatur durchgeführt.

Als funktionelle Abwandlungen von reaktionsfähigen Gruppen nach der Verfahrensvariante d) sind die Spaltung von in der Gruppe Q enthaltenen nieder-Alkoxycarbonylgruppen -COO-T oder -COO-T', oder von $C_{1-6}$-Alkanoyloxy- oder Aroyloxygruppen O-U oder O-U', oder die Veresterung einer Carboxygruppe in einer Säure I, und die Halogenierung, insbesondere die Jodierung einer in einer Seitenkette R'' oder R''' enthaltenen Arylgruppe zu nennen.

So lassen sich in der Gruppe Q enthaltene Butoxycarbonyl- bzw. Methoxycarbonylgruppen mit einer Säure, wie wässriger Salzsäure bzw. Essigsäure, oder unter basischen Bedingungen, z.B. mit wässriger Natronlauge in Methanol, Acetoxygruppen mit Kaliumcarbonat in Methanol verseifen. Die Veresterung einer Carboxygruppe wird z.B. durch Reaktion der Säure mit dem geeigneten Alkohol in Gegenwart von katalytischen Mengen $H_2SO_4$ bewerkstelligt.

Die Jodierung einer Arylgruppe, insbesondere der Hydroxyphenylgruppe in einer Seitenkette R'' oder R''' kann man durch Reaktion der Verbindung I mit Chloramin T gefolgt von Natriumjodid in Wasser/DMF durchführen.

Ein Amin I, worin L eine Gruppe $H_2N(CH_2)_t$ ist, wird in das entsprechende Guanidin I umgewandelt, worin L für $HN=C(NH_2)NH(CH_2)_t$ steht, indem man das Amin mit 2-S-Isothioharnstoffäthansulfonat in Gegenwart einer Base, wie $Na_2CO_3$ oder NaOH bei Temperaturen bis zu 40 °C umsetzt.

Die Verbindungen der Formeln II und III sind neu und als solche ebenfalls Gegenstand der vorliegenden Erfindung. Sie werden in an sich bekannter Weise hergestellt.

So wird eine Verbindung II, worin $L^{o}$ für eine Arylgruppe $L^{01}$ steht, durch Umsetzung eines Amins der Formel

E'-NHC(E'',E''')CO-G'     V

worin G' für eine der Gruppen $Q^1$ bis $Q^9$ steht, in der die Gruppe -COO-T und eine gegebenenfalls vorhandene Gruppe -COO-T' als Carbonsäureestergruppen vorliegen,
mit einer Säure der Formel

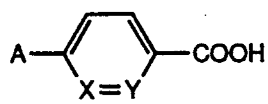

VI

oder einem reaktionsfähigen Derivat davon, z.B. dem Säurechlorid, hergestellt.

Diese Umsetzung kann man gegebenenfalls in Gegenwart von Tetra-n-buyylammoniumhydrogensulfat, in einem Lösungsmittel, wie Dichlormethan, und einer Base wie wässrigem Natriumbicarbonat durchführen.

Man kann ein Amin $H\text{-}Q^{o}$, worin $Q^{o}$ für eine der Aminogruppen $Q^1$ bis $Q^8$ steht, in der die Gruppe -COO-T und eine gegebenenfalls vorhandene Gruppe -COO-T als Carbonsäureestergruppen vorliegen, mit einer Säure der Formel

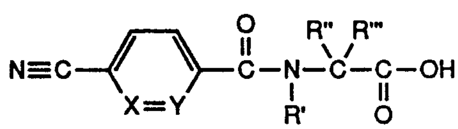

VII

zum Nitril III umsetzen.

Diese Reaktion kann man in Gegenwart von 2-(1H-Benzotriazol-1-yl)-1,1,3,3-tetramethyluronium-hexafluorphosphat (HBTU) und einer organischen Base, wie N-Methylmorpholin, in einem Lösungsmittel, wie DMF, durchführen.

Eine Verbindung II, worin A Amidino ist, kann man durch Umwandlung der Cyangruppe in die Amidinogruppe in dem der Verbindung II entsprechenden Nitril erhalten. Letzteres kann durch Kupplung eines Amins der obigen Formel V mit einer Säure der Formel

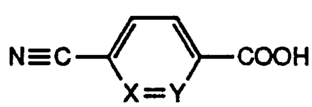

VIII

oder mit einem funktionellen Derivat davon, z.B. dem Säurechlorid, hergestellt werden.

Diese Kupplung lässt sich in Gegenwart von 2-Chlor-4,6-dimethoxy-1,3,5-triazin (CDMT) und einer Base, wie N-Methylmorpholin, in einem Lösungsmittel, wie Dichlormethan, durchführen.

Eine Verbindung II, worin $L^{o}$ für eine Gruppe $L^{02}$ mit geschützter Amino- oder Guanidinogruppe $R^{o1}$ steht, wird durch Kupplung eines Amins V mit einer Säure der Formel $R^{o1}\text{-}(CH_2)_t\text{-}COOH$ bewerkstelligt, z.B. in Gegenwart von HBTU und N-Methylmorpholin.

Ein Nitril III, worin Q eine Gruppe $Q^9$ ist, lässt sich z.B. wie folgt herstellen:

Ein Amin der Formel

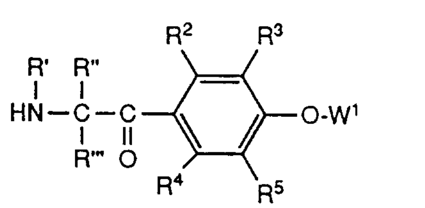

IX

worin R' und R'' zusammen mit dem N- und C-Atom einen Cyclus bilden, und $W^1$ eine Schutzgruppe ist,
wird mit einer Säure VIII oder einem funktionellen Derivat davon umgesetzt, die Schutzgruppe abgespalten und das entstandene Phenol mit einem Bromessigsäurederivat $BrCH_2COO$-T umgesetzt.

Die Reaktion des Amins IX mit dem der Säure VIII entsprechenden Säurechlorid kann man in Gegenwart einer Base, wie Triäthylamin, in DMF durchführen. Die Abspaltung einer Schutzgruppe $W^1$, z.B. Benzyl, lässt sich durch Hydrogenolyse über Pd/C in Aethanol, und die Reaktion des obigen Phenols mit dem Bromessigsäurederivat in DMF in Gegenwart von Kaliumcarbonat bewerkstelligen.

Die Amine IV und V lassen sich z.B. durch Kupplung einer N-geschützten Aminosäure der Formel

$W^2$-N(E')C(E'',E''')COOH X

mit einem Amin H-$Q^o$ und Entfernung der Schutzgruppe $W^2$, z.B. Z oder Boc, im Kupplungsprodukt herstellen.

Die Säuren VII kann man durch Kupplung eines funktionellen Derivats der Säure VIII, z.B. dem Säurechlorid, mit einem Amin

R'-NHC(R'',R''')COO-nieder-Alkyl X'

und Spaltung der Estergruppe im Kupplungsprodukt herstellen. Diese Kupplung kann man z.B. in Dichlormethan in Gegenwart von Triäthylamin bewerkstelligen. Die Niederalkylgruppe, z.B. Methyl, lässt sich mit wässrigem LiOH in Methanol entfernen.

Man kann auch eine Aminosäure der Formel

R'-NHC(R'',R''')COOH X''

z.B. Glycin, direkt mit dem der Säure VIII entsprechenden Säurechlorid in Gegenwart von wässrigem Natriumbicarbonat, gegebenenfalls in Gegenwart von Tetramethylammoniumsulfat in Dichlormethan zu einer Säure VII umsetzen.

Ein Amin IX lässt sich dadurch herstellen, dass man das Grignard-Reagenz eines Bromids der Formel

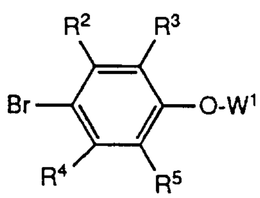

XI

mit einer Verbindung der Formel

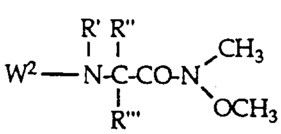

XII

umsetzt und die Aminoschutzgruppe $W^2$ aus dem Reaktionsprodukt entfernt.

Die weiter oben verwendeten Amine $HQ^o$, worin $Q^o$ für eine der Aminogruppen $Q^1$ bis $Q^8$ steht, in der die Gruppe -COO-T und eine gegebenenfalls vorhandene Gruppe -COO-T' als Carbonsäureestergruppen vorliegen, kann man wie in den nachstehenden Beispielen 1a)b)c), 2a), 46a)b), 47a) und 48a)b) beschrieben herstellen.

Die Verbindungen der Formel I, ihre Solvate und ihre Salze hemmen sowohl die Bindung von Fibrinogen, Fibronectin und des Willebrand-Faktors an den Fibrinogenrezeptor der Blutplättchen (Glykoprotein IIb/IIIa) als auch die Bindung derselben und weiterer adhäsiver Proteine, wie Vitronectin, Kollagen und Laminin, an die entsprechenden Rezeptoren auf der Oberfläche verschiedener Zelltypen. Die besagten Verbindungen beeinflussen somit Zell-Zell- und Zell-Matrix-Wechselwirkungen. Sie verhindern insbesondere

die Entstehung von Blutplättchenthromben und können bei der Bekämpfung bzw. Verhütung von Krankheiten, wie Thrombose, Hirnschlag, Herzinfarkt, Entzündung und Arteriosklerose verwendet werden. Ferner haben diese Verbindungen einen Effekt auf Tumorzellen, indem sie deren Metastasierung hemmen. Somit können sie auch als Antitumor-Mittel eingesetzt werden. Weiter können sie die Wundheilung beschleunigen. Da sie auch den Knochenabbau verhindern, kann man sie bei der Behandlung der Osteoporose verwenden.

Die Hemmung der Fibrinogenbindung an den Fibrinogenrezeptor, Glykoprotein IIb/IIIa, kann wie folgt nachgewiesen werden:

Das Glykoprotein IIb/IIIa wird aus Triton X-100 Extrakten von menschlichen Blutplättchen gewonnen und durch Lectin-Affinitätschromatographie (Analytical Biochemistry 151, 1985, 169-177) und Chromatographie an einer Arg-Gly-Asp-Ser-Affinitätssäule (Science 231, 1986, 1559-62) gereinigt. Das so erhaltene Rezeptorprotein wird an Mikrotiterplatten gebunden. Die spezifische Bindung von Fibrinogen an den immobilisierten Rezeptor wird mit Hilfe eines ELISA-Systems ("enzyme-linked immunosorbent assay") bestimmt. Die nachstehenden $IC_{50}$-Werte entsprechen derjenigen Konzentration der Testsubstanz, welche benötigt wird, um die Bindung von Fibrinogen an den immobilisierten Rezeptor um 50% zu hemmen:

| Produkt von Beispiel | 1 | 3 | 4 | 5 | 6 | 7 | 8 | 9 |
|---|---|---|---|---|---|---|---|---|
| $IC_{50}$ (µM) | 0,01 | 0,0017 | 0,14 | 0,001 | 0,027 | 0,033 | 0,008 | 0,08 |

| Produkt von Beispiel | 10 | 13 | 14 | 15 | 16 | 18 | 21 | 22 |
|---|---|---|---|---|---|---|---|---|
| $IC_{50}$ (µM) | 0,017 | 0,001 | 0,018 | 0,053 | 0,002 | 0,0017 | 0,16 | 0,47 |

| Produkt von Beispiel | 24 | 27 | 30 | 37 | 39 | 40 | 41 |
|---|---|---|---|---|---|---|---|
| $IC_{50}$ (µM) | 0,026 | 0,008 | 0,015 | 0,0003 | 0,0008 | 0,05 | 0,0007 |

| Produkt von Beispiel | 42 | 43 | 44 |
|---|---|---|---|
| $IC_{50}$ (µM) | 0,007 | 0,0016 | 0,01 |

Diese Verbindungen haben geringe Toxizität, liegt doch die $LD_{50}$ der Produkte der Beispiele 3 und 14 bei 250 und des Beispiels 5 bei 500 mg/kg i.v. an der Maus.

Wie eingangs erwähnt, sind Arzneimittel, enthaltend eine Verbindung der Formel I, ein Solvat davon oder ein Salz davon, ebenfalls Gegenstand der vorliegenden Erfindung, weiterhin auch ein Verfahren zur Herstellung derartiger Arzneimittel, welches dadurch gekennzeichnet ist, dass man eine oder mehrere der besagten Verbindungen und erwünschtenfalls einen oder mehrere andere therapeutisch wertvolle Stoffe in eine galenische Darreichungsform bringt. Die Arzneimittel können enteral, z.B. oral in Form von Tabletten, Lacktabletten, Dragées, Hart- und Weichgelatinekapseln, Lösungen, Emulsionen oder Suspensionen, oder rektal, z.B. in Form von Suppositorien; oder als Spray verabreicht werden. Die Verabreichung kann aber auch parenteral, z.B. in Form von Injektionslösungen oder als Infusion, erfolgen.

Zur Herstellung von Tabletten, Lacktabletten, Dragées und Hartgelatinekapseln kann der Wirkstoff mit pharmazeutisch inerten, anorganischen oder organischen Excipientien vermischt werden. Als solche Excipientien kann man für Tabletten, Dragées und Hartgelatinekapseln z.B. Lactose, Maisstärke oder Derivate davon, Talk, Stearinsäure oder deren Salze, verwenden. Für Weichgelatinekapseln eignen sich als Excipientien z.B. vegetabile Oele, Wachse, Fette, halbfeste und flüssige Polyole; je nach Beschaffenheit des Wirkstoffes sind jedoch bei Weichgelatinekapseln überhaupt keine Excipientien erforderlich. Zur Herstellung von Lösungen und Sirupen eignen sich als Excipientien z.B. Wasser, Polyole, Saccharose, Invertzucker und Glukose; für Injektionslösungen eignen sich z.B. Wasser, Alkohole, Polyole, Glycerin und vegetabile Oele, und für Suppositorien eignen sich z.B. natürliche oder gehärtete Oele, Wachse, Fette und halbflüssige oder flüssige Polyole. Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten.

Für die Bekämpfung bzw. Verhütung der weiter oben genannten Krankheiten kann die Dosierung des Wirkstoffes innerhalb weiter Grenzen variieren und ist natürlich in jedem einzelnen Fall den individuellen Gegebenheiten anzupassen. Im allgemeinen dürfte bei oraler Verabreichung eine Dosis von etwa 0,1 bis 20 mg/kg, vorzugsweise von etwa 0,5 bis 4 mg/kg, pro Tag für den Erwachsenen angemessen sein, wobei aber die soeben angegebene obere Grenze auch überschritten werden kann, wenn sich dies als angezeigt erweisen sollte.

Beispiel 1

Eine Lösung von 2,43 g [[1-[N-(p-Amidinobenzoyl)glycyl]-4-piperidinyl]oxy]essigsäure-t-butylester in 15 ml Dichlormethan/Trifluoressigsäure 1:1 wird 5 Stunden bei Raumtemperatur stehengelassen. Nach Abdampfen der Lösungsmittel und Chromatographie [silyliertes Kieselgel (LiChroprep RP-18), Methanol/Wasser-Gradient] erhält man 0,46 g des Trifluoracetats von [[1-[N-(p-Amidinohenzoyl)glycyl]-4-piperidinyl]oxy]essigsäure,Smp. 233-236 °C. MS (FAB): 363 $(M+H)^+$.

Das Ausgangsmaterial kann man wie folgt herstellen:

a) Zu einer Lösung von 50 g 4-Hydroxypiperidin in 500 ml Dichlormethan gibt man nacheinander bei 0 °C 69,1 ml Triäthylamin und 70,2 ml Chlorameisensäurebenzylester. Die entstandene Suspension wird über Nacht bei Raumtemperatur gerührt und anschliessend filtriert. Der nach Einengen des Filtrats anfallende Rückstand wird in Essigester aufgenommen, mit Wasser und 1N Salzsäure gewaschen, getrocknet und eingeengt. Man erhält 73,6 g N-Benzyloxycarbonyl-4-hydroxypiperidin, $R_f$ = 0,56 (Essigester/Methanol 9:1), MS (EI): 235 $(M^+)$.

b) Zu einer Lösung von 30,1 g N-Benzyloxycarbonyl-4-hydroxypiperidin in 300 ml Toluol gibt man 28 ml Bromessigsäure-t-butylester und 1,4 g Tetra-n-butylammoniumhydrogensulfat in 10 ml Wasser. Danach tropft man eine Lösung von 125 g Natriumhydroxid in 125 ml Wasser zu. Nach Rühren über Nacht werden die organischen Extrakte abgetrennt, getrocknet und eingeengt. Nach Trocknen erhält man 34,1 g N-Benzyloxycarbonyl-4-[(t-butoxycarbonyl)methoxy]-piperidin, $R_f$ = 0,76 (Essigester). MS (EI): 293 $(M-C_4H_8)^+$.

c) Zu einer Lösung von 30 g des Produkts von b) in 50 ml Aethanol gibt man 1,5 g Pd/C (10%). Das Reaktionsgemisch wird bei Raumtemperatur hydriert. Danach wird vom Katalysator abfiltriert, mit Aethanol gewaschen und das Filtrat eingeengt. Man erhält 17,4 g 4-Piperidinyloxyessigsäure-t-butylester, $R_f$ = 0,14 (Essigester/Methanol 1:1). MS (EI): 215 $(M^+)$.

d) Durch Kupplung von 5,8 g (mit 5,4 g CDMT aktiviertem) Z-Glycin mit 6,0 g 4-Piperidinyloxyessigsäure-t-butylester und 6,3 ml N-Methylmorpholin in Dichlormethan erhält man 10 g Benzyl-[[[4-[(t-butoxycarbonyl)methoxy]piperidinyl]carbonyl]methyl]carbamat. MS (EI): 406 $(M^+)$.

e) Durch Hydrogenolyse einer Lösung von 10 g des Produkts von d) in 200 ml Aethanol in Gegenwart von 0,7 g Pd/C (10%) und 1,4 ml Essigsäure isoliert man nach Chromatographie an Kieselgel mit Essigester/Methanol 1:1 4,1 g 1-[1-Glycyl-4-piperidinyl)oxy]essigsäure-t-butylester, MS (EI): 273 $(M+H)^+$. IR: 1746 $cm^{-1}$.

f) Zu einem Gemisch von 4,1 g des Produkts von e) und 0,03 g Tetra-n-butylammoniumhydrogensulfat in 210 ml Dichlormethan/gesättigte Natriumhydrogencarbonatlösung 4:3 gibt man bei Raumtemperatur 2,95 g p-Amidinobenzoylchlorid-hydrochlorid (hergestellt aus p-Amidinobenzoesäure durch Umsetzung mit Thionylchlorid in THF in Gegenwart von DMF). Nach Rühren über Nacht wird mit Dichlormethan und Wasser verdünnt, durch Zugabe von 1N Natronlauge auf pH 9-10 eingestellt, die organischen Extrakte werden abgetrennt, getrocknet und eingeengt. Nach Trocknen erhält man 2,43 g des erwünschten Ausgangsmaterials, MS (FAB): 419 $(M+H)^+$.

Beispiel 2

A) Eine Lösung von 1,5 g [[1-[N-(p-Cyanbenzoyl)glycyl]-4-piperidinyl]oxy]essigsäuremethylester in 215 ml Pyridin/Triäthylamin 40:3 wird mit Schwefelwasserstoff gesättigt und 24 Stunden bei Raumtemperatur belassen. Nach Entfernen der Lösungsmittel wird der Rückstand in Essigester aufgenommen und mit gesättigter Natriumchloridlösung gewaschen. Die organischen Extrakte werden getrocknet und eingeengt. Nach Chromatographie des Rückstandes an Kieselgel mit Essigester gefolgt von Essigester/Methanol isoliert man 1,34 g [[1-[N-[p-(Thiocarbamoyl)benzoyl]glycyl]-4-piperidinyl]oxy]-essigsäuremethylester, MS (FAB): 394 $(M+H)^+$.

B) Die Umsetzung von 1,25 g der Vorstufe mit 7,5 ml Methyljodid in 150 ml Aceton bei Siedetemperatur ergibt nach Filtration und Entfernen des Lösungsmittels 1,65 g [[1-[N-(p-[1-(Methylthio)formimidoyl]-benzoyl]glycyl]-4-piperidinyl]oxy]essigsäuremethylester-hydrojodid, MS (FAB): 408 $(M+H)^+$.

C) Durch Ammonolyse von 1,5 g des Produkts von B) in Gegenwart von 0,32 g Ammoniumacetat in 100 ml Methanol bei Siedetemperatur erhält man 0,76 g [[1-[N-(p-Amidinobenzoyl)glycyl]-4-piperidinyl]oxy]-essigsäuremethylester-hydrojodid. Smp. 103-105 °C. MS (FAB): 377 $(M+H)^+$.

Das Ausgangsnitril kann man wie folgt herstellen:

a) Durch Veresterung des Trifluoracetats von 4-Piperidinyloxyessigsäure (hergestellt durch Behandlung des Produktes von Beispiel 1c) mit Trifluoressigsäure in Dichlormethan) in Methanol in Gegenwart von Thionylchlorid erhält man 4-Piperidinyloxyessigsäuremethylester-hydrochlorid, MS (EI): 173 $(M)^+$.

b) Die Kupplung von 1,35 g des Produkts von a) mit 1,18 g N-(p-Cyanbenzoyl)glycin (hergestellt durch Umsetzung von Glycin mit p-Cyanbenzoylchlorid in gesättigter Natriumhydrogencarbonatlösung) in Gegenwart von HBTU und N-Methylmorpholin in DMF liefert nach Chromatographie an Kieselgel (Essigester/Methanol 9:1 bis 1:1) 1,66 g des erwünschten Ausgangsnitrils, MS (EI): 359 $(M)^+$.

Beispiel 3

Aus 13 g [[1-[N-(p-Amidinobenzoyl)-L-alanyl]-4-piperidinyl]oxy]essigsäure-t-butylester erhält man durch Behandlung mit Trifluoressigsäure in Dichlormethan (wie in Beispiel 1 beschrieben) nach Umkristallisation aus Methanol/Diäthyläther 8,9 g des Trifluoracetats von [[1-(N-(p-Amidinobenzoyl)-L-alanyl]-4-piperidinyl]-oxy]essigsäure. Smp. 120 °C (Zersetzung). MS (FAB): 377 $(M+H)^+$. $[\alpha]_D^{20}$ = +24,7 ° (c = 0,7, Wasser).

Das Ausgangsmaterial kann man wie folgt herstellen:

a) Kupplung von 18 g Z-L-Alanin mit 17,4 g 4-Piperidinyloxyessigsäure-t-butylester und nachfolgende Hydrogenolyse des erhaltenen Produktes, wie in Beispiel 1d) und e), ergibt 15,8 g des Acetats von 1-[(1-L-Alanyl-4-piperidinyl)oxy]essigsäure-t-butylester. Smp. 93-96 °C. $[\alpha]_D^{20}$ = +2,0 ° (c = 1,0, Methanol).

b) Die Kupplung von 4,7 g des Produktes von a) mit 3,4 g p-Amidinobenzoylchlorid-hydrochlorid, wie in Beispiel 1f), ergibt 4,2 g des erwünschten Ausgangsmaterials. MS (EI): 433 $(M+H)^+$.

Beispiel 4

Aus 0,3 g [[1-[N-[p-[N-(t-Butoxycarbonyl)amidinobenzoyl]-D-alanyl]-4-piperidinyl]oxy]essigsäure-t-butylester erhält man in Analogie zu Beispiel 1 0,1 g [[1-[N-(p-Amidinobenzoyl)-D-alanyl]-4-piperidinyl]oxy]-essigsäure als Trifluoracetat. Smp. 115 °C (Zersetzung). $[\alpha]_D^{20}$ = -27,5 ° (c = 0,8, Wasser). MS (FAB): 377 $(M+H)^+$

Das Ausgangsmaterial kann man wie folgt herstellen:

a) Durch Umsetzung von 3 g 4-Piperidinyloxyessigsäure-t-butylester mit 2,43 g Z-D-Alanin, wie in Beispiel 1d) beschrieben, erhält man nach Chromatographie an Kieselgel mit Essigester/Hexan 1:1 3,1 g Benzyl-[(R)-1-[[4-[(t-butoxycarbonyl)methoxy]piperidinyl]carbonyl]äthyl]carbamat. MS (EI): 420 $(M)^+$.

b) Durch Hydrogenolyse von 3,1 g des Produktes von a), wie in Beispiel 1e) beschrieben, erhält man 2,5 g des Acetats von 1-[(1-D-Alanyl-4-piperidinyl)oxy]essigsäure-t-butylester. MS (EI): 215 $(M\text{-}C_3H_5NO)$.

c) Durch Umsetzung von 1 g der Vorstufe mit 0,66 g p-Amidinobenzoylchlorid-hydrochlorid in DMF in Gegenwart von Triäthylamin und nachfolgender Behandlung mit Di-t-butyl-dicarbonat gewinnt man nach Chromatographie an Kieselgel mit Dichlormethan/Methanol 20:1 0,3 g des erwünschten Ausgangsmaterials. MS (FAB): 533 $(M+H)^+$.

Beispiel 5

Durch Hydrolyse von 1,6 g [[1-[N-[(5-Amidino-2-pyridyl)carbonyl]-L-alanyl]-4-piperidinyl]oxy]essigsäure-t-butylester in mit Chlorwasserstoff gesättigtem Eisessig erhält man nach Chromatographie an silyliertem Kieselgel RP-18 und Umkristallisation aus THF/Essigester 0,15 g [[1-[N-[(5-Amidino-2-pyridyl)carbonyl]-L-alanyl]-4-piperidinyl]oxy]essigsäure. Smp. über 200 °C (Zersetzung). MS (FAB): 378 $(M+H)^+$.

Das Ausgangsmaterial kann man wie folgt herstellen:

a) Durch Umsetzung von 2,4 g des Acetats von 1-[(1-L-Alanyl-4-piperidinyl)oxy]essigsäure-t-butylester (Beispiel 3a)) mit 1,0 g 5-Cyan-2-picolinsäure gemäss Beispiel 1d) erhält man 2,43 g [[1-[N-[(5-Cyan-2-pyridyl)carbonyl]-L-alanyl]-4-piperidinyl]oxy]essigsäure-t-butylester, MS (FAB): 417 $(M+H)^+$.

b) Die aufeinanderfolgende Behandlung von 2,4 g der Vorstufe wie in Beispiel 2A)B)C) beschrieben liefert 2 g des erwünschten Ausgangsmaterials. Smp. 142-145 °C. MS (FAB): 434 $(M+H)^+$.

Beispiel 6

Aus 1 g des Acetats von [[1-[N-(p-Amidinobenzoyl)-L-valyl]-4-piperidinyl]oxy]essigsäure-t-butylester erhält man in Analogie zu Beispiel 1 nach Kristallisation aus Essigester 0,8 g [[1-[N-(p-Amidinobenzoyl)-L-valyl]-4-piperidinyl]oxy]essigsäure als Trifluoracetat. Smp. 210-211 °C. MS (FAB): 405 $(M+H)^+$. $[\alpha]_D^{20}$ = +32,6 ° (c = 0,8, Wasser).

Das Ausgangsmaterial kann man wie folgt herstellen:

a) Durch Kupplung von 2,5 g Z-L-Valin mit 2 g 4-Piperidinyloxyessigsäure-t-butylester, wie in Beispiel 2b) beschrieben erhält man 4 g [[1-[N-[(Benzyloxy)carbonyl]-L-valyl]-4-piperidinyl]oxy]essigsäure-t-butylester, MS (EI): 449 $(M+H)^+$.

b) In Analogie zu Beispiel 1e), jedoch ohne Zugabe von Essigsäure, erhält man aus 1,9 g des Produkts von Beispiel 6a) 1,4 g 1-[(1-L-Valyl-4-piperidinyl)oxy]essigsäure-t-butylester, MS (EI): 315 $(M+H)^+$.

c) In Analogie zu Beispiel 1f) erhält man aus 3,3 g des Produkts von Beispiel 6b) und 2,5 g p-Amidinobenzoylchlorid-hydrochlorid nach Chromatographie (Kieselgel; Dichlormethan/Methanol/Essigsäure 95:4:1) und Kristallisation aus Diäthyläther, 1,1 g des erwünschten Ausgangsacetats. Smp. 179-182 °C. MS (FAB): 461 $(M+H)^+$.

Beispiel 7

Aus 1,5 g des Acetats von [[1-[N-(p-Amidinobenzoyl)-L-leucyl]-4-piperidinyl]oxy]essigsäure-t-butylester erhält man in Analogie zu Beispiel 1 nach Kristallisation aus Essigester/Diäthyläther 1,1 g [[1-[N-(p-Amidinobenzoyl)-L-leucyl]-4-piperidinyl]oxy]essigsäure als Trifluoracetat. Smp. 216-218 °C. MS (FAB): 419 $(M+H)^+$. $[\alpha]_D^{20}$ = +22,5 ° (c = 0,8, Wasser).

Das Ausgangsacetat kann man wie folgt herstellen:

a) Durch Kupplung von 2,6 g Z-L-Leucin mit 2 g 4-Piperidinyloxyessigsäure-t-butylester wie in Beispiel 1d) beschrieben, erhält man 4,1 g [[1-[N-[(Benzyloxy)carbonyl]-L-leucyl]-4-piperidinyl]oxy]essigsäure-t-butylester. MS (FAB): 463 $(M+H)^+$.

b) In Analogie zu Beispiel 6b) und 1f) erhält man aus 4,1 g des Produkts von Beispiel 7a) nach Chromatographie (Kieselgel; Dichlormethan/Methanol/Essigsäure 95:4:1) und Kristallisation aus Diäthyläther 1,5 g des erwünschten Acetats. Smp. 120-129 °C (Zersetzung). MS (FAB): 475 $(M+H)^+$.

Beispiel 8

Aus 1,4 g des Acetats von [[1-[(p-Amidino-N-methylbenzamido)acetyl]-4-piperidinyl]oxy]essigsäure-t-butylester erhält man in Analogie zu Beispiel 1 nach Kristallisation aus Diäthyläther 0,9 g [[1-[(p-Amidino-N-methylbenzamido)acetyl]-4-piperidinyl]oxy]essigsäure als Trifluoracetat. Smp. 134-135 °C. MS (FAB): 377 $(M+H)^+$.

Das Ausgangsmaterial kann man wie folgt herstellen:

a) Durch Kupplung von 2,0 g Z-Sarcosin-N-hydroxysuccinimidester mit 1,3 g 4-Piperidinyloxyessigsäure-t-butylester in Gegenwart von Triäthylamin in THF erhält man 2,1 g Benzyl-[4-[[[(t-Butoxycarbonyl)-methoxy]piperidino]carbamoyl]methyl]methylcarbamat. MS (FAB): 421 $(M+H)^+$.

b) In Analogie zu Beispiel 6b) und 1f) erhält man aus 4 g des Produkts von Beispiel 8a) nach Chromatographie (Kieselgel; Dichlormethan/Methanol/Essigsäure 93:5:2) und Kristallisation aus Diäthyläther 1,5 g des erwünschten Acetats. Smp. 188-189 °C. MS (FAB): 432 $(M+H)^+$.

Beispiel 9

Aus 5,4 g [[1-[$N^2$-(p-Amidinobenzoyl)-$N^5$-(t-butoxycarbonyl)-L-ornithyl]-4-piperidinyl]oxy]essigsäure-t-butylester erhält man in Analogie zu Beispiel 1 4,9 g [[1-[$N^2$-(p-Amidinobenzoyl)-L-ornithyl]-4-piperidinyl]oxy]-essigsäure als Trifluoracetat. MS (FAB): 420 $(M+H)^+$. $[\alpha]_D^{20}$ = +4,5 ° (c = 0,8, MeOH).

Das Ausgangsmaterial kann man wie folgt herstellen:

a) Durch Umsetzung von 6 g 4-Piperidinyloxyessigsäure-t-butylester mit 10,2 g $N^2$-Z-$N^5$-Boc-L-Ornithin, wie in Beispiel 1d) beschrieben erhält man nach Chromatographie an Kieselgel mit Essigester/Hexan 1:1 11 g [[1-[$N^2$-(Benzyloxycarbonyl)-$N^5$-(t-butoxycarbonyl)-L-ornithyl]-4-piperidinyl]oxy]essigsäure-t-butylester. MS (FAB): 564 $(M+H)^+$.

b) Durch Hydrogenolyse von 11 g des Produkts von a) wie in Beispiel 1e) beschrieben erhält man 9 g des Acetats von [[1-[$N^5$-(t-Butoxycarbonyl)-L-ornithyl]-4-piperidinyl]oxy]essigsäure-t-butylester MS (FAB): 430 $(M+H)^+$.

c) Durch Umsetzung von 9 g des Produkts von b) mit 4,4 g p-Amidinobenzoylchlorid-hydrochlorid, wie in Beispiel 1f) beschrieben gewinnt man 5,7 g des erwünschten Ausgangsmaterials. MS (FAB): 576 $(M+H)^+$.

Beispiel 10

Aus 0,54 g [[[1-[$N^2$-[p-N-(t-Butoxycarbonyl)amidinobenzoyl]-$N^6$-(t-butoxycarbonyl)-L-lysyl]-4-piperidinyl]-oxy]essigsäure-t-butylester erhält man in Analogie zu Beispiel 1 0,35 g [[1-[$N^2$-(p-Amidinobenzoyl)-L-lysyl]-4-piperidinyloxy]essigsäure als Trifluoracetat. MS (FAB): 434 $(M+H)^+$. $[\alpha]_D^{20}$ = +12,4° (c = 0,8, Wasser).

Das Ausgangsmaterial kann man wie folgt herstellen:

a) Durch Umsetzung von 2 g 4-Piperidinyloxyessigsäure-t-butylester mit 2,8 g $N^2$-Z-$N^6$-Boc-L-Lysin, wie in Beispiel 1d) beschrieben, erhält man nach Chromatographie an Kieselgel mit Essigester/Hexan 1:1 2,6 g [[1-[$N^2$-(Benzyloxycarbonyl)-$N^6$-(t-butoxycarbonyl)-L-lysyl]-4-piperidinyl]oxy]essigsäure-t-butylester. MS (FAB): 578 $(M+H)^+$.

b) Durch Hydrogenolyse von 2,6 g des erhaltenen Produkts, wie in Beispiel 1e) beschrieben, erhält man 2 g des Acetats von [[1-[$N^6$-(t-Butoxycarbonyl)-L-lysyl]-4-piperidinyl]oxy]essigsäure-t-butylester. MS (FAB): 444 $(M+H)^+$.

c) Durch Umsetzung von 2 g des Produkts der Vorstufe mit 1 g p-Amidinobenzoylchlorid-hydrochlorid wie in Beispiel 4c) beschrieben gewinnt man nach Chromatographie (Kieselgel; Dichlormethan/Methanol 20:1) 1,95 g des erwünschten Ausgangsmaterials. MS (FAB): 690 $(M+H)^+$.

Beispiel 11

Aus 0,4 g des Acetats von [[1-[N-(p-Amidinobenzoyl)-L-phenylglycyl]-4-piperidinyl]oxy]essigsäure-t-butylester erhält man in Analogie zu Beispiel 1 0,25 g [[1-[N-(p-Amidinobenzoyl)-L-phenylglycyl]-4-piperidinyl]oxy]essigsäure als Trifluoracetat. Smp. über 250°C (Essigester/Diäthyläther 1:1). MS (FAB): 439 $(M+H)^+$. $[\alpha]_D^{20}$ = +6,5° (c = 0,6, MeOH).

Das Ausgangsmaterial kann man wie folgt herstellen:

a) Durch Umsetzung von 1,85 g 4-Piperidinyloxyessigsäure-t-butylester mit 3,5 g Z-L-Phenylglycin-N-hydroxysuccinimidester, wie in Beispiel 8a) beschrieben, erhält man nach Chromatographie an Kieselgel mit Petroläther/Diäthyläther 1:1 3,8 g [[1-(N-Benzyloxycarbonyl-L-phenylglycyl)-4-piperidinyl]oxy]-essigsäure-t-butylester. MS (FAB): 483 $(M+H)^+$.

b) Durch Hydrogenolyse von 4,7 g des erhaltenen Produktes wie in Beispiel 6b) beschrieben erhält man 3,2 g [[1-(L-Phenylglycyl)-4-piperidinyl]oxy]essigsäure-t-butylester. MS (FAB): 349 $(M+H)^+$.

c) Durch Umsetzung von 3,2 g der Vorstufe mit 2,2 g p-Amidinobenzoylchlorid-hydrochlorid, wie in Beispiel 1f) beschrieben, gewinnt man nach Chromatographie (Kieselgel; Dichlormethan/Methanol/Essigsäure 95:5:2) 0,4 g des erwünschten Acetats. Smp. 207-220°C (Essigester, Zersetzung). MS (FAB): 495 $(M+H)^+$.

Beispiel 12

Aus 0,5 g des Acetats von [[1-[1-(p-Amidinobenzoyl)-2-methy-L-prolyl]-4-piperidinyl]oxy]essigsäure-t-butylester erhält man in Analogie zu Beispiel 1 0,14 g [[1-[1-(p-Amidinobenzoyl)-2-methyl-L-prolyl]-4-piperidinyl]oxy]essigsäure als Trifluoracetat. Smp. 219-220°C (Acetonitril). MS (FAB): 417 $(M+H)^+$. $[\alpha]_D^{20}$ = +17,1° (c = 0,9, MeOH).

Das Ausgangsacetat kann man wie folgt herstellen:

a) Durch Umsetzung von 2-Methyl-L-prolin-hydrobromid mit p-Cyanbenzoylchlorid analog Beispiel 2b) erhält man 1-(p-Cyanbenzoyl)-2-methyl-L-prolin. MS (EI): 213 $(M-COOH)^+$.

b) Durch Umsetzung von 1,67 g 4-Piperidinyloxyessigsäure-t-butylester mit 0,8 g des Säurechlorids von 1-(p-Cyanbenzoyl)-2-methyl-L-prolin (erhalten durch Behandlung des Produktes der Vorstufe mit Thionylchlorid) erhält man 0,89 g [[1-[1-(p-Cyanbenzoyl)-2-methyl-L-prolyl]-4-piperidinyl]oxy]essigsäure-t-butylester. Smp. 180-182°C (Essigester).

c) Durch aufeinanderfolgende Behandlung von 0,89 g des Produktes von b), wie in Beispiel 2A)B)C) beschrieben, gewinnt man nach Chromatographie (silyliertes Kieselgel RP-18; Wasser/Methanol 9:1) 0,59 g des erwünschten Acetats. Smp. 191-192°C (Essigester, Zersetzung). MS (FAB): 473 $(M+H)^+$.

Beispiel 13

Aus 2,5 g des Acetats von [[1-[N-(p-Amidinobenzoyl)-3-phenyl-L-alanyl]-4-piperidinyl]oxy]essigsäure-t-butylester erhält man in Analogie zu Beispiel 1 1,9 g [[1-[N-(p-Amidinobenzoyl)-3-phenyl-L-alanyl]-4-piperidinyl]oxy]essigsäure ah Trifluoracetat. Smp. 234-235 °C (Essigester). $[\alpha]_D^{20}$ = +17,9° (c = 1,0, MeOH). MS (EI): 453 $(M+H)^+$.

Das Ausgangsacetat kann man wie folgt herstellen:

a) Durch Umsetzung von 2,15 g 4-Piperidinyloxyessigsäure-t-butylester mit 3,0 g Z-L-Phenylalanin, wie in Beispiel 2b) beschrieben, erhält man 4,8 g [[1-(N-Benzyloxycarbonyl-3-pheny-L-alanyl)-4-piperidinyl]-oxy]essigsäure-t-butylester. MS (FAB): 497 $(M+H)^+$.

b) Durch Hydrogenolyse von 4,8 g des erhaltenen Produktes, wie in Beispiel 6b) beschrieben, und nachfolgende Umsetzung mit 2,0 g p-Amidinobenzoylchlorid-hydrochlorid, wie in Beispiel 1f) beschrieben, gewinnt man nach Chromatographie (Kieselgel; Dichlormethan/Methanol/Essigsäure 22:2:1) 2,5 g des erwünschten Acetats. Smp. 176-178 °C (Diäthyläther). MS (FAB): 509 $(M+H)^+$.

Beispiel 14

Aus 2,5 g des Acetats von [[1-[N-(p-Amidinobenzoyl)-3-(p-t-butoxyphenyl)-L-alanyl]-4-piperidinyl]oxy]-essigsäure-t-butylester erhält man in Analogie zu Beispiel 1 nach Chromatographie (silyliertes Kieselgel RP-18, Wasser/Methanol-Gradient) 1,0 g [[1-[N-(p-Amidinobenzoyl)-L-tyrosyl]-4-piperidinyl]oxy]essigsäure als Trifluoracetat. Smp. 125-130 °C (Essigester, Zersetzung). MS (FAB): 469 $(M+H)^+$.

Das Ausgangsacetat kann man wie folgt herstellen:

a) Durch Umsetzung von 2,15 g 4-Piperidinyloxyessigsäure-t-butylester mit 3,71 g N-Z-(OtBu)-L-Tyrosin, wie in Beispiel 2b) beschrieben, erhält man nach Chromatographie (Kieselgel; Diäthyläther/Petroläther 1:1) 4,8 g [[1-[N-(Benzyloxycarbonyl)-3-[p-(t-butoxyphenyl)]-L-alanyl]-4-piperidinyl]oxy]essigsäure-t-butylester. Smp. 96 °C (Diäthyläther), MS (EI): 417 $(M-C_7H_7-C_4H_8)^+$, $[\alpha]_D^{20}$ = +5,4° (c = 0.8, $CH_3OH$).

b) Durch Hydrogenolyse von 4,8 g der Vorstufe wie in Beispiel 6b) und nachfolgende Umsetzung mit 1,5 g p-Amidinobenzoylchlorid-hydrochlorid wie in Beispiel 1f) gewinnt man nach Chromatographie (Kieselgel; Dichlormethan/Methanol/Essigsäure 22:2:1) 2,6 g des erwünschten Acetats. Smp. 170-172 °C (Diäthyläther). MS (FAB): 581 $(M+H)^+$.

Beispiel 15

Als Nebenprodukt der in Beispiel 14 beschriebenen Chromatographie isoliert man 0,09 g des Trifluoracetats von [[1-[N-(p-Amidinobenzoyl)-L-tyrosyl]-4-piperidinyl]oxy]essigsäuremethylester. Smp. 189-190 °C (Essigester). MS (FAB): 483 $(M+H)^+$.

Beispiel 16

Durch Umsetzung von 0,58 g des Trifluoracetats von [[1-[N-(p-Amidinobenzoyl)-L-tyrosyl]-4-piperidinyl]-oxy]essigsäure (Beispiel 14) mit Chloramin T gefolgt von Natriumjodid in Wasser/DMF 8:1 erhält man nach Chromatographie (silyliertes Kieselgel RP-18, Wasser/Acetonitril-Gradient) 0,04 g [[1-[N-(p-Amidinobenzoyl)-3-(4-hydroxy-3-jodphenyl)-L-alanyl]-4-piperidinyl]oxy]essigsäure. Smp. 230 °C (Wasser, Zersetzung). MS (FAB): 595 $(M+H)^+$.

Beispiel 17

Aus der in Beispiel 16 beschriebenen Reaktion isoliert man zusätzlich 0,09 g [[1-[N-(p-Amidinobenzoyl)-3-(4-hydroxy-3,5-dijodphenyl)-L-alanyl]-4-piperidinyl]oxy]essigsäure. Smp. 220-221 °C (Wasser, Zersetzung). MS (FAB): 720 $(M+H)^+$.

Beispiel 18

Aus 1,3 g [[1-[3-t-Butoxy-N-[p-[N-(t-butoxycarbonyl)amidino]benzoyl]-L-alanyl]-4-piperidinyl]oxy]-essigsäure-t-butylester erhält man durch Behandlung mit Chlorwasserstoff in Eisessig nach Chromatographie (silyliertes Kieselgel RP-18, Methanol/Wasser-Gradient) 0,45 g des Hydrochlorids von [[1-[3-Acetoxy-N-(p-amidinobenzoyl)-L-alanyl]-4-piperidinyl]oxy]essigsäure. $[\alpha]_D^{20}$ = +0,9° (c = 1,0, MeOH). MS (FAB): 435 $(M+H)^+$.

Das Ausgangsmaterial kann man wie folgt herstellen:

a) Durch Kupplung von 7,5 g Z-L-Ser(tBu)-OH mit 7,0 g 4-Piperidinyloxyessigsäure-t-butylester und nachfolgende Hydrogenolyse des erhaltenen Produktes, wie in Beispiel 1d)e) beschrieben, erhält man 10,6 g des Acetats von [[1-(3-t-Butoxy-L-alanyl)-4-piperidinyl]oxy]essigsäure-t-butylester Smp. 76-78 °C. MS (FAB): 359 $(M+H)^+$.

b) Durch Umsetzung von 9,9 g der Vorstufe mit 5,2 g p-Amidinobenzoylchlorid-hydrochlorid in DMF in Gegenwart von Triäthylamin und nachfolgende Behandlung mit Di-t-butyl-dicarbonat gewinnt man nach Chromatographie an Kieselgel mit Dichlormethan/Methanol 20:1 gefolgt von Essigester/Hexan 3:1 4,3 g [-[1-[3-t-Butoxy-N-[p-[N-(t-butoxycarbonyl)amidino]benzoyl]-L-alanyl]-4-piperidinyl]oxy]essigsäure-t-butylester Smp. 162-165 °C. MS (FAB): 605 $(M+H)^+$.

## Beispiel 19

Aus 1,0 g [[1-[3-t-Butoxy-N-[p-[N-(t-butoxycarbonyl)amidino)benzoyl]-L-alanyl]-4-piperidinyl]oxy]-essigsäure-t-butylester (Beispiel 18) erhält man in Analogie zu Beispiel 1 nach Chromatographie (silyliertes Kieselgel RP-18, Wasser) 0,58 g des Trifluoracetats von [[1-[N-(p-Amidinobenzoyl)-L-seryl]-4-piperidinyl]-oxy]essigsäure. $[\alpha]_D^{20}$ = +17,6° (c = 1,0, Wasser). MS (FAB): 393 $(M+H)^+$.

## Beispiel 20

Aus 5 g L-N-(p-Amidinobenzoyl)-3-[[4-[(t-butoxycarbonyl)methoxy]piperidinyl]carbonyl]-β-alanin-t-butylester erhält man in Analogie zu Beispiel 1 nach Kristallisation mit Essigester/THF 2,0 g des Trifluoracetats von L-N-(p-Amidinobenzoyl)-3-[[4-(carboxymethoxy)piperidino]carbonyl]-β-alanin, Smp. 145-150 °C. MS (FAB): 421 $(M+H)^+$.

Das Ausgangsmaterial kann man wie folgt herstellen:

a) Durch Kupplung von 11 g des Monohydrats von Z-L-Asp(O-tBu)-OH mit 7,0 g 4-Piperidinyloxyessigsäure-t-butylester, wie in Beispiel 2b) beschrieben, erhält man 16 g L-N-(Benzyloxycarbonyl)-3-[[4-[(t-butoxycarbonyl)methoxy]piperidino]carbonyl]-β-alanin-t-butylester. MS (FAB): 521 $(M+H)^+$.

b) Nach Hydrogenolyse von 17 g der Vorstufe, wie in Beispiel 6b) ausgeführt, isoliert man 11 g L-3-[[4-[(t-Butoxycarbonyl)methoxy]piperidino]carbonyl]-β-alanin-t-butylester. MS (FAB): 387 $(M+H)^+$.

c) Durch Kupplung von 11 g der Vorstufe mit 6,9 g p-Amidinobenzoylchlorid-hydrochlorid, wie in Beispiel 1f) ausgeführt, isoliert man nach Chromatographle (Kieselgel, Dichlormethan/Methanol 9:1) 10,2 g des erwünschten Ausgangsmaterials. MS (FAB): 533 $(M+H)^+$.

## Beispiel 21

Aus 0,5 g [[1-[N-(p-Amidinobenzoyl)-4-t-butoxy-L-glutamoyl]-4-piperidinyl]oxy]essigsäure-t-butylester erhält man in Analogie zu Beispiel 1 nach Kristallisation mit Essigester 0,25 g des Trifluoracetats von [[1-[N-(p-Amidinobenzoyl)-L-α-glutamoyl]-4-piperidinyl]oxy]essigsäure. Smp. 105-108 °C. $[\alpha]_D^{20}$ = +6,9° (c = 0,8, Methanol). MS (FAB): 435 $(M+H)^+$.

Das Ausgangsmaterial kann man wie folgt herstellen:

a) Durch Kupplung von 11 g Z-L-Glu(Ot-Bu)-OH mit 7,0 g 4-Piperidinyloxyessigsäure-t-butylester, wie in Beispiel 1d) beschrieben, erhält man 15,4g [[1-[N-(Benzyloxycarbonyl)-4-t-butoxy-L-glutamoyl]-4-piperidinyl]oxy]essigsäure-t-butylester. MS (FAB): 535 $(M+H)^+$.

b) Nach Hydrogenolyse von 15,4 g der Vorstufe, wie in Beispiel 6b) ausgeführt, erhält man 7,5 g des Acetats von [[1-(4-t-Butoxy-L-glutamoyl]-4-piperidinyl]oxy]essigsäure-t-butylester. MS (FAB): 401 $(M+H)^+$.

c) Durch Kupplung von 7,5 g der Vorstufe mit 3,9 g p-Amidinobenzoylchlorid-hydrochlorid, wie in Beispiel 1f) ausgeführt, erhält man 6,9 g [[1-[N-(p-Amidinobenzoyl)-4-t-butoxy-L-glutamoyl]-4-piperidinyl]-oxy]essigsäure-t-butyl ester. MS (FAB): 547 $(M+H)^+$.

## Beispiel 22

Aus 2 g [[(R/S)-1-[N-(p-Amidinobenzoyl)-L-alanyl]-3-piperidinyl]methoxy]essigsäure-t-butylester erhält man in Analogie zu Beispiel 1 0,6 g des Trifluoracetats von [[(R/S)-1-[N-(p-Amidinobenzoyl)-L-alanyl]-3-piperidinyl]methoxy]essigsäure. Smp. 87-90 °C (Essigester). MS (FAB): 391 $(M+H)^+$.

Das Ausgangsmaterial kann man wie folgt herstellen:

a) Aus rac-3-(Hydroxymethyl)piperidin erhält man in Analogie zu Beispiel 1a) rac-N-Benzyloxycarbonyl-3-(hydroxymethyl)piperidin. MS (EI): 249 $(M)^+$.

b) Aus dem Produkt von a) erhält man in Analogie zu Beispiel 1b) Benzyl-rac-3-[[(t-butoxycarbonyl)-methoxy]methyl]-1-piperidincarboxylat. MS (EI): 307 $(M-C_4H_8)^+$.

c) Das Produkt von b) wird in Analogie zu Beispiel 1c) zu rac-(3-Piperidinylmethoxy)essigsäure-t-butylester hydriert. MS (EI): 172 $(M-C_4H_8)^+$.

d) Durch Kupplung des Produktes von c) mit Z-L-Alanin, wie in Beispiel 1d), erhält man Benzyl-[(S)-1-[[-(R/S)-3-[(t-butoxycarbonyl)methoxy]piperidino]carbonyl]äthyl]carbamat. MS (EI): 434 $(M)^+$.

e) Durch Hydrierung des Produktes von d), wie in Beispiel 1e), gewinnt man das Acetat von [[(R/S)-1-L-Alanyl-3-piperidinyl]methoxy]essigsäure-t-butylester. MS (EI): 285 $(M-CH_3)^+$.

f) Durch Kupplung des Produktes von e) mit p-Amidinobenzoylchloridhydrochlorid, wie in Beispiel 1f), erhält man nach Chromatographie (silyliertes Kieselgel RP-18) das erwünschte Ausgangsmaterial. MS (FAB): 447 $(M+H)^+$.

## Beispiel 23

Aus 2,7 g [[1-[N-(p-Amidinobenzoyl)-L-alanyl]-4-(α,α,α-trifluor-m-tolyl)-4-piperidinyl]oxy]essigsäure-t-butylester erhält man in Analogie zu Beispiel 1 0,7 g [[1-[N-(p-Amidinobenzoyl)-L-alanyl]-4-(α,α,α-trifluor-m-tolyl)-4-piperidinyl]oxy]essigsäure. Smp. über 280 °C (Wasser/Methanol). MS (FAB): 521 $(M+H)^+$.

Das Ausgangsmaterial kann man wie folgt herstellen:

a) Aus 4-(3-(Trifluormethyl)phenyl)piperidin-4-ol erhält man in Analogie zu Beispiel 1a) Benzyl-4-hydroxy-4-(α,α,α-trifluor-m-tolyl)-1-piperidincarboxylat. MS (EI): 379 $(M)^+$.

b) Aus dem Produkt von a) erhält man in Analogie zu Beispiel 1b) Benzyl-4-[(t-butoxycarbonyl)methoxy]-4-(α,α,α-trifluor-m-tolyl)-1-piperidincarboxylat. MS (FAB): 494 $(M+H)^+$.

c) Durch Hydrierung des Produktes von b), wie in Beispiel 1e), gewinnt man das Acetat von [[4-(α,α,α-Trifluor-m-tolyl)-4-piperidinyl]oxy]essigsäure-t-butylester. MS (EI): 227 $(M-C_6H_{12}O_3)^+$.

d) Durch Kupplung des Produktes von c) mit Z-L-Alanin, wie in Beispiel 1d), erhält man Benzyl-[(S)-1-[[4-[(t-butoxycarbonyl)methoxy]-4-(α,α,α,-trifluor-m-tolyl)-1-piperidinyl]carbonyl]äthyl]carbamat. MS (FAB): 565 $(M+H)^+$.

e) Durch Hydrierung des Produktes von d), wie in Beispiel 1e), gewinnt man das Acetat von 1-[[L-Alanyl-4-(α,α,α-trifluor-m-tolyl)-4-piperidinyl]oxy]essigsäure-t-butylester. MS (EI): 415 $(M-CH_3)^+$.

f) Durch Kupplung des Produktes von e) mit p-Amidinobenzoylchloridhydrochlorid, wie in Beispiel 1f), erhält man das erwünschte Ausgangsmaterial. MS (EI): 577 $(M+H)^+$.

## Beispiel 24

Eine Lösung von 150 mg t-Butyl-[[1-[1-(p-amidinobenzoyl)-L-prolyl]-4-piperidinyl]oxy]acetat in 10 ml Dichlormethan und 10 ml Trifluoressigsäure wird 2 Stunden bei Raumtemperatur gerührt und eingedampft. Der Rückstand wird in Aether suspendiert und abgenutscht. Man erhält 141 mg [[1-[1-(p-Amidinobenzoyl)-L-prolyl]-4-piperidinyl]oxy]essigsäure-trifluoracetat. Smp. 234-236 °C.

Das Ausgangsmaterial kann wie folgt hergestellt werden:

a) 4,97 g 4-Cyanobenzoesäurechlorid, 3,45 g L-Prolin und 0,73 g Tetramethylammoniumsulfat werden in 300 ml Dichlormethan und 150 ml 5%iger Natriumhydrogencarbonatlösung 48 Stunden gerührt. Die wässrige Phase wird mit 3N Schwefelsäure angesäuert und mit Essigester extrahiert. Die Essigesterphase wird mit gesättigter Natriumchloridlösung gewaschen, getrocknet und eingedampft. Chromatographie des Rückstandes an Kieselgel (RP-18) mit Wasser ergibt 3,70 g 1-(p-Cyanobenzoyl)-L-prolin. Smp. 80-85 °C.

b) Die Kupplung von 250 mg 1-(p-Cyanobenzoyl)-L-prolin mit 215 mg 4-Piperidinyloxyessigsäure-t-butylester ergibt nach Chromatographie an Kieselgel mit Essigester/Methanol (98:2) 300 mg t-Butyl-[[1-[1-(p-cyanobenzoyl)-L-prolyl]-4-piperidinyl]oxy]acetat. MS: 442 $(M+H)^+$.

c) Die Behandlung von 1 g der Vorstufe wie in Beispiel 2A)B)C) führt via t-Butyl-[[1-[1-[p-(thiocarbamoyl)-benzoyl]-L-prolyl]-4-piperidinyl]oxy]acetat. Smp. 108-110 °C, und

t-Butyl-[[1-[1-[p-[1-methylthio)formimidoyl]benzoyl]-L-prolyl]-4-piperdinyl]oxy]acetat, Smp. 132-133 °C,

zu 72 mg des erwünschten Acetats, Smp. 100 °C (Zersetzung).

Beispiel 25

Analog Beispiel 24 erhält man aus 150 mg t-Butyl-[[1-[(4R)-1-(p-amidinobenzoyl)-4-benzyloxy-L-prolyl]-4-piperidinyl)oxy]acetat-hydrojodid nach Chromatographie an Kieselgel (RP-18, Wasser/THF 95:5) 72 mg [-[1-[(4R)-1-(p-Amidinobenzoyl)-4-benzyloxy-L-prolyl]-4-piperidinyl]oxy]essigsäure. Smp. 226-227 °C.

Das Ausgangsacetat kann wie folgt hergestellt werden:

a) Zu einer Lösung von 905 mg (4R)-Hydroxy-L-prolinmethylester und 828 mg 4-Cyanobenzoylchlorid in 50 ml Dichlormethan werden 1,46 ml Triäthylamin zugegeben. Nach Rühren wird die Lösung mit gesättigter Natriumchloridlösung gewaschen, getrocknet und eingedampft. Chromatographie des Rückstandes an Kieselgel (Essigester/Hexan 5:1) ergibt 810 mg (4R)-1-(p-Cyanobenzoyl)-4-hydroxy-L-prolinmethylester. Smp. 101-102 °C.

b) Zu einer Lösung von 730 mg der Vorstufe und 600 µl Benzyltrichloracetimidat in 5 ml Cyclohexan und 5 ml Dichlormethan werden 40 µl Trifluormethansulfonsäure zugetropft. Der entstandene Niederschlag wird abgenutscht und das Filtrat mit 5%iger Natriumhydrogencarbonatlösung gewaschen, getrocknet und eingedampft. Chromatographie des Rückstandes an Kieselgel (Essigester) ergibt 940 mg (4R)-4-Benzyloxy-1-(p-cyanobenzoyl)-L-prolinmethylester. MS: 305 (M-59).

c) 880 mg der Vorstufe und 1,2 ml 2N Lithiumhydroxidlösung werden in 10 ml Methanol gerührt. Nach Entfernen des Methanols wird der wässrige Rückstand mit 2,4 ml 1N Salzsäure angesäuert und mit Essigester extrahiert. Trocknen der organischen Phase und Eindampfen ergibt 470 mg (4R)-4-Benzyloxy-1-(p-cyanobenzoyl)-L-prolin. Smp. 58-60 °C.

d) 450 mg des Produkts von c) werden in Gegenwart von HBTU mit 280 mg 4-Piperidinyloxyessigsäure-t-butylester gekuppelt. Der Rückstand wird in Essigester gelöst und die Essigesterphase mit 5%iger Natriumhydrogencarbonatlösung,1N Kaliumhydrogensulfatlösung und gesättigter Natriumchloridlösung gewaschen, getrocknet und eingedampft. Nach Chromatographie des Rückstands an Kieselgel (Dichlormethan/Methanol 98:2) erhält man 500 mg t-Butyl-[[1-[(4R)-4-benzyloxy-1-(p-cyanobenzoyl)-L-prolyl]-4-piperidinyl]oxy]acetat. MS: 548 $(M+H)^+$.

e) Die Behandlung von 400 mg der Vorstufe wie in Beispiel 2A)B)C) führt zu 177 mg des erwünschten Acetats. Smp. des Hydrojodids 148-150 °C.

Beispiel 26

Eine Lösung von 1,60 g t-Butyl-[[1-[(4R)-1-(p-amidinobenzoyl)-4-hydroxy-L-prolyl]-4-piperidinyl]oxy]-acetat in 20 ml Dichlormethan und 20 ml Trifluoressigsäure wird 2 Stunden bei Raumtemperatur gerührt und eingedampft. Der Rückstand wird in Aethanol gelöst und mit Aether versetzt. Abnutschen und Trocknen des Niederschlags ergibt 1,25 g [[1-[(4R)-1-(p-Amidinobenzoyl)-4-hydroxy-L-prolyl]-4-piperidinyl]oxy]-essigsäure-trifluoracetat Smp. 220 °C (Zersetzung).

Das Ausgangsacetat kann wie folgt hergestellt werden:

a) Die Kupplung von 14,78 g (4R)-1-(Benzyloxycarbonyl)-4-hydroxy-L-prolinmit 12,0 g 4-Piperidinyloxyessigsäure-t-butylester ergibt nach Chromatographie an Kieselgel (Essigester/Methanol 95:5) 17,83 g t-Butyl-[[1-[(4R)-1-(benzyloxycarbonyl)-4-hydroxy-L-prolyl]-4-piperidinyl]oxy]acetat. MS: 463 $(M+H)^+$.

b) Hydrierung von 17,0 g der Vorstufe in Aethanol in Anwesenheit von 2,0 g Pd/C (10%) ergibt nach Abfiltieren des Katalysators und Einengen 11,06 g t-Butyl-[[1-[(4R)-4-hydroxy-L-prolyl]-4-piperidinyl]oxy]-acetat MS: 329 $(M+H)^+$.

c) Die Reaktion von 2,0 g der Vorstufe mit 1,34 g p-Amidinobenzoylchlorid nach Beispiel 1f) ergibt 1,95 g des erwünschten Acetats.

Beispiel 27

Eine Lösung von 700 mg t-Butyl-[[1-[[1-(p-amidinobenzoyl)-2-piperidinyl]carbonyl]-4-piperidinyl]oxy]-acetat in 20 ml Dichlormethan und 20 ml Trifluoressigsäure wird 3 Stunden bei Raumtemperatur gerührt und eingedampft. Der Rückstand wird in Aethanol gelöst und mit Aether versetzt. Abnutschen und Trocknen des Niederschlags und Chromatographle an Kieselgel (RP-18, Wasser/THF 9:1) ergeben 111 mg [[1-[[1-(p-Amidinobenzoyl)-2-piperidinyl]carbonyl]-4-piperidinyl]oxy]essigsäure. Smp. 233-234 °C.

Das Ausgangsacetat wird wie folgt hergestellt:

a) Die Kupplung von 5,26 g 1-(Benzyloxycarbonyl)-2-piperidincarbonsäure mit 4,30 g 4-Piperidinyloxyessigsäure-t-butylester und Chromatographie an Kieselgel (Essigester/Hexan 2:1) ergibt 7,33 g 2-[[4-[(t-Butoxycarbonyl)methoxy]piperidino]carbonyl]-1-piperidincarbonsäurebenzylester. MS: 461 $(M+H)^+$.

b) Hydrierung von 4,6 g der Vorstufe in Aethanol in Anwesenheit von 0,4 g Pd/C (10%) ergibt nach Abfiltrieren des Katalysators und Einengen des Lösungsmittels 3,2 g t-Butyl-[[1-(2-piperidinylcarbonyl)-4-piperidinyl]oxy]acetat. MS: 327 $(M+H)^+$.

c) Die Reaktion von 3,26 g der Vorstufe mit 2,49 g p-Amidinobenzoylchlorid nach Beispiel 1f) ergibt 1,56 g des erwünschten Acetats, Smp. 93-95 °C.

Beispiel 28

Eine Lösung von 130 mg t-Butyl-[[(1RS,2RS,3RS,4SR)-4-[[N-(p-amidinobenzoyl)-L-alanyl]amino]-2,3-diacetoxycyclohexyl]oxy]acetat-hydrochlorid in 5 ml Dichlormethan und 5 ml Trifluoressigsäure wird 2 Stunden bei Raumtemperatur gerührt und eingeengt. Suspendieren des Rückstandes in Aether und Abnutschen ergibt 126 mg [[(1RS,2RS,3RS,4SR)-4-[[N-(-Amidinobenzoyl)-L-alanyl]amino]-2,3-diacetoxycyclohexyl]oxy]essigsäure-trifluoracetat. MS: 507 $(M+H)^+$.

Das Ausgangsmaterial wird wie folgt hergestellt:

a) Eine Lösung von 4,64 g cis-4-Amino-2-cyclohexen-1-ol, 10,2 g N-(Benzyloxycarbonyloxy)succinimid und 5,7 ml Triäthylamin in DMF wird nach Rühren mit Aether verdünnt, mit gesättigter Natriumchloridlösung gewaschen, getrocknet und eingeengt. Chromatographie des Rückstandes an Kieselgel (Essigester/Hexan 2:1) ergibt 5,62 g Benzyl-(1RS,4SR)-4-hydroxy-2-cyclohexen-1-carbamat. MS: 156 $(M-91)^+$.

b) Unter Phasentransferbedingungen (30 ml Toluol, 30 ml 50%ige Natriumhydroxidlösung, 100 mg Tetrabutylammoniumhydrogensulfat) werden 2,1 g der Vorstufe mit 1,76 ml Bromessigsäure-t-butylester umgesetzt. Nach Rühren wird die organische Phase abgetrennt, mit gesättigter Natriumchloridlösung gewaschen, getrocknet und eingeengt. Chromatographie des Rückstandes an Kieselgel (Hexan/Essigester 3:1) ergibt 1,91 g Benzyl-(1RS,4SR)-4-[(t-butoxycarbonyl)methoxy]-2-cyclohexen-1-carbamat. MS: 333 $(M-28)^+$.

c) Eine Lösung von 722 mg der Vorstufe, 280 mg N-Methylmorpholin-N-oxid und 26 mg Osmiumtetroxid in 20 ml Aceton und 10 ml Wasser wird gerührt und dann das Aceton unter reduziertem Druck entfernt und die wässrige Phase mit Aether extrahiert. Waschen der organischen Phase mit gesättigter Natriumchloridlösung, Trocknen und Einengen ergibt nach Chromatographie an Kieselgel (Essigester/Hexan 2:1) 476 mg Benzyl-(1RS,2SR,3SR,4SR)-4-[(t-butoxycarbonyl)methoxy]-2,3-dihydroxycyclohexancarbamat. MS: 396 $(M+H)^+$.

d) Eine Lösung von 728 mg der Vorstufe in 10 ml Aethanol wird in Anwesenheit von 100 mg 10% Pd/C hydriert. Dann wird der Katalysator abfiltriert, das Filtrat eingedampft und der Rückstand in 30 ml THF in Gegenwart von 697 mg HBTU und 200 µl Triäthylamin mit 410 mg N-Benzyloxycarbonyl-L-alanin gekuppelt. Die Reaktionslösung wird mit Aether verdünnt, mit gesättigter Natriumbicarbonatlösung und gesättigter Natriumchloridlösung gewaschen, getrocknet und eingeengt. Chromatographie an Kieselgel (Essigester/Methanol 95:5) ergibt 521 mg Benzyl-[(S)-1-[((1RS,2SR,3SR,4SR)-4-[(t-butoxycarbonyl)-methoxy]-2,3-dihydroxycyclohexyl]carbamoyl]äthyl]carbamat. MS: 467 $(M+H)^+$.

e) Acetylierung von 800 mg der Vorstufe in 10 ml Acetanhydrid und 10 ml Pyridin und Einengen der Reaktionslösung ergibt nach Chromatographie an Kieselgel (Essigester/Hexan 2:1) 670 mg Benzyl-[(S)-1-[[(1RS,2SR,3SR,4SR)-4-[(t-butoxycarbonyl)methoxy]-2,3-acetoxycyclohexyl]carbamoyl]äthyl]carbamat. MS: 551 $(M+H)^+$.

f) Die Hydrierung von 670 mg der Vorstufe in 10 ml Aethanol in Gegenwart von 100 mg 10% Pd/C, Abfiltrieren des Katalysators und Eindampfen der Lösung ergibt nach Behandlung (analog Beispiel 1f)) mit 329 mg p-Amidinobenzoylchlorid und Chromatographie an Kieselgel (RP-18, Wasser/Methanol 9:1)-230 mg des erwünschten Ausgangsmaterials. MS: 563 $(M+H)^+$.

Beispiel 29

220 mg des Produkts von Beispiel 28 und 300 mg Kaliumcarbonat werden in 10 ml Methanol bei Raumtemperatur gerührt und das Methanol dann abgedampft. Chromatographie an Kieselgel (RP-18, Wasser/Acetonitril 95:5) ergibt 110 mg [[(1RS,2RS,3RS,4SR)-4-[[N-(p-Amidinobenzoyl)-L-alanyl]amino]-2,3-dihydroxycyclohexyl]oxy]essigsäure.

Beispiel 30

Behandlung von 1.3 g rac-[p-[[1-(p-Cyanbenzoyl)-2-pyrrolidinyl]carbonyl]phenoxy]essigsäuremethylester wie in Beispiel 2A)B)C) beschrieben, ergibt nach Chromatographie (silyliertes Kieselgel RP-18, Wasser/Methanol-Gradient) und Umkristallisation aus Aethanol 0.45 g des Acetats von rac-[p-[[1-(p-Amidinobenzoyl)-2-pyrrolidinyl]carbonyl]phenoxy]essigsäuremethylester. Smp. 210-211°C. MS (FAB): 410 $(M+H)^+$.

Das Ausgangsmaterial kann man wie folgt herstellen:

a) Durch Umsetzung des Grignard-Reagenzes aus 8.3 g p-Benzyloxybrombenzol und 0.8 g Magnesiumspänen mit 9.34 g Z-L-Prolin-N-methoxymethylamid in THF isoliert man nach Chromatographie (Kieselgel, Diethyläther/Petroläther 1:1) 4.3 g rac-1-(Benzyloxycarbonyl)-2-(p-benzyloxybenzoyl)pyrrolidin. MS (EI): 211 $(C_{14}H_{11}O_2)^+$, 204 $(C_{12}H_{14}NO_2)^+$.

b) Durch Hydrierung von 3.3 g der Vorstufe, wie in Beispiel 6b) und nachfolgende Umsetzung mit 1.32 g p-Cyanbenzoylchlorid in DMF in Gegenwart von Triethylamin erhält man 2.8 g rac-1-(p-Cyanbenzoyl)-2-(p-hydroxybenzoyl)pyrrolidin. Smp. 194-196°C (Essigester). MS (EI): 320 $(M)^+$.

c) Die Umsetzung von 2.8 g der Vorstufe mit 1.53 g Bromessigsäuremethylester in DMF in Gegenwart von Kaliumcarbonat ergibt nach Chromatographie (Kieselgel, Dichlormethan/Methanol 99:1) 1.3 g des erwünschten Ausgangsmateriais. MS (EI): 392 $(M)^+$.

Beispiel 31

Durch Erhitzen von 0.30 g des Produkts von Beispiel 30 in wässriger Essigsäure erhält man nach Chromatographie (silyliertes Kieselgel RP-18, Wasser/Acetonitril Gradient) 0.11 g rac-[p-[[1-(p-Amidinobenzoyl)-2-pyrrolidinyl]carbonyl]phenoxy]essigsäure. Smp. über 250°C. MS (FAB): 396 $(M+H)^+$.

Beispiel 32

Durch Behandlung von 0.85 g [[4-[1-(p-Cyanbenzoyl)-DL-prolyl]-m-phenylen]dioxy]diessigsäure-dimethylester wie in Beispiel 2A)B)C) beschrieben, erhält man nach Chromatographie (silyliertes Kieselgel RP-18, Wasser/Methanol-Gradient) und Kristallisation aus Diethylether 0.09 g des Acetats von [[4-[1-(p-Amidinobenzoyl)-DL-prolyl]-m-phenylen]dioxy]diessigsäuredimethylester. Smp. 93-95°C. MS (FAB): 498 $(M+H)^+$.

Das Ausgangsmaterial kann man wie folgt herstellen:

a) Durch Umsetzung des Magnesiumsalzes von 4 g 3-Hydroxy-phenoxyessigsäure-methylester mit 5.8 g Z-L-Prolinal und Verätherung des Produktes mit 3.8 g Bromessigsäuremethylester, wie in Beispiel 30c) beschrieben, erhält man nach Chromatographie (Kieselgel, Diethyläther/Petroläther 4:1) 7.6 g [[4-[(RS)-[1-(Benzyloxycarbonyl)-DL-pyrrolyl]hydroxymethyl]-m-phenylen]dioxy]diessigsäuredimethylester, MS (FAB): 488 $(M+H)^+$.

b) Aus 5.3 g der Vorstufe erhält man durch Oxidation mit 7.5 ml Jones-Reagens in Diethyläther nach Chromatographie (Kieselgel, Dichlormethan/Methanol 99:1) 2.2 g [[4-[1-(Benzyloxycarbonyl)-DL-prolyl]-m-phenylen]dioxy]diessigsäuredimethylester. MS (EI): 485 $(M)^+$.

c) Durch Hydrierung von 2.2 g der Vorstufe, wie in Beispiel 6 b) und nachfolgende Umsetzung mit 1.0 g p-Cyanbenzoylchlorid in Chloroform in Gegenwart von Triethylamin erhält man nach Chromatographie (Kieselgel, Dichlormethan/Methanol 99:1) 0.85 g des erwünschten Ausgangsmaterials. MS (EI): 480 $(M)^+$.

Beispiel 33

Aus 0.09 g des Produkts von Beispiel 32 erhält man durch Hydrolyse mit wässriger Natronlauge in Methanol bei 50°C nach Neutralisation mit Essigsäure, Chromatographie (silyliertes Kieselgel RP-18, Wasser/Acetonitril-Gradient) und Kristallisation aus Aethanol 0.09 g des Mononatriumsalzes von [[4-[1-(p-Amidinobenzoyl)-DL-prolyl]-m-phenylen]dioxy]diessigsäure. Smp. 241-242°C. MS (FAB): 492 $(M+Na)^+$, 470 $(M+H)^+$.

Beispiel 34

Aus 0,47g [[1-[N-(p-Amidinobenzoyl)-L-tyrosyl]-4-piperidinyl]oxy]essigsäure (Beispiel 14) erhält man durch Veresterung in Ethanol in Gegenwart katalytischer Mengen konz. Schwefelsäure nach Chromatographie (LiChroprep RP-18, Wasser/Ethanol-Gradient) 0,3 g [[1-[N-(p-Amidinobenzoyl)-L-tyrosyl]-4-piperidinyl]-oxy]essigsäureethylester-hemisulfat, Smp. 182-184°C (Ethanol). MS (ISO = Ionspray): 497 $(M+H)^+$.

Beispiel 35

Aus 0,48 g [[1-[N-[5-(1-t-Butoxyformamido)pentanoyl]-3-(p-t-butoxyphenyl)-L-alanyl]-4-piperidinyl]oxy]-essigsäure-t-butylester erhält man in Analogie zu Beispiel 1 nach Kristallisation aus Diethylether 0,2 g des

Trifluoracetatsalzes von [[1-[N-(5-Aminopentanoyl)-L-tyrosyl]-4-piperidinyl]oxy]essigsäure, Smp. 78-88 °C (Zersetzung). $[\alpha]_D^{20}$ = +11,6° (c = 0,7, Methanol). MS (FAB): 422 $(M+H)^+$.

Den Ausgangsester kann man wie folgt herstellen:

Die Umsetzung von 0,7 g [[1-[3-(p-t-Butoxyphenyl)-L-alanyl]-4-piperdinyl]oxy]essigsäure-t-butylester (hergestellt durch Hydrogenolyse des Produkts von Beispiel 14a) mit 0,35 g N-Boc-5-amino-pentansäure in Gegenwart von HBTU und N-Methylmorpholin (wie in Beispiel 2b) liefert 0,55 g Ausgangsester, $[\alpha]_D^{20}$ = +1,2° (c = 0,4, Methanol). MS (FAB): 634 $(M+H)^+$.

Beispiel 36

Aus 0,6 g [(S)-3-(p-Amidinobenzamido)-3-[[4-[(t-butoxycarbonyl)methoxy]piperidino]carbonyl]propyl]-t-butylcarbamat erhält man in Analogie zu Beispiel 1 nach Chromatographie (LiChroprep RP-18, Wasser/Methanol-Gradient) und Verrühren in THF 0,26 g des Trifluoracetatsalzes von [[1-[(S)-2-(p-Amidinobenzamido)-4-aminobutanoyl]-4-piperidinyl]oxy]essigsäure. Smp. über 170 °C (Zersetzung). $[\alpha]_D^{20}$ = +5,8° (c = 0,5, Wasser). MS (EI): 406 $(M+H)^+$.

Das Ausgangsmaterial kann man wie folgt herstellen:

a) Die Umsetzung von 1,0 g 4-Piperidinyloxyessigsäure-t-butylester mit 2,0 g $N^2$-Fmoc-$N^4$-Boc-(S)-2,4-Diaminobuttersäure in Gegenwart von HBTU und Hünig's Base liefert, wie in Beispiel 2b) beschrieben, nach Chromatographie (Kieselgel, EtOAc/Hexan 1:1,5) 2,2 g 3-t-Butyl-1-(fluoren-9-ylmethyl)-(S)-1-[[4-[(t-butoxycarbonyl)methoxy]piperidino]carbonyl]trimethylen-dicarbamat, MS (FAB): 638 $(M+H)^+$.

b) Die Umsetzung von 2,3 g des Produktes von a) mit Piperidin (20% in DMF) ergibt nach Chromatographie (Kieselgel, Essigester/Methanol 4:1) 0,65 g t-Butyl-[(S)-3-amino-3-[[4-[(t-butoxycarbonyl)methoxy]-piperidino]carbonyl]propyl]carbamat, MS (FAB): 416 $(M+H)^+$.

c) Durch Umsetzung von 0,65 g des Produktes von b) mit 0,38 g p-Amidinobenzoylchlorid-hydrochlorid (wie in Beispiel 1f) gewinnt man 0,6 g Ausgangscarbamat, MS (FAB): 562 $(M+H)^+$.

Beispiel 37

Aus 0,25 g des Acetatsalzes von [[1-[N-[(5-Amidino-2-pyridyl)carbonyl]-3-(p-t-butoxyphenyl)-L-alanyl]-4-piperidinyl]oxy]essigsäure-t-butylester erhält man (wie in Beispiel 1) nach Chromatographie (LiChroprep RP-18, Wasser/Methanol-Gradient) und Verrühren in Essigester 0,12 g [[1-[N-[(5-Amidino-2-pyridyl)-carbonyl]-L-tyrosyl]-4-piperidinyl]oxy]essigsäure, Smp. 198-200 °C (Zersetzung). MS (FAB): 470 $(M+H)^+$.

Das Ausgangsmaterial kann man wie folgt herstellen:

a) Die Umsetzung von 2,5 g [[1-[3-(p-t-Butoxyphenyl)-L-alanyl]-4-piperidinyl]oxy]essigsäure-t-butylester mit 0,85 g 5-Cyan-2-picolinsäure (wie in Beispiel 1d) liefert 1,55 g [[1-[3-(p-t-Butoxyphenyl)-N-[[5-cyano-2-pyridyl]carbonyl]-L-alanyl]-4-piperidinyl]oxy]essigsäure-t-butylester. Smp. 122-123 °C (Diäthyläther/Petroläther 4:1). MS (FAB): 565 $(M+H)^+$.

b) Die aufeinanderfolgende Behandlung von 1,43 g des Produktes der Vorstufe, wie in Beispiel 2A)B)C) beschrieben, liefert 0,98 g des erwünschten Ausgangsmaterials. Smp. 183-186 °C. MS (EI): 582 $(M+H)^+$.

Beispiel 38

Die Umsetzung von 0,7 g (S)-1-[2-(5-Cyanopyridin-2-ylcarbonylamino)-3-(4-methoxyphenyl)propionyl]-piperidin-4-yloxyessigsäureäthylester, wie in Beispiel 2A)B)C) beschrieben, liefert nach Kristallisation aus Wasser 0,1 g des Acetatsalzes von (S)-1-[2-(5-Amidinopyridin-2-ylcarbonylamino)-3-(4-methoxyphenyl)-propionyl]piperidin-4-yloxyessigsäureäthylester. Smp. 180-181 °C (Zersetzung). MS (ISP): 512 $(M+H)^+$.

Das Ausgangsnitril kann man wie folgt herstellen:

a) Die Umsetzung von 7 g N-Z-L-Tyrosin-dihydrat mit 4,5 g 4-Piperidinyloxyessigsäureäthylester [erhalten durch Behandlung des entsprechenden t-Butylesters, Beispiel 1c), mit Trifluoressigsäure, gefolgt von äthanolischer Salzsäure], wie in Beispiel 1d) liefert 6,5 g [[1-[N-(Benzyloxycarbonyl)-L-tyrosyl]-4-piperidinyl]oxy]essigsäureäthylester. Dieses Produkt wird in DMF in Gegenwart von Kaliumcarbonat mit Methyljodid behandelt, wobei man nach Chromatographie (Kieselgel, Methylenchlorid/Methanol 99:1) 4,2 g (S)-1-[2-Benzyloxycarbonylamino-3-(4-methoxyphenyl)propionyl]piperidin-4-yloxyessigsäure-äthylester, $[\alpha]_D^{20}$ = +1,9° (c = 0,8, Methanol). MS (ISP): 499 $(M+H)^+$ erhält.

b) Aus 4 g des Produktes von a) erhält man in Analogie zu Beispiel 1e) 3,5 g (S)-1-[2-Amino-3-(4-methoxyphenyl)propionyl]piperidin-4-yloxyessigsäureäthylester, MS (EI): 365 $(M+H)^+$.

c) Die Kupplung von 1,46 g des Produktes von b) mit 0,74 g 5-Cyan-2-picolinsäure gemäss Beispiel 1d) ergibt nach Chromatographie an Kieselgel (Methylenchlorid/Methanol 40:1) 0,72 g Ausgangsnitril, MS

(ISP): 495,5 $(M+H)^+$.

Beispiel 39

Durch Verseifung von (S)-1-[2-(5-Amidinopyridin-2-ylcarbonylamino)-3-(4-methoxyphenyl)propionyl]-piperidin-4-yloxyessigsäureäthylester (Beispiel 38) bei pH = 12 erhält man nach Chromatographie (LiChroprep RP-18, Wasser/Methanol-Gradient) und Verrühren in Aethanol (S)-1-[2-(5-Amidinopyridin-2-ylcarbonylamino)-3-(4-methoxyphenyl)propionyl]piperidin-4-yloxyessigsäure. Smp. über 250 °C. MS (ISP): 484.4 $(M+H)^+$.

Beispiel 40

Durch Kupplung von 1,2 g (S)-1-[2-Amino-3-(4-methoxyphenyl)propionyl]piperidin-4-yloxyessigsäureäthylester (Beispiel 38b) mit 0,77 g 4-Amidinobenzoesäurechlorid-hydrochlorid in 3-Picolin (analog Beispiel 1f) erhält man nach Chromatographie (LiChroprep RP-18, Wasser/Aethanol-Gradient) und Verrühren mit Essigester 0,25 g des Hydrochlorides von (S)-1-[2-(4-Amidinobenzamido)-3-(4-methoxyphenyl)propionyl]-piperidin-4-yloxyessigsäureäthylester, Smp. 105-107 °C. MS (ISP): 511,3 $(M+H)^+$.

Beispiel 41

Durch Verseifung von 0,35 g des Hydrochlorides von (S)-1-[2-(4-Amidinobenzamido)-3-(4-methoxyphenyl)propionyl]piperidin-4-yloxyessigsäureäthylester (Beispiel 40) bei pH = 12 erhält man nach Chromatographie (LiChroprep RP-18, Wasser/Methanol-Gradient) und Kristallisation aus Aethanol/Wasser 0,05 g (S)-1-[2-(4-Amidinobenzamido)-3-(4-methoxyphenyl)propionyl]piperidin-4-yloxyessigsäure, Smp. 191-192 °C. MS (ISP): 483,3 $(M+H)^+$.

Beispiel 42

Aus 1,6 g [1-[N-(4-Amidinobenzoyl)-L-tryptophanyl]piperidin-4-yloxy]essigsäure-t-butylester erhält man in Analogie zu Beispiel 1 nach Chromatographie (LiChroprep RP-18, Wasser/Methanol-Gradient) und Verrühren mit THF und Acetonitril 0,7 g [1-[N-(4-Amidinobenzoyl)-L-tryptophanyl]piperidin-4-yloxy]-essigsäure, Smp. 210 °C (Zersetzung). MS (ISP): 492,2 $(M+H)^+$.

Der Ausgangsester wird wie folgt hergestellt:

a) Die Umsetzung von 5,1 g 4-Piperidinyloxyessigsäure-t-butylester (Beispiel 1c) mit 8,0 g Z-Trp-OH in Gegenwart von HBTU und N-Methylmorpholin liefert, wie in Beispiel 2b) beschrieben, nach Chromatographie (Kieselgel, Methylenchlorid/Methanol 20:1) 11,5 g [1-(N-Benzyloxycarbonyl-L-tryptophanyl)piperidin-4-yloxy]essigsäure-t-butylester, MS (ISP): 536,0 $(M+H)^+$.

b) Eine Lösung von 6,6 g des Produktes von a) in Methanol wird in Gegenwart von 10 proz. Pd-C und Ammoniumformiat auf Siedetemperatur erhitzt. Nach Filtration und Chromatographie (Kieselgel, Methylenchlorid/Methanol 9:1) erhält man 4,3 g (1-L-Tryptophany-piperidin-4-yloxy)essigsäure-t-butylester. MS (EI): 384 $(M-NH_3)^+$.

c) Durch Umsetzung von 1,9 g des Produktes von b) mit 1,15 g 4-Amidinobenzoesäurechlorid-hydrochlorid in Pyridin, wie in Beispiel 1f) beschrieben, erhält man nach Chromatographie (Kieselgel, Methylenchlorid/Methanol 7:1) 1,6 g Ausgangsester, MS (ISP): 548,3 $(M+H)^+$.

Beispiel 43

Eine Lösung von 3,2 g [1-[N-(4-Amidinobenzoyl)-4'-hexyloxy-L-phenylalanyl]piperidin-4-yloxy]essigsäure-t-butylester in Ameisensäure wird über Nacht bei Raumtemperatur belassen. Nach Einengen, Chromatographie (LiChroprep RP-18, Wasser/Methanol-Gradient) und Verrühren mit Diäthyläther isoliert man 0,45 g [1-[N-(4-Amidinobenzoyl)-4'-hexyloxy-L-phenylalanyl]piperidin-4-yloxy]essigsäure, Smp. 160 °C (Zersetzung). $[\alpha]_D^{20}$ = -3,2 ° (c = 0,5, Methanol). MS (ISP): 553,2 $(M+H)^+$.

Der Ausgangsester wird wie folgt hergestellt:

a) In Analogie zu Beispiel 38a) ergibt die Umsetzung von 5,6 g [[1-[N-(Benzyloxycarbonyl)-L-tyrosyl]-4-piperidinyl]oxy]essigsäure-t-butylester mit 1-Jodhexan bei 80 °C nach Chromatographie (Kieselgel, Hexan/Essigester 2,5:1) 3,9 g [1-(N-Benzyloxycarbonyl-4'-hexyloxy-L-phenylalanyl)piperidin-4-yloxy]-essigsäure-t-butylester, MS (EI): 445 $(M-Z-NH_2)^+$.

b) Durch Hydrierung von 3,9 g des Produktes von a) in Methanol (analog Beispiel 1c) erhält man 2,85 g [1-(4'-Hexyloxy-L-phenylalanyl)piperidin-4-yloxy]essigsäure-t-butylester, MS (EI): 462 $(M)^+$, 445 $(M-NH_3)^+$.

c) Durch Umsetzung von 0,5 g des Produktes von b) mit 0,3 g 4-Amidinobenzoesäurechlorid-hydrochlorid in Pyridin (analog Beispiel 1f) erhält man nach Chromatographie (Kieselgel, Methylenchlorid/Methanol 5:1) 0,7 g Ausgangsester, MS (ISP): 609,4 $(M+H)^+$.

Beispiel 44

Eine Lösung von 0,65 g (R,S)-1-[2-(4-Aminoiminomethyl-N-methylbenzoylamino)-3-(4-methoxyphenyl)-propionyl]piperidln-4-yloxy]essigsäure-t-butylester in Ameisensäure wird über Nacht bei Raumtemperatur belassen. Nach Einengen und Chromatographie (LiChroprep RP-18, Wasser/Acetonitril-Gradient) isoliert man 0,13 g (R,S)-1-[2-(4-Aminoiminomethyl-N-methylbenylamino)-3-(4-methoxyphenyl)propionyl]piperidin-4-yloxy]essigsäure, Smp. 181-182 °C. MS (ISP): 497,1 $(M+H)^+$.

Der Ausgangester wird wie folgt hergestellt:

a) Durch Kupplung von 1,37 g Z-N-Me-Tyr(Me)-OH (J.A.C.S., 112, 1990, 7663) mit 0,86 g 4-Piperidinyloxyessigsäure-t-butylester (Beispiel 1c), wie in Beispiel 2b) beschrieben, erhält man nach Chromatographie (Kieselgel, Diäthyläther/Hexan 5:1) 1,6 g (R,S)-1-[2-(N-Benzyloxycarbonyl-N-methylamino)-3-(4-methoxyphenyl)propionyl]piperidin-4-yloxy]essigsäure-t-butylester, MS (EI): 541,0 $(M+H)^+$.

b) Durch Hydrierung von 1,5 g des Produktes von a) in Methanol, wie in Beispiel 1c) beschrieben, erhält man 1,05 g eines Oeles, das direkt mit 0,58 g 4-Amidinobenzoesäurechlorid-hydrochlorid in Pyridin, wie in Beispiel 1f) beschrieben, umgesetzt wird. Nach Chromatographie (Kieselgel, Methylenchlorid/Methanol 9:1) erhält man 0,7 g Ausgangsester, Smp. 109-111 °C. MS (ISP): 553,2 $(M+H)^+$.

Beispiel 45

Durch Veresterung von 0,07 g (R,S)-1-[2-(4-Aminoiminomethyl-N-methylbenzoylamino)-3-(4-methoxyphenyl)propionyl]piperidin-4-yloxyessigsäure in Aethanol, wie in Beispiel 34 beschrieben, erhält man nach Chromatographie (LiChroprep RP-18, Wasser/Aethanol-Gradient) und Verrühren mit Diäthyläther 0,056 g (R,S)-1-[2-(4-Aminoiminomethyl-N-methylbenzoylamino)-3-(4-methoxyphenyl)propionyl]piperidin-4-yloxyessigsäureäthylester, Smp. 126-128 °C. MS (ISP): 525,5 $(M+H)^+$.

Beispiel 46

Zu 100 mg (S)-cis-1-[2-(4-Amidinobenzoylamino)propionyl]-4-t-butoxycarbonylmethoxy-pyrrolidin-3-yloxyessigsäure-t-butylester in 5 ml Methylenchlorid gibt man 5 ml Trifluoressigsäure. Nach Rühren bei Raumtemperatur werden die Lösungsmittel abgedampft und der Rückstand an Kieselgel RP-18 mit Wasser/THF (0-50%) chromatographiert. Man erhält 73 mg (S)-cis-1-[2-(4-Amidinobenzoylamino)propionyl]-4-carboxymethoxy-pyrrolidin-3-yloxyessigsäure. MS: 437 $(M+H)^+$.

Der Ausgangsester wird wie folgt hergestellt:

a) Unter Phasentransferbedingungen werden 237 mg cis-N-Benzyloxycarbonylpyrrolidin-3,4-diol, 1 mi Bromessigsäure-t-butylester und 100 mg Tetrabutylammoniumhydrogensulfat in 10 ml Toluol mit 10 ml 50%iger Natronlauge gerührt. Die organische Phase wird mit Wasser gewaschen und eingedampft. Chromatographie des Eindampfrückstandes an Kieselgel mit Essigester/Hexan (1:3) ergibt 354 mg cis-3,4-Bis-t-butoxycarbonylmethoxy-pyrrolidin-1-carbonsäure-benzylester. MS: 354 (M-111).

b) Von der Vorstufe werden 320 mg in 10 ml EtOH in Gegenwart von 100 mg 10% Pd/C hydriert, der Katalysator nach 2 Stunden abfiltriert und der Rückstand in 10 ml THF mit 224 mg N-Benzyloxycarbonyl-L-alanin-N-hydroxysuccinimidester in Gegenwart von 100 µl Triäthylamin gerührt. Die Reaktionslösung wird mit Aether verdünnt, die organische Phase mit 1M $KHSO_4$-Lösung gewaschen, getrocknet und eingedampft. Chromatographie des Rückstandes mit Hexan/Essigester (1:1) an Kieselgel ergibt 260 mg (S)-cis-1-(2-Benzyloxycarbonylamino-propionyl)-4-t-butoxycarbonylmethoxy-pyrrolidin-3-yloxyessigsäure-t-butylester. MS: 537 $(M+H)^+$.

c) Von der Vorstufe werden 250 mg in 10 ml EtOH in Gegenwart von 100 mg 10% Pd/C hydriert, der Katalysator nach 4 Stunden abfiltriert und der Rückstand in 10 ml Pyridin mit 102 mg p-Amidinobenzoylchlorid-hydrochlorid gerührt. Eindampfen der Lösung und Chromatographie des Rückstandes an Kieselgel RP-18 mit Wasser/THF (5-30%) ergibt 143 mg Ausgangsester, Smp. 127 °C (d).

Beispiel 47

Eine Lösung von 150 mg (S)-8-[2-(4-Aminoiminomethyl-benzoylamino)-3-(4-t-butoxyphenyl)propionyl]-8-azabicyclo[3.2.1]octan-endo-3-yloxyessigsäureäthylester-hydrochlorid wird in 5 ml $CH_2Cl_2$ und 2,5 ml Trifluoressigsäure bei Raumtemperatur gerührt und eingedampft. Der Rückstand ergibt mit Aether Kristalle, die abgenutscht und in 5 ml EtOH gelöst werden. Zu der Lösung gibt man 40 mg NaOH gelöst in 1 ml Wasser und rührt bei Raumtemperatur. Die Reaktionslösung wird mit 1N Salzsäure neutralisiert und eingedampft. Chromatographie des Rückstandes mit Wasser/THF an Kieselgel RP-18 ergibt 75 mg (S)-8-[2-(4-Aminoiminomethyl-benzoylamino)-3-(4-hydroxyphenyl)propionyl]-8-azabicyclo[3.2.1]octan-endo-3-yloxyessigsäure, MS: 495 $(M+H)^+$.

Der Ausgangsester wird wie folgt hergestellt:

a) Zu einer Lösung von 1 g N-Benzyloxycarbonyl-nortropin und 20 mg Rhodium(II)-acetat in 3 ml Toluol werden bei 80 °C 2 ml Diazoessigsäureäthylester in 2 ml Toluol zugegeben. Nach 3,5 Stunden wird die Lösung eingedampft und der Rückstand mit Hexan/Essigester (20-50%) an Kieselgel chromatographiert. Man erhält 555 mg Endo-3-äthoxycarbonylmethoxy-8-azabicyclo[3.2.1]octan-8-carbonsäure-benzylester. M: 348 $(M+H)^+$.

b) Eine Lösung von 500 mg der Vorstufe in 20 ml EtOH wird in Gegenwart von 100 mg 10% Pd/C hydriert, der Katalysator nach 3 Stunden abfiltriert und das Filtrat eingedampft. Der Rückstand wird in 10 ml THF gelöst und zu einer Lösung von 828 mg N-Z-L-Tyr(tBu)-OH, 140 µl N-Methylmorpholin und 569 mg HBTU in 10 ml THF, die 1 Stunde bei 0 °C gerührt wurde, zugegeben. Nach Rühren wird die Reaktionslösung eingedampft und der Rückstand mit Hexan/Essigester (1:1) an Kieselgel chromatographiert. Man erhält 650 mg (S)-8-[2-Benzyloxycarbonylamino-3-(4-t-butoxyphenyl)propionyl]-8-azabicyclo-[3.2.1]octan-endo-3-yloxyessigsäureäthylester. MS: 567 $(M+H)^+$.

c) Von der Vorstufe werden 600 mg in 20 ml EtOH in Gegenwart von 100 mg 10% Pd/C hydriert, der Katalysator nach 16 Stunden abfiltriert und der Rückstand in 10 ml Pyridin mit 262 mg p-Amidinobenzoylchlorid-hydrochlorid bei Raumtemperatur gerührt. Eindampfen der Lösung und Chromatographie des Rückstandes an Kieselgel RP-18 mit Wasser/THF (0-50%) ergibt 198 mg Ausgangsester, MS: 579 $(M+H)^+$.

Beispiel 48

706 mg (E)- oder (Z)-(S)-[3-[2-[4-(t-Butoxycarbonylimino-di-t-butoxycarbonylaminomethyl)-benzoylamino]propionylamino]propoxy]essigsäurebutylester werden in 1,5 ml Methylenchlorid und 1,5 ml Trifluoressigsäure bei 20 °C gerührt. Nach dem Eindampfen des Lösungsmittels im Vakuum, Abdampfen mit Toluol und Kristallisation aus Acetonitril erhält man 407 mg (S)-[3-[2-[4-(Aminoiminomethyl)-benzoylamino]propionylamino]propoxy]essigsäurebutylester-trifluoracetat (1:1), Smp. 163-165 °C, $[\alpha]_D^{20}$ = +19 ° (c = 0,5 in Methanol).

Das Ausgangsmaterial erhält man auf folgendem Wege:

a) Acrylnitril, Glycolsäurebutylester und Kaliumcarbonat werden auf 60 °C erwärmt. Nach dem Aufarbeiten mit Essigester und Wasser destilliert man den 2-Cyanäthoxyessigsäurebutylester. Sdp. 100-120 °C, 0,3 mmHg (Kugelrohr).

b) Dieser wird in Essigsäure an Pd/C hydriert und das dabei erhaltene Amin mit N-Benzyloxycarbonyl-L-alanin zum (S)-[3-(2-Benzyloxycarbonylaminopropionylamino)propoxy]essigsäurebutylester, Smp. 54-55 °C, $[\alpha]_D^{20}$ = -11,0 ° (c = 0,5 in Methanol) gekoppelt.

c) Durch Hydrierung an Pd/C in Essigsäure erhält man daraus den [3-(2-Aminopropionylamino)propoxy]-essigsäurebutylester, welchem man mit p-[E/Z]-Tri(t-butoxycarbonyl)amidinobenzoesäure zum Ausgangsmaterial koppelt. MS: 707 (27 M+H), $[\alpha]_D^{20}$ = +21,4 ° (c = 0,5 in Methanol).

Beispiel 49

416 mg (S)-[3-[2-[4-(Aminoiminomethyl)benzoylamino]propionylamino]propoxy]essigsäurebutylester werden in 8,3 ml 25-proz. Salzsäure bei 20 °C gerührt. Die Lösung wird eingedampft und der Rückstand mit Wasser abgedampft. Aus THF erhält man 211 mg (S)-[3-[2-[4-(Aminoiminomethyl)benzoylamino]-propionylamino]propoxy]essigsäure-hydrochlorid als Hydrat (1:1), Smp. 89-90 °C, $[\alpha]_D^{20}$ = +23,4 ° (c = 0,5 in Methanol).

Beispiel 50

1 g 1-[N-[4-(t-Butoxycarbonylimino-di-t-butoxycarbonylamino-methyl)benzoyl]-N-(2-methoxyäthyl)-glycyl]piperidin-4-yloxyessigsäure-t-butylester werden in 3,8 ml Methylenchlorid und 3,8 ml Trifluoressigsäure bei 20 °C gerührt. Das Lösungsmittelgemisch wird eingedampft, der Rückstand mit Wasser abgedampft, in Aethylalkohol gelöst und mit methanolischer Ammoniaklösung auf pH 8 eingestellt, worauf die 1-[N-[4-(Aminoiminomethyl)benzoyl]-N-(2-methoxyäthyl)glycyl]piperidin-4-yloxyessigsäure als Hydrat (2:1) auskristallisiert. Smp. >250 °C. MS: 421 (100, M+H).

Der Ausgangsester kann auf folgendem Wege erhalten werden:

a) N-(2-Methoxyäthyl)glycin-t-butylester wird in Aether und gesättigter, wässriger Natriumbicarbonatlösung mit Chlorameisensäurebenzylester zum N-Benzyloxycarbonyl-N-(2-methoxyäthyl)glycin-t-butylester umgesetzt, MS: 324 (82, M+H).

b) Dieser wird in Methylenchlorid/Trifluoressigsäure zum N-Benzyloxycarbonyl-N-(2-methoxyäthyl)glycin gespalten, MS: 267 (1, M).

c) Kopplung des Letzteren mit Piperidin-4-yloxyessigsäure-t-butylester führt zum 1-[N-Benzyloxycarbonyl-N-(2-methoxyäthyl)glycyl]piperidin-4-yloxyessigsäure-t-butylester, MS: 465 (100, M+H).

d) Durch katalytische Hydrierung an Pd/C in Aethanol erhält man daraus den 1-[N-(2-Methoxyäthyl)-glycyl]piperidin-4-yloxyessigsäure-t-butylester, MS: 331 (100, M+H).

e) Dieser wird mit 4-(t-Butoxycarbonylimino-di-t-butoxycarbonylaminomethyl)benzoesäure zum Ausgangsester gekoppelt, MS: 777 (70, M+H).

Beispiel A

Eine Verbindung der Formel I kann man in an sich bekannter Weise als Wirkstoff zur Herstellung von Tabletten folgender Zusammensetzung verwenden:

| | pro Tablette |
|---|---|
| Wirkstoff | 200 mg |
| mikrokristalline Cellulose | 155 mg |
| Maisstärke | 25 mg |
| Talk | 25 mg |
| Hydroxypropylmethylcellulose | 20 mg |
| | 425 mg |

Beispiel B

Eine Verbindung der Formel I kann man in an sich bekannter Weise als Wirkstoff zur Herstellung von Kapseln folgender Zusammensetzung verwenden:

| | pro Kapsel |
|---|---|
| Wirkstoff | 100,0 mg |
| Maisstärke | 20,0 mg |
| Milchzucker | 95,0 mg |
| Talk | 4,5 mg |
| Magnesiumstearat | 0,5 mg |
| | 220,0 mg |

**Patentansprüche**

**Patentansprüche für folgende Vertragsstaaten : BE, CH, DE, DK, FR, GB, IT, LI, LU, MC, NL, PT, SE**

**1.** N-Acyl-$\alpha$-aminosäurederivate der Formel

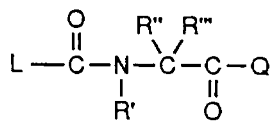

I

worin

L eine Gruppe der Formel

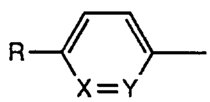

$L^1$

oder

$R^o$-NH($CH_2$)$_t$ $L^2$

R Amidino oder Guanidino,

eins von X und Y CH und das andere CH oder N,

$R^o$ Wasserstoff oder Amidino,

t eine ganze Zahl von 2 bis 6,

R', R'' und R''' Wasserstoff oder in $\alpha$-Aminocarbonsäuren übliche N-Substituenten bzw. Seitenketten sind, wobei in R', R'' und R''' vorkommende Hydroxy- bzw. Carboxygruppen veräthert bzw. verestert oder amidiert, und vorkommende Aminogruppen $C_{1-6}$-alkanoyliert oder aroyliert, sein können,

Q eine Gruppe der Formel

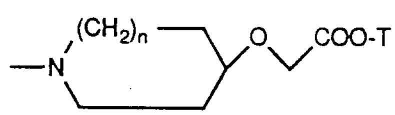

$Q^1$

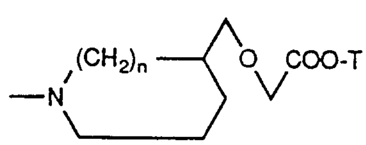

$Q^2$

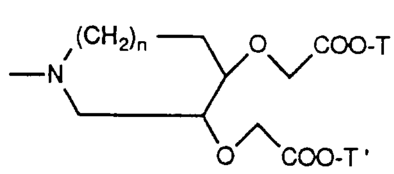

$Q^3$

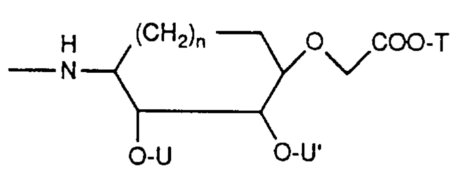

$Q^4$

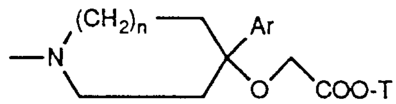

$Q^5$

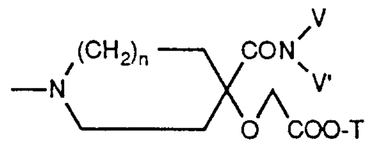

$Q^6$

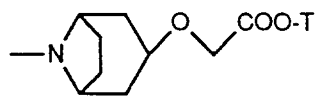

$Q^7$

oder

$-N(V')(CH_2)_v-C(V'',V''')CH_2OCH_2COO-T$ Q8

ist oder, falls R' und R'' zusammen mit dem N- und C-Atom an das sie gebunden sind, einen Ring bilden, auch eine Gruppe der Formel

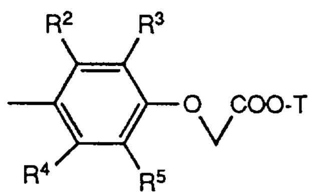

$Q^9$

sein kann,

| | |
|---|---|
| n | die Zahl 0 oder 1, |
| v | eine ganze Zahl von 0 bis 3, |
| T und T' | Wasserstoff oder unter physiologischen Bedingungen spaltbares nieder-Alkyl oder Phenyl-nieder-alkyl, |
| V bis V''' | Wasserstoff oder nieder-Alkyl, |
| U und U' | Wasserstoff, $C_{1-6}$-Alkanoyl oder Aroyl, |
| Ar | Aryl, und |
| $R^2$ bis $R^5$ | Wasserstoff, nieder-Alkyl, nieder-Alkoxy, Halogen oder eine Gruppe $-OCH_2COO-T'$ sind, oder |
| $R^2$ und $R^3$ | zusammen mit der Phenylgruppe, an die sie gebunden sind, eine 1-Naphthylgruppe bilden, wobei "nieder" Gruppen mit 1-6 C-Atomen bezeichnet, |

sowie Hydrate oder Solvate und physiologisch verwendbare Salze davon.

**2.** Verbindungen nach Anspruch 1, worin L eine Gruppe $L^1$,

| | |
|---|---|
| R', R'' und R''' | Wasserstoff oder in α-Aminocarbonsäuren übliche N-Substituenten bzw. Seffenketten sind, wobei in R', R'' und R''' vorkommende Hydroxy- bzw. Carboxygruppen veräthert bzw. verestert oder amidiert sein können, und |
| T | in der Gruppe Q Wasserstoff oder unter physiologischen Bedingungen spaltbares nieder-Alkyl ist. |

**3.** Verbindungen nach Anspruch 1 oder 2, worin L eine Gruppe $L^1$, R Amidino, X CH, Y CH oder N, und Q eine Gruppe $Q^1$, $Q^2$, $Q^4$, $Q^5$ oder $Q^9$ ist.

**4.** Verbindungen nach Anspruch 1, 2 oder 3, worin Q eine Gruppe $Q^1$, insbesondere worin n = 1 und T Wasserstoff oder Methyl, und -N(R')C(R'',R''')CO- einer der Reste Gly, Ala, D-Ala, Val, Leu, Sar, Orn, Lys, Phg, 2-Methyl-Pro, Phe, Tyr 3-Jod-Tyr, 3,5-Dijod-Tyr, Ser(Ac), Ser, Asp, Glu, Pro, 4-Benzyloxy-Pro, 4-Hydroxy-Pro, 2-Piperidylencarbonyl, $NHCH(CH_2CH_2NH_2)CO$, Trp, Tyr(Me), Tyr(Hexyl) und O,N-$(Me)_2$-Tyr ist.

**5.** Verbindungen nach Anspruch 1, 2 oder 3, worin Q eine Gruppe $Q^2$, insbesondere worin n = 1 ist, T Wasserstoff und -N(R')C(R'',R''')CO- der Rest Ala ist.

**6.** Verbindungen nach Anspruch 1, 2 oder 3, worin Q eine Gruppe $Q^4$, insbesondere diejenigen worin n = 1 ist, T Wasserstoff, U und U' Wasserstoff oder Ac, und -N(R')C(R'',R''')CO- der Rest Ala sind.

**7.** Verbindungen nach Anspruch 1, 2 oder 3, worin Q eine Gruppe $Q^5$, insbesondere worin n = 1, T Wasserstoff, Ar $\alpha,\alpha,\alpha$-Trifluor-m-tolyl und -N(R')C(R'',R''')CO- der Rest Ala ist.

**8.** Verbindungen nach Anspruch 1, 2 oder 3, worin Q eine Gruppe $Q^9$, insbesondere worin $R^2$ bis $R^5$ Wasserstoff, oder $R^2$ die Gruppe $-OCH_2COO$(H oder Methyl), T Wasserstoff oder Methyl und -N(R')C-(R'',R''')CO- der Rest Pro ist.

**9.** Verbindungen nach Anspruch 1 oder 3 aus der Gruppe von
[[1-[N-(p-Amidinobenzoyl)-L-alanyl]-4-piperidinyl]oxy]essigsäure,
[[1-[N-[(5-Amidino-2-pyridyl)carbonyl]-L-alanyl]-4-piperidinyl]oxy]essigsäure,
[[1-[N-(p-Amidinobenzoyl)-3-(4-hydroxy-3-jodphenyl)-L-alanyl]-4-piperidinyl]oxy]essigsäure,
[[1-[3-Acetoxy-N-(p-amidinobenzoyl)-L-alanyl]-4-piperidinyl]oxy]essigsäure,
[p-[[1-(p-Amidinobenzoyl)-2-pyrrolidinyl]carbonyl]phenoxy]essigsäure,
[[1-[N-[(5-Amidino-2-pyridyl)carbonyl]-L-tyrosyl]-4-piperidinyl]oxy]essigsäure und insbesondere
[[1-[N-(p-Amidinobenzoyl)-L-tyrosyl]-4-piperidinyl]oxy]essigsäure.

**10.** Verbindungen nach Anspruch 1, worin Q eine Gruppe $Q^3$, insbesondere worin n = 0 und T Wasserstoff, oder eine Gruppe $Q^7$, insbesondere worin T Wasserstoff ist.

**11.** Verbindungen nach Anspruch 1, worin Q eine Gruppe $Q^8$, insbesondere worin v = 1, T Wasserstoff oder Butyl und V' bis V''' Wasserstoff sind.

**12.** Verbindungen nach Anspruch 1, worin -N(R')C(R'',R''')CO- der Rest N(Methoxyäthyl)Gly ist.

**13.** Verbindungen nach Anspruch 1 aus der Gruppe von:
(S)-1-[2-(5-Amidinopyridin-2-ylcarbonylamino)-3-(4-methoxyphenyl)propionyl]piperidin-4-yloxyessigsäure,
(S)-1-[2-(4-Amidinobenzamido)-3-(4-methoxyphenyl)propionyl]piperidin-4-yloxyessigsäureäthylester,
(S)-1-[2-(4-Amidinobenzamido)-3-(4-methoxyphenyl)propionyl]piperidin-4-yloxyessigsäure,
[1-[N-(4-Amidinobenzoyl)-4'-hexyloxy-L-phenylalanyl]piperidin-4-yloxy]essigsäure.

**14.** Verbindungen der Formel

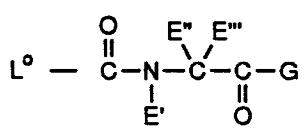

II

oder

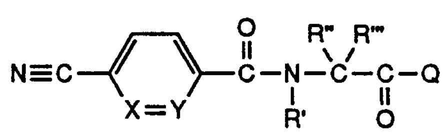

III

worin

L° eine Gruppe der Formel

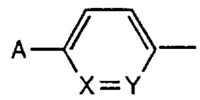

$L^{o1}$

oder

$R^{o1}-(CH_2)_t$ $L^{o2}$

ist, in der A eine gegebenenfalls geschützte Amidino- oder Guanidinogruppe,

$R^{o1}$ eine gegebenenfalls geschützte Amino- oder Guanidinogruppe,

E', E'', E''' und G die gleiche Bedeutung wie R', R'', R''' bzw. Q in der Formel I haben, wobei, falls $R^{o1}$ Amino oder Guanidino, oder falls A Amidino oder Guanidino ist, zumindest eins von E', E'', E''' und G zumindest eine Carbonsäureestergruppe und/oder Aethergruppe und/oder geschützte Aminogruppe enthält.

**15.** Verbindungen nach einem der Ansprüche 1-13 zur Verwendung als pharmazeutische Wirkstoffe.

**16.** Verfahren zur Herstellung der Verbindungen nach einem der Ansprüche 1-13, dadurch gekennzeichnet, dass man

a) in einer Verbindung der Formel

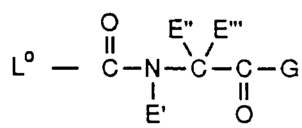

II

worin

$L^o$ eine Gruppe der Formel

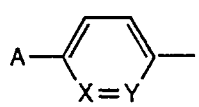

$L^{o1}$

oder

$R^{o1}-(CH_2)_t$ $L^{o2}$

ist, in der A eine gegebenenfalls geschützte Amidino- oder Guanidinogruppe,

$R^{o1}$ eine gegebenenfalls geschützte Amino- oder Guanidinogruppe,

E', E'', E''' und G die gleiche Bedeutung wie R', R'', R''' bzw. Q in der Formel I haben, wobei, falls $R^{o1}$ Amino oder Guanidino, oder falls A Amidino oder Guanidino ist, zumindest eins von E', E'', E''' und G zumindest eine Carbonsäureestergruppe und/oder Aethergruppe und/oder geschützte Aminogruppe enthält,

eine Aethergruppe bzw. eine geschützte Amino-, Amidino- bzw. Guanidinogruppe bzw. einen Carbonsäureester spaltet, oder

b) in einem Nitril der Formel

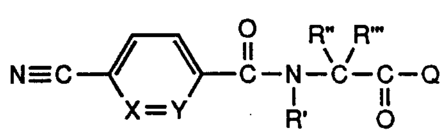

III

die Cyangruppe in die Amidinogruppe überführt, oder

c) ein Amin der Formel

R'-NHC(R'',R''')CO-Q IV

mit einer Säure der Formel $L^1$-COOH oder einem reaktionsfähigen Derivat davon umsetzt, und
d) gewünschtenfalls eine in einer Verbindung der Formel I enthaltene reaktionsfähige Gruppe funktionell abwandelt, und
e) gewünschtenfalls eine Verbindung der Formel I in ein physiologisch verträgliches Satz überführt oder ein Satz einer Verbindung der Formel I in die freie Säure oder Base überführt.

**17.** Pharmazeutische Präparate, insbesondere für die Behandlung oder Prophylaxe von Krankheiten, die durch die Bindung von adhäsiven Proteinen an Blutplättchen, sowie durch die Blutplättchenaggregation und die Zell-Zell-Adhäsion verursacht sind, enthaltend eine Verbindung nach einem der Ansprüche 1-13 als Wirkstoff.

**18.** Verwendung einer Verbindung nach einem der Ansprüche 1-13 zur Herstellung von Arzneimitteln zur Behandlung oder Prophylaxe von Krankheiten, die durch die Bindung von adhäsiven Proteinen an Blutplättchen, sowie durch die Blutplättchenaggregation und die Zell-Zell-Adhäsion verursacht sind, insbesondere zur Behandlung oder Prophylaxe von Blutplättchenthromben, Thrombose, Hirnschlag, Herzinfarkt, Entzündung, Arteriosklerose oder Osteoporose, oder als Antitumor-Mittel oder als Mittel für die Wundheilung.

**Patentansprüche für folgenden Vertragsstaat : ES**

**1.** Verfahren zur Herstellung von N-Acyl-α-aminosäurederivaten der Formel

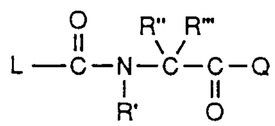

I

worin

L eine Gruppe der Formel

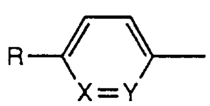

$L^1$

oder

$R^o$-NH$(CH_2)_t$ $L^2$

R Amidino oder Guanidino,

eins von X und Y CH und das andere CH oder N,

$R^o$ Wasserstoff oder Amidino,

t eine ganze Zahl von 2 bis 6,

R', R'' und R''' Wasserstoff oder in α-Aminocarbonsäuren übliche N-Substituenten bzw. Seitenketten sind, wobei in R', R'' und R''' vorkommende Hydroxy- bzw. Carboxygruppen veräthert bzw. verestert oder amidiert, und vorkommende Aminogruppen $C_{1-6}$-alkanoyliert oder aroyliert, sein können,

Q eine Gruppe der Formel

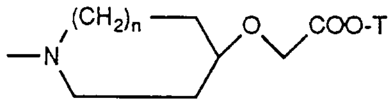

Q[1]

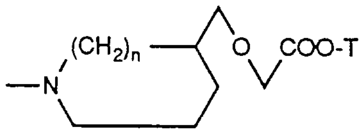

Q[2]

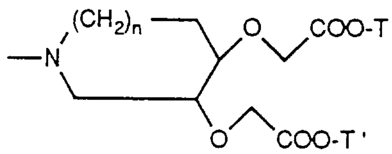

Q[3]

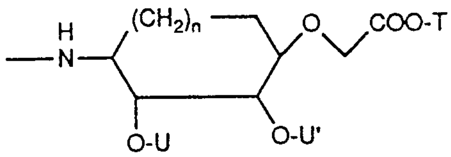

Q[4]

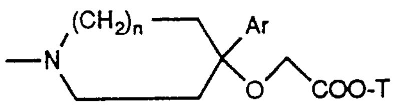

Q[5]

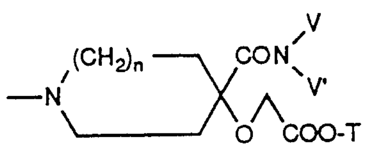

Q[6]

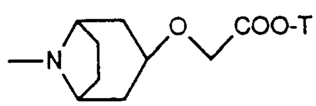

Q[7]

oder

$-N(V')(CH_2)_v-C(V'',V''')CH_2OCH_2COO-T$ Q8

ist oder, falls R' und R'' zusammen mit dem N- und C-Atom an das sie gebunden sind, einen Ring bilden, auch eine Gruppe der Formel

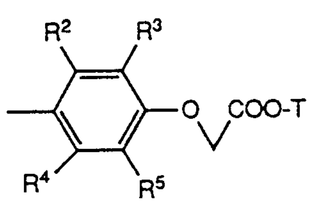

Q[9]

sein kann,

n die Zahl 0 oder 1,

v eine ganze Zahl von 0 bis 3,
T und T' Wasserstoff oder unter physiologischen Bedingungen spaltbares nieder-Alkyl oder Phenyl-nieder-alkyl,
V bis V''' Wasserstoff oder nieder-Alkyl,
U und U' Wasserstoff, $C_{1-6}$-Alkanoyl oder Aroyl,
Ar Aryl, und
$R^2$ bis $R^5$ Wasserstoff, nieder-Alkyl, nieder-Alkoxy, Halogen oder eine Gruppe $-OCH_2COO-T'$ sind, oder
$R^2$ und $R^3$ zusammen mit der Phenylgruppe, an die sie gebunden sind, eine 1-Naphthylgruppe bilden, wobei "nieder" Gruppen mit 1-6 C-Atomen bezeichnet,

sowie von Hydraten oder Solvaten und physiologisch verwendbaren Salzen davon, dadurch gekennzeichnet, dass man

a) in einer Verbindung der Formel

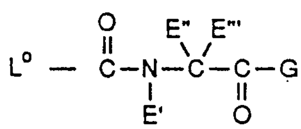 II

worin
$L^o$ eine Gruppe der Formel

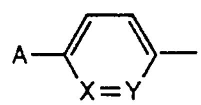 $L^{o1}$

oder

$R^{o1}-(CH_2)_t$ $L^{o2}$

ist, in der A eine gegebenenfalls geschützte Amidino- oder Guanidinogruppe,
$R^{o1}$ eine gegebenenfalls geschützte Amino- oder Guanidinogruppe,
E', E'', E''' und G die gleiche Bedeutung wie R', R'', R''' bzw. Q in der Formel I haben, wobei, falls $R^{o1}$ Amino oder Guanidino, oder falls A Amidino oder Guanidino ist, zumindest eins von E', E'', E''' und G zumindest eine Carbonsäureestergruppe und/oder Aethergruppe und/oder geschützte Aminogruppe enthält,

eine Aethergruppe bzw. eine geschützte Amino-, Amidino- bzw. Guanidinogruppe bzw. einen Carbonsäureester spaltet, oder

b) in einem Nitril der Formel

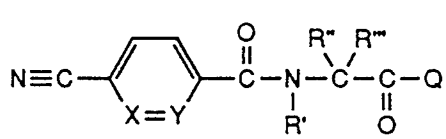 III

die Cyangruppe in die Amidinogruppe überführt, oder

c) ein Amin der Formel

R'-NHC(R'',R''')CO-Q IV

mit einer Säure der Formel $L^1$-COOH oder einem reaktionsfähigen Derivat davon umsetzt, und

d) gewünschtenfalls eine in einer Verbindung der Formel I enthaltene reaktionsfähige Gruppe funktionell abwandelt, und

e) gewünschtenfalls eine Verbindung der Formel I in ein physiologisch verträgliches Salz überführt oder ein Salz einer Verbindung der Formel I in die freie Säure oder Base überführt.

**2.** Verfahren nach Anspruch 1, worin L eine Gruppe $L^1$,

| | |
|---|---|
| R', R'' und R''' | Wasserstoff oder in α-Aminocarbonsäuren übliche N-Substituenten bzw. Seitenketten sind, wobei in R', R'' und R''' vorkommende Hydroxy- bzw. Carboxygruppen veräthert bzw. verestert oder amidiert sein können, und |
| T | in der Gruppe Q Wasserstoff oder unter physiologischen Bedingungen spaltbares nieder-Alkyl ist. |

**3.** Verfahren nach Anspruch 1 oder 2, worin L eine Gruppe $L^1$, R Amidino, X CH, Y CH oder N, und Q eine Gruppe $Q^1$, $Q^2$, $Q^4$, $Q^5$ oder $Q^9$ ist.

**4.** Verfahren nach Anspruch 1, 2 oder 3, worin Q eine Gruppe $Q^1$, insbesondere worin n = 1 und T Wasserstoff oder Methyl, und -N(R')C(R'',R''')CO- einer der Reste Gly, Ala, D-Ala, Val, Leu, Sar, Orn, Lys, Phg, 2-Methyl-Pro, Phe, Tyr 3-Jod-Tyr, 3,5-Dijod-Tyr, Ser(Ac), Ser, Asp, Glu, Pro, 4-Benzyloxy-Pro, 4-Hydroxy-Pro, 2-Piperidylencarbonyl, $NHCH(CH_2CH_2NH_2)CO$, Trp, Tyr(Me), Tyr(Hexyl) und O,N-$(Me)_2$-Tyr ist.

**5.** Verfahren nach Anspruch 1, 2 oder 3, worin Q eine Gruppe $Q^2$, insbesondere worin n = 1 ist, T Wasserstoff und -N(R')C(R'',R''')CO- der Rest Ala ist.

**6.** Verfahren nach Anspruch 1, 2 oder 3, worin Q eine Gruppe $Q^4$, insbesondere diejenigen worin n = 1 ist, T Wasserstoff, U und U' Wasserstoff oder Ac, und -N(R')C(R'',R''')CO- der Rest Ala sind.

**7.** Verfahren nach Anspruch 1, 2 oder 3, worin Q eine Gruppe $Q^5$, insbesondere worin n = 1, T Wasserstoff, Ar α,α,α,-Trifluor-m-tolyl und -N(R')C(R'',R''')CO- der Rest Ala ist.

**8.** Verfahren nach Anspruch 1, 2 oder 3, worin Q eine Gruppe $Q^9$, insbesondere worin $R^2$ bis $R^5$ Wasserstoff, oder $R^2$ die Gruppe $-OCH_2COO$(H oder Methyl), T Wasserstoff oder Methyl und -N(R')C-(R'',R''')CO- der Rest Pro ist.

**9.** Verfahren nach Anspruch 1 oder 3 zur Herstellung einer Verbindung aus der Gruppe von
[[1-[N-(p-Amidinobenzoyl)-L-alanyl]-4-piperidinyl]oxy]essigsäure,
[[1-[N-[(5-Amidino-2-pyridyl)carbonyl]-L-alanyl]-4-piperidinyl]oxy]essigsäure,
[[1-[N-(p-Amidinobenzoyl)-3-(4-hydroxy-3-jodphenyl)-L-alanyl]-4-piperidinyl]oxy]essigsäure,
[[1-[3-Acetoxy-N-(p-amidinobenzoyl)-L-alanyl]-4-piperidinyl]oxy]essigsäure,
[p-[[1-(p-Amidinobenzoyl)-2-pyrrolidinyl]carbonyl]phenoxy]essigsäure,
[[1-[N-[(5-Amidino-2-pyridyl)carbonyl]-L-tyrosyl]-4-piperidinyl]oxy]essigsäure und insbesondere
[[1-[N-(p-Amidinobenzoyl)-L-tyrosyl]-4-piperidlnyl]oxy]essigsäure.

**10.** Verfahren nach Anspruch 1, worin Q eine Gruppe $Q^3$, insbesondere worin n = 0 und T Wasserstoff, oder eine Gruppe $Q^7$, insbesondere worin T Wasserstoff ist.

**11.** Verfahren nach Anspruch 1, worin Q eine Gruppe $Q^8$, insbesondere worin v = 1, T Wasserstoff oder Butyl und V' bis V''' Wasserstoff sind.

**12.** Verfahren nach Anspruch 1, worin -N(R')C(R'',R''')CO- der Rest N(Methoxyäthyl)Gly ist.

**13.** Verfahren nach Anspruch 1 zur Herstellung einer Verbindung aus der Gruppe von:
(S)-1-[2-(5-Amidinopyridin-2-ylcarbonylamino)-3-(4-methoxyphenyl)propionyl]piperidin-4-yloxyessigsäure,
(S)-1-[2-(4-Amidinobenzamido)-3-(4-methoxyphenyl)propionyl]piperidin-4-yloxyessigsäureäthylester,
(S)-1-[2-(4-Amidinobenzamido)-3-(4-methoxyphenyl)propionyl]piperidin-4-yloxyessigsäure,
[1-[N-(4-Amidinobenzoyl)-4'-hexyloxy-L-phenylalanyl]piperidin-4-yloxy]essigsäure.

**14.** Verwendung einer Verbindung nach einem der Ansprüche 1-13 zur Herstellung von Arzneimitteln zur Behandlung oder Prophylaxe von Krankheiten, die durch die Bindung von adhäsiven Proteinen an

Blutplättchen, sowie durch die Blutplättchenaggregation und die Zell-Zell-Adhäsion verursacht sind, insbesondere zur Behandlung oder Prophylaxe von Blutplättchenthromben, Thrombose, Hirnschlag, Herzinfarkt, Entzündung, Arteriosklerose oder Osteoporose, oder als Antitumor-Mittel oder als Mittel für die Wundheilung.

**Patentansprüche für folgenden Vertragsstaat : GR**

**1.** Verfahren zur Herstellung von N-Acyl-α-aminosäurederivaten der Formel

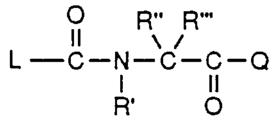

I

worin

L eine Gruppe der Formel

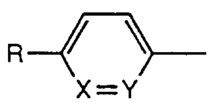

$L^1$

oder

$R^o$-$NH(CH_2)_t$ $L^2$

R Amidino oder Guanidino,

eins von X und Y CH und das andere CH oder N,

$R^o$ Wasserstoff oder Amidino,

t eine ganze Zahl von 2 bis 6,

R', R'' und R''' Wasserstoff oder in α-Aminocarbonsäuren übliche N-Substituenten bzw. Seitenketten sind, wobei in R', R'' und R''' vorkommende Hydroxy- bzw. Carboxygruppen veräthert bzw. verestert oder amidiert, und vorkommende Aminogruppen $C_{1-6}$-alkanoyliert oder aroyliert, sein können,

Q eine Gruppe der Formel

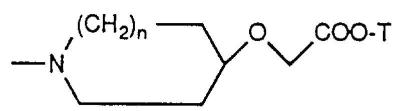

$Q^1$

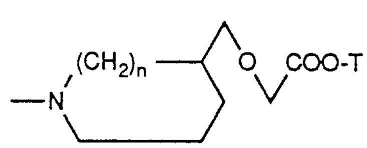

$Q^2$

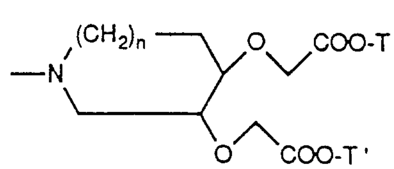

$Q^3$

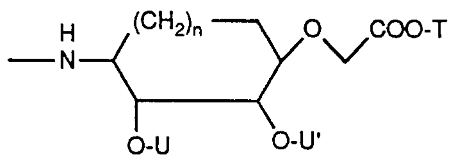

$Q^4$

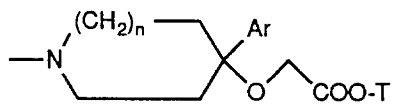

$Q^5$

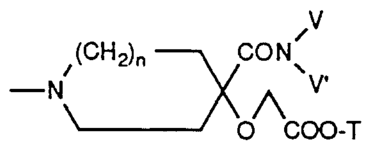

$Q^6$

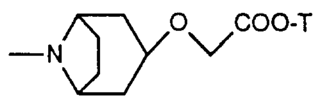

$Q^7$

oder

$-N(V')(CH_2)_v-C(V'',V''')CH_2OCH_2COO-T$ Q8

ist oder, falls R' und R'' zusammen mit dem N- und C-Atom an das sie gebunden sind, einen Ring bilden, auch eine Gruppe der Formel

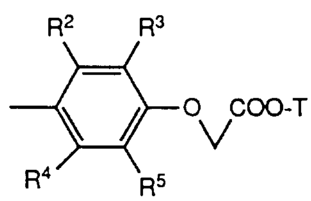

$Q^9$

sein kann,

| | |
|---|---|
| n | die Zahl 0 oder 1, |
| v | eine ganze Zahl von 0 bis 3, |
| T und T' | Wasserstoff oder unter physiologischen Bedingungen spaltbares nieder-Alkyl oder Phenyl-nieder-alkyl, |
| V bis V''' | Wasserstoff oder nieder-Alkyl, |
| U und U' | Wasserstoff, $C_{1-6}$-Alkanoyl oder Aroyl, |
| Ar | Aryl, und |
| $R^2$ bis $R^5$ | Wasserstoff, nieder-Alkyl, nieder-Alkoxy, Halogen oder eine Gruppe $-OCH_2COO-T'$ sind, oder |
| $R^2$ und $R^3$ | zusammen mit der Phenylgruppe, an die sie gebunden sind, eine 1-Naphthylgruppe bilden, wobei "nieder" Gruppen mit 1-6 C-Atomen bezeichnet, |

sowie von Hydraten oder Solvaten und physiologisch verwendbaren Salzen davon, dadurch gekennzeichnet, dass man

a) in einer Verbindung der Formel

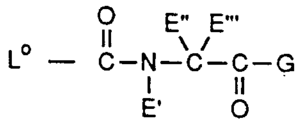

II

worin

L° eine Gruppe der Formel

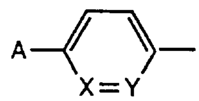

$L^{o1}$

oder

$R^{o1}$-$(CH_2)_t$ $L^{o2}$

ist, in der A eine gegebenenfalls geschützte Amidino- oder Guanidinogruppe,

$R^{o1}$ eine gegebenenfalls geschützte Amino- oder Guanidinogruppe,

E', E'', E''' und G die gleiche Bedeutung wie R', R'', R''' bzw. Q in der Formel I haben, wobei, falls $R^{o1}$ Amino oder Guanidino, oder falls A Amidino oder Guanidino ist, zumindest eins von E', E'', E''' und G zumindest eine Carbonsäureestergruppe und/oder Aethergruppe und/oder geschützte Aminogruppe enthält,

eine Aethergruppe bzw. eine geschützte Amino-, Amidino- bzw. Guanidinogruppe bzw. einen Carbonsäureester spaltet, oder

b) in einem Nitril der Formel

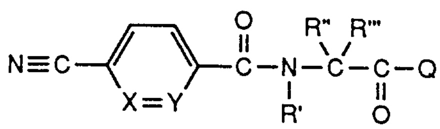

III

die Cyangruppe in die Amidinogruppe überführt, oder

c) ein Amin der Formel

R'-NHC(R'',R''')CO-Q IV

mit einer Säure der Formel $L^1$-COOH oder einem reaktionsfähigen Derivat davon umsetzt, und

d) gewünschtenfalls eine in einer Verbindung der Formel I enthaltene reaktionsfähige Gruppe funktionell abwandelt, und

e) gewünschtenfalls eine Verbindung der Formel I in ein physiologisch verträgliches Salz überführt oder ein Salz einer Verbindung der Formel I in die freie Säure oder Base überführt.

**2.** Verfahren nach Anspruch 1, worin L eine Gruppe $L^1$,

R', R'' und R''' Wasserstoff oder in α-Aminocarbonsäuren übliche N-Substituenten bzw. Seitenketten sind, wobei in R', R'' und R''' vorkommende Hydroxy- bzw. Carboxygruppen veräthert bzw. verestert oder amidiert sein können, und

T in der Gruppe Q Wasserstoff oder unter physiologischen Bedingungen spaltbares nieder-Alkyl ist.

**3.** Verfahren nach Anspruch 1 oder 2, worin L eine Gruppe $L^1$, R Amidino, X CH, Y CH oder N, und Q eine Gruppe $Q^1$, $Q^2$, $Q^4$, $Q^5$ oder $Q^9$ ist.

4. Verfahren nach Anspruch 1, 2 oder 3, worin Q eine Gruppe $Q^1$, insbesondere worin n = 1 und T Wasserstoff oder Methyl, und -N(R')C(R'',R''')CO- einer der Reste Gly, Ala, D-Ala, Val, Leu, Sar, Orn, Lys, Phg, 2-Methyl-Pro, Phe, Tyr 3-Jod-Tyr, 3,5-Dijod-Tyr, Ser(Ac), Ser, Asp, Glu, Pro, 4-Benzyloxy-Pro, 4-Hydroxy-Pro, 2-Piperidylencarbonyl, $NHCH(CH_2CH_2NH_2)CO$, Trp, Tyr(Me), Tyr(Hexyl) und O,N-$(Me)_2$-Tyr ist.

5. Verfahren nach Anspruch 1, 2 oder 3, worin Q eine Gruppe $Q^2$, insbesondere worin n = 1 ist, T Wasserstoff und -N(R')C(R'',R''')CO- der Rest Ala ist.

6. Verfahren nach Anspruch 1, 2 oder 3, worin Q eine Gruppe $Q^4$, insbesondere diejenigen worin n = 1 ist, T Wasserstoff, U und U' Wasserstoff oder Ac, und -N(R')C(R'',R''')CO- der Rest Ala sind.

7. Verfahren nach Anspruch 1, 2 oder 3, worin Q eine Gruppe $Q^5$, insbesondere worin n = 1, T Wasserstoff, Ar $\alpha,\alpha,\alpha$-Trifluor-m-tolyl und -N(R')C(R'',R''')CO- der Rest Ala ist.

8. Verfahren nach Anspruch 1, 2 oder 3, worin Q eine Gruppe $Q^9$, insbesondere worin $R^2$ bis $R^5$ Wasserstoff, oder $R^2$ die Gruppe $-OCH_2COO$(H oder Methyl), T Wasserstoff oder Methyl und -N(R')C-(R'',R''')CO- der Rest Pro ist.

9. Verfahren nach Anspruch 1 oder 3 zur Herstellung einer Verbindung aus der Gruppe von
   [[1-[N-(p-Amidinobenzoyl)-L-alanyl]-4-piperidinyl]oxy]essigsäure,
   [[1-[N-[(5-Amidino-2-pyridyl)carbonyl]-L-alanyl]-4-piperidinyl]oxy]essigsäure,
   [[1-[N-(p-Amidinobenzoyl)-3-(4-hydroxy-3-jodphenyl)-L-alanyl]-4-piperidinyl]oxy]essigsäure,
   [[1-[3-Acetoxy-N-(p-amidinobenzoyl)-L-alanyl]-4-piperidinyl]oxy]essigsäure,
   [p-[[1-(p-Amidinobenzoyl)-2-pyrrolidinyl]carbonyl]phenoxy]essigsäure,
   [[1-[N-[(5-Amidino-2-pyridyl)carbonyl]-L-tyrosyl]-4-piperidinyl]oxy]essigsäure und insbesondere
   [[1-[N-(p-Amidinobenzoyl)-L-tyrosyl]-4-piperidinyl]oxy]essigsäure.

10. Verfahren nach Anspruch 1, worin Q eine Gruppe $Q^3$, insbesondere worin n = 0 und T Wasserstoff, oder eine Gruppe $Q^7$, insbesondere worin T Wasserstoff ist.

11. Verfahren nach Anspruch 1, worin Q eine Gruppe $Q^8$, insbesondere worin v = 1, T Wasserstoff oder Butyl und V' bis V''' Wasserstoff sind.

12. Verfahren nach Anspruch 1, worin -N(R')C(R'',R''')CO- der Rest N(Methoxyäthyl)Gly ist.

13. Verfahren nach Anspruch 1 zur Herstellung einer Verbindung aus der Gruppe von:
   (S)-1-[2-(5-Amidinopyridin-2-ylcarbonylamino)-3-(4-methoxyphenyl)propionyl]piperidin-4-yloxyessigsäure,
   (S)-1-[2-(4-Amidinobenzamido)-3-(4-methoxyphenyl)propionyl]piperidin-4-yloxyessigsäureäthylester,
   (S)-1-[2-(4-Amidinobenzamido)-3-(4-methoxyphenyl)propionyl]piperidin-4-yloxyessigsäure,
   [1-[N-(4-Amidinobenzoyl)-4'-hexyloxy-L-phenylalanyl]piperidin-4-yloxy]essigsäure.

14. Verfahren zur Herstellung von pharmazeutischen Präparaten, insbesondere für die Behandlung oder Prophylaxe von Krankheiten, die durch die Bindung von adhäsiven Proteinen an Blutplättchen, sowie durch die Blutplättchenaggregation und die Zell-Zell-Adhäsion verursacht sind, dadurch gekennzeichnet, dass man eine Verbindung nach einem der Ansprüche 1-13 als Wirkstoff in eine galenische Form bringt.

15. Verwendung einer Verbindung nach einem der Ansprüche 1-13 zur Herstellung von Arzneimitteln zur Behandlung oder Prophylaxe von Krankheiten, die durch die Bindung von adhäsiven Proteinen an Blutplättchen, sowie durch die Blutplättchenaggregation und die Zell-Zell-Adhäsion verursacht sind, insbesondere zur Behandlung oder Prophylaxe von Blutplättchenthromben, Thrombose, Hirnschlag, Herzinfarkt, Entzündung, Arteriosklerose oder Osteoporose, oder als Antitumor-Mittel oder als Mittel für die Wundheilung.

**16.** Verbindungen der Formel

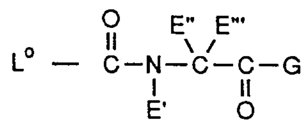 II

oder

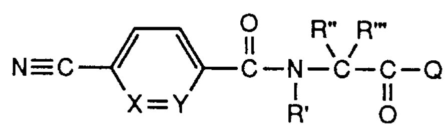 III

worin

$L^o$ eine Gruppe der Formel

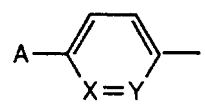 $L^{o1}$

oder

$R^{o1}$-$(CH_2)_t$ $L^{o2}$

ist, in der A eine gegebenenfalls geschützte Amidino- oder Guanidinogruppe,

$R^{o1}$ eine gegebenenfalls geschützte Amino- oder Guanidinogruppe,

E', E'', E''' und G die gleiche Bedeutung wie R', R'', R''' bzw. Q in der Formel I haben, wobei, falls $R^{o1}$ Amino oder Guanidino, oder falls A Amidino oder Guanidino ist, zumindest eins von E', E'', E''' und G zumindest eine Carbonsäureestergruppe und/oder Aethergruppe und/oder geschützte Aminogruppe enthält.

**Claims**

**Claims for the following Contracting States : BE, CH, DE, DK, FR, GB, IT, LI, LU, MC, NL, PT, SE**

**1.** N-Acyl-α-amino acid derivatives of the formula

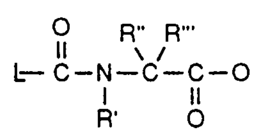 I

wherein

L is a group of the formula

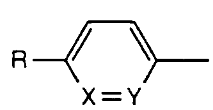 $L^1$

or

$R^o$-$NH(CH_2)_t$ $L^2$

R is amidino or guanidino,

one of X and Y is CH and the other is CH or N,

$R^o$ is hydrogen or amidino,

t is a whole number of 2 to 6,

R', R'' and R''' are hydrogen or N-substituents or side-chains usual in α-aminocarboxylic acids, whereby hydroxy or carboxy groups present in R', R'' and R''' can be etherified or, respectively, esterified or amidated and amino groups present in R', R'' and R''' can be $C_{1-6}$-alkanoylated or aroylated,

Q is a group of the formula

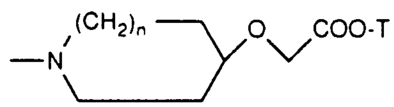

Q[1]

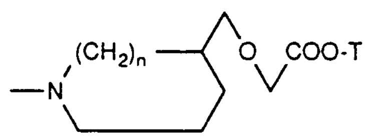

$Q^2$

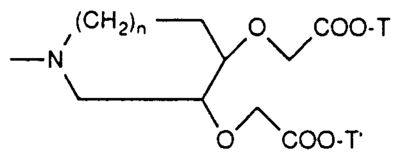

$Q^3$

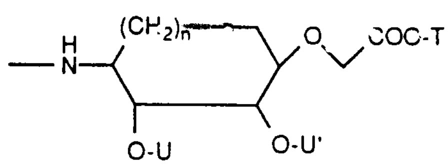

$Q^4$

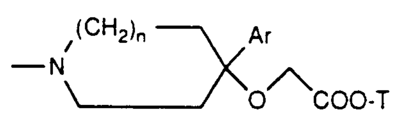

$Q^5$

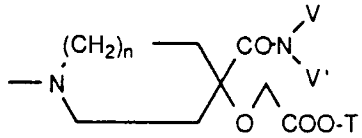

$Q^6$

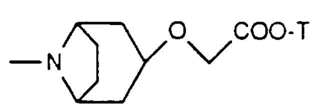

O[7]

or

-N(V')$(CH_2)$v-C(V'',V''')$CH_2OCH_2COO$-T $Q^8$

or, where R' and R'' together with the N atom and C atom to which they are

attached form a ring, can also be a group of the formula

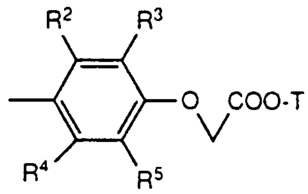

Q9

| | |
|---|---|
| n | is the number 0 or 1, |
| v | is a whole number of 0 to 3, |
| T and T' | are hydrogen or lower-alkyl or phenyl-lower-alkyl which is cleavable under physiological conditions, |
| V to V''' | are hydrogen or lower-alkyl, |
| U and U' | are hydrogen, $C_{1-6}$-alkanoyl or aroyl, |
| Ar | is aryl and |
| $R^2$ to $R^5$ | are hydrogen, lower-alkyl, lower-alkoxy, halogen or a group $-OCH_2COO$-T' or |
| $R^2$ and $R^3$ | together with the phenyl group to which they are attached form a 1-naphthyl group, whereby "lower" denotes groups with 1-6 C atoms, |

as well as hydrates or solvates and physiologically usable salts thereof.

2. Compounds according to claim 1, wherein L is a group $L^1$,

| | |
|---|---|
| R', R'' and R''' | are hydrogen or N-substituents or side-chains usual in α-aminocarboxylic acids, whereby hydroxy or carboxy groups present can be etherified or, respectively, esterified or amidated, and |
| T | in group Q is hydrogen or lower-alkyl which is cleavable under physiological conditions. |

3. Compounds according to claim 1 or 2, wherein L is a group $L^1$, R is amidino, X is CH, Y is CH or N and Q is a group $Q^1$, $Q^2$, $Q^4$, $Q^5$ or $Q^9$.

4. Compounds according to claim 1, 2 or 3, wherein Q is a group $Q^1$, especially in which n = 1 and T is hydrogen or methyl and -N(R')C(R'',R''')CO- is one of the residues Gly, Ala, D-Ala, Val, Leu, Sar, Orn, Lys, Phg, 2-methyl-Pro, Phe, Tyr, 3-iodo-Tyr, 3,5-diiodo-Tyr, Ser(Ac), Ser, Asp, Glu, Pro, 4-benzyloxy-Pro, 4-hydroxy-Pro, 2-piperidylenecarbonyl, $NHCH(CH_2CH_2NH_2)CO$, Trp, Tyr(Me), Tyr(hexyl) and O,N-$(Me)_2$-Tyr.

5. Compounds according to claim 1, 2 or 3, wherein Q is a group $Q^2$, especially in which n = 1, T is hydrogen and -N(R')C(R'',R''')CO- is the Ala residue.

6. Compounds according to claim 1, 2 or 3, wherein Q is a group $Q^4$, especially those in which n = 1, T is hydrogen, U and U' are hydrogen or Ac and -N(R')C(R'',R''')CO- is the Ala residue.

7. Compounds according to claim 1, 2 or 3, wherein Q is a group $Q^5$, especially in which n = 1, T is hydrogen, Ar is α,α,α-trifluoro-m-tolyl and -N(R')C(R'',R''')CO- is the Ala residue.

8. Compounds according to claim 1, 2 or 3 wherein Q is a group $Q^9$, especially in which $R^2$ to $R^5$ are hydrogen or $R^2$ is the group $-OCH_2COO$(H or methyl), T is hydrogen or methyl and -N(R')C(R'',R''')CO- is the Pro residue.

9. Compounds according to claim 1 or 3 from the group of
[[1-N-(p-amidinobenzoyl)-L-alanyl]-4-piperidinyl]oxy]acetic acid,
[[1-[N-[(5-amidino-2-pyridyl)carbonyl]-L-alanyl]-4-piperidinyl]oxy]acetic acid,
[[1-N-(p-amidinobenzoyl)-3-(4-hydroxy-3-iodophenyl)-L-alanyl]-4-piperidinyl]oxy]acetic acid,
[[1-[3-acetoxy-N-(p-amidinobenzoyl)-L-alanyl]-4-piperidinyl]oxy]acetic acid,
[p-[[1-(p-amidinobenzoyl)-2-pyrrolidinyl]carbonyl]phenoxy]acetic acid,

[[1-[N-[(5-amidino-2-pyridyl)carbonyl]-L-tyrosyl]-4-piperidinyl]oxy]acetic acid and especially [[1-N-(p-amidinobenzoyl)-L-tyrosyl]-4-piperidinyl]oxy]acetic acid.

**10.** Compounds according to claim 1, wherein Q is a group $Q^3$, especially in which n = 0 and T is hydrogen, or a group $Q^7$, especially in which T is hydrogen.

**11.** Compounds according to claim 1, wherein Q is a group $Q^8$, especially in which v = 1, T is hydrogen or butyl and V' to V''' are hydrogen.

**12.** Compounds according to claim 1, wherein -N(R')C(R'',R''')CO- is the N(methoxyethyl)Gly residue.

**13.** Compounds according to claim 1 from the group of:
(S)-1-[2-(5-Amidinopyridin-2-ylcarbonylamino)-3-(4-methoxyphenyl)propionyl]piperidin-4-yloxyacetic acid,
ethyl (S)-1-[2-(4-amidinobenzamido)-3-(4-methoxyphenyl)propionyl]piperidin-4-yloxyacetate,
(S)-1-[2-(4-amidinobenzamido)-3-(4-methoxyphenyl)propionyl]piperidin-4-yloxyacetic acid,
[1-[N-(4-amidinobenzoyl)-4'-hexyloxy-L-phenylalanyl]piperidin-4-yloxy]-acetic acid.

**14.** Compounds of the formula

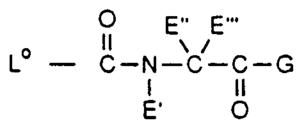

II

or

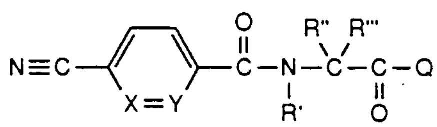

III

wherein

$L^o$ is a group of the formula

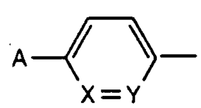

$L^{o1}$

or

$R^{o1}-(CH_2)_t$ $L^{o2}$

in which A is an optionally protected amidino or guanidino group,

$R^{o1}$ is an optionally protected amino or guanidino group,

E', E'', E''' and G have the same significance as R', R'', R''' and, respectively, Q in formula I, whereby where $R^{o1}$ is amino or guanidino or where A is amidino or guanidino, at least one of E', E'', E''' and G contains at least one carboxylic acid ester group and/or ether group and/or protected amino group.

**15.** Compounds according to any one of claims 1-13 for use as pharmaceutically active substances.

**16.** A process for the manufacture of the compounds according to any one of claims 1-13, characterized by
a) cleaving an ether group or a protected amino, amidino or guanidino group or a carboxylic acid ester in a compound of the formula

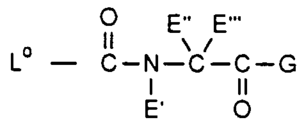 II

wherein

$L^o$ is a group of the formula

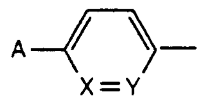 $L^{o1}$

or

$R^{o1}$-$(CH_2)_t$ $L^{o2}$

in which A is an optionally protected amidino or guanidino group,

$R^{01}$ is an optionally protected amino or guanidino group,

E', E'', E''' and G have the same significance as R', R'', R''' and, respectively, Q in formula I, whereby where $R^{01}$ is amino or where A is amidino or guanidino, at least one of E', E'', E''' and G contains at least one carboxylic acid ester group and/or ether group and/or protected amino group,

or

b) converting the cyano group in a nitrile of the formula

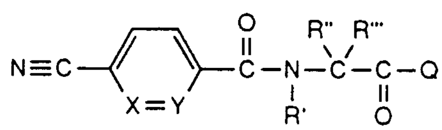 III

into the amidino group, or

c) reacting an amine of the formula

R'-NHC(R'',R'')CO-Q IV

with an acid of the formula $L^1$-COOH or a reactive derivative thereof, and

d) if desired, functionally modifying a reactive group present in a compound of formula I, and

e) if desired, converting a compound of formula I into a physiologically compatible salt or converting a salt of a compound of formula I into the free acid or base.

**17.** Pharmaceutical preparations, especially for the treatment or prophylaxis of illnesses which are caused by the binding of adhesive proteins to blood platelets and by blood platelet aggregation and cell-cell adhesion, containing a compound according to any one of claims 1-13 as the active ingredient.

**18.** The use of a compound according to any one of claims 1-13 for the manufacture of medicaments for the treatment or prophylaxis of illnesses which are caused by the binding of adhesive proteins to blood platelets and by blood platelet aggregation and cell-cell adhesion, especially for the treatment or prophylaxis of blood platelet thrombi, thrombosis, stroke, cardiac infarct, inflammation, arteriosclerosis or osteoporosis, or as an antitumour agent or as an agent for wound healing.

**Claims for the following Contracting State : ES**

**1.** A process for the manufacture of N-Acyl-α-amino acid derivatives of the formula

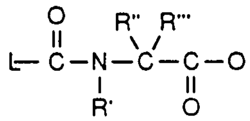

I

wherein

L is a group of the formula

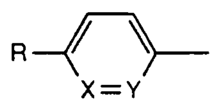

$L^1$

or

$R^o$-NH($CH_2$)$_t$ $L^2$

R is amidino or guanidino,

one of X and Y is CH and the other is CH or N,

| | |
|---|---|
| $R^o$ | is hydrogen or amidino, |
| t | is a whole number of 2 to 6, |
| R', R'' and R''' | are hydrogen or N-substituents or side-chains usual in α-aminocarboxylic acids, whereby hydroxy or carboxy groups present in R', R'' and R''' can be etherified or, respectively, esterified or amidated and amino groups present in R', R'' and R''' can be $C_{1-6}$-alkanoylated or aroylated, |
| Q | is a group of the formula |

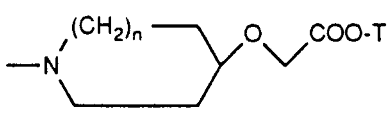

$Q^1$

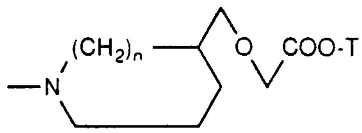

$Q^2$

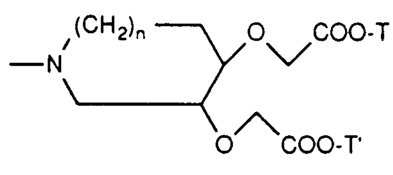

$Q^3$

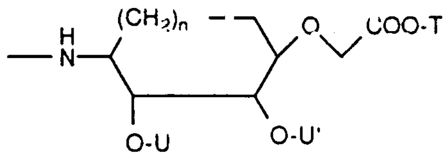

Q4

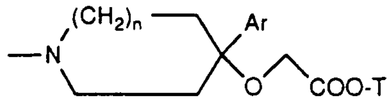

Q5

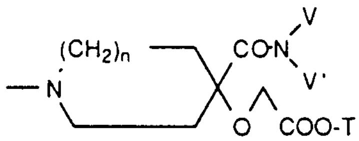

Q6

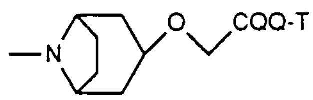

Q7

or

$-N(V')(CH_2)v-C(V'',V''')CH_2OCH_2COO-T$ Q8

or, where R' and R" together with the N atom and C atom to which they are attached form a ring, can also be a group of the formula

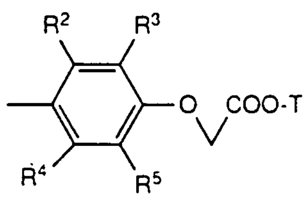

Q9

| | |
|---|---|
| n | is the number 0 or 1, |
| v | is a whole number of 0 to 3, |
| T and T' | are hydrogen or lower-alkyl or phenyl-lower-alkyl which is cleavable under physiological conditions, |
| V to V''' | are hydrogen or lower-alkyl, |
| U and U' | are hydrogen, $C_{1-6}$-alkanoyl or aroyl, |
| Ar | is aryl and |
| $R^2$ to $R^5$ | are hydrogen, lower-alkyl, lower-alkoxy, halogen or a group $-OCH_2COO-T'$ or |
| $R^2$ and $R^3$ | together with the phenyl group to which they are attached form a 1 -naphthyl group, whereby "lower" denotes groups with 1-6 C atoms, |

as well as of hydrates or solvates and physiologically usable salts thereof, characterized by

a) cleaving an ether group or a protected amino, amidino or guanidino group or a carboxylic acid ester in a compound of the formula

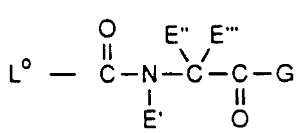

II

wherein

$L^o$ is a group of the formula

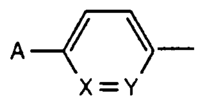

$L^{o1}$

or

$R^{o1}$-$(CH_2)_t$ $L^{o2}$

in which A is an optionally protected amidino or guanidino group,

$R^{01}$ is an optionally protected amino or amidino group,

E', E'', E''' and G have the same significance as R', R'', R''' and, respectively, Q in formula I, whereby where $R^{01}$ is amino or where A is amidino or guanidino, at least one of E', E'', E''' and G contains at least one carboxylic acid ester group and/or ether group and/or protected amino group,

or

b) converting the cyano group in a nitrile of the formula

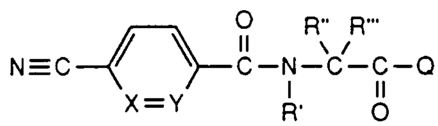

III

into the amidino group, or

c) reacting an amine of the formula

R'-NHC(R'',R'')CO-Q IV

with an acid of the formula $L^1$-COOH or a reactive derivative thereof, and

d) if desired, functionally modifying a reactive group present in a compound of formula I, and

e) if desired, converting a compound of formula I into a physiologically compatible salt or converting a salt of a compound of formula I into the free acid or base.

**2.** A process according to claim 1, wherein L is a group $L^1$,

R',R'' and R''' are hydrogen or N-substituents or side-chains usual in $\alpha$-aminocarboxylic acids, whereby hydroxy or carboxy groups present can be etherified or, respectively, esterified or amidated, and

T in group Q is hydrogen or lower-alkyl which is cleavable under physiological conditions.

**3.** A process according to claim 1 or 2, wherein L is a group $L^1$, R is amidino, X is CH, Y is CH or N and Q is a group $Q^1$, $Q^2$, $Q^4$, $Q^5$ or $Q^9$.

**4.** A process according to claim 1, 2 or 3, wherein Q is a group $Q^1$, especially in which n = 1 and T is hydrogen or methyl and -N(R')C(R'',R''')CO- is one of the residues Gly, Ala, D-Ala, Val, Leu, Sar, Orn, Lys, Phg, 2-methyl-Pro, Phe, Tyr, 3-iodo-Tyr, 3,5-diiodo-Tyr, Ser(Ac), Ser, Asp, Glu, Pro, 4-benzyloxy-Pro, 4-hydroxy-Pro, 2-piperidylenecarbonyl, $NHCH(CH_2CH_2NH_2)CO$, Trp, Tyr(Me), Tyr(hexyl) and O,N-$(Me)_2$-Tyr.

**5.** A process according to claim 1, 2 or 3, wherein Q is a group $Q^2$, especially in which n = 1, T is hydrogen and -N(R')C(R'',R''')CO- is the Ala residue.

**6.** A process according to claim 1, 2 or 3, wherein Q is a group $Q^4$, especially those in which n = 1, T is hydrogen, U and U' are hydrogen or Ac and -N(R')C(R'',R''')CO- is the Ala residue.

**7.** A process according to claim 1, 2 or 3, wherein Q is a group $Q^5$, especially in which n = 1, T is hydrogen, Ar is α,α,α-trifluoro-m-tolyl and -N(R')C(R'',R''')CO- is the Ala residue.

**8.** A process according to claim 1, 2 or 3, wherein Q is a group $Q^9$, especially in which $R^2$ to $R^5$ are hydrogen or $R^2$ is the group $-OCH_2COO$(H or methyl), T is hydrogen or methyl and -N(R')C(R'',R''')CO- is the Pro residue.

**9.** A process according to claim 1 or 3 for the manufacture of a compound from the group of
[[1-N-(p-amidinobenzoyl)-L-alanyl]-4-piperidinyl]oxy]acetic acid,
[[1-[N-[(5-amidino-2-pyridyl)carbonyl]-L-alanyl]-4-piperidinyl]oxy]acetic acid,
[[1-N-(p-amidinobenzoyl)-3-(4-hydroxy-3-iodophenyl)-L-alanyl]-4-piperidinyl]oxy]acetic acid,
[[1-[3-acetoxy-N-(p-amidinobenzoyl)-L-alanyl]-4-piperidinyl]oxy]acetic acid,
[p-[[1-(p-amidinobenzoyl)-2-pyrrolidinyl]carbonyl]phenoxy]acetic acid
[[1-[N-[(5-amidino-2-pyridyl)carbonyl]-L-tyrosyl]-4-piperidinyl]oxy]acetic acid and especially
[[1-N-(p-amidinobenzoyl)-L-tyrosyl]-4-piperidiny]oxy]acetic acid.

**10.** A process according to claim 1, wherein Q is a group $Q^3$, especially in which n = 0 and T is hydrogen, or a group $Q^7$, especially in which T is hydrogen.

**11.** A process according to claim 1, wherein Q is a group $Q^8$, especially in which v = 1, T is hydrogen or butyl and V' to V''' are hydrogen.

**12.** Compounds according to claim 1, wherein -N(R')C(R'',R''')CO- is the N(methoxyethyl)Gly residue.

**13.** A process according to claim 1 for the manufacture of a compound from the group of:
(S)-1-[2-(5-Amidinopyridin-2-ylcarbonylamino)-3-(4-methoxyphenyl)propionyl]piperidin-4-yloxyacetic acid,
ethyl (S)-1-[2-(4-amidinobenzamido)-3-(4-methoxyphenyl)propionyl]piperidin-4-yloxyacetate,
(S)-1-[2-(4-amidinobenzamido)-3-(4-methoxyphenyl)propionyl]piperidin-4-yloxyacetic acid,
[1-[N-(4-amidinobenzoyl)-4'-hexyloxy-L-phenylalanyl]piperidin-4-yloxy]-acetic acid.

**14.** The use of a compound according to any one of claims 1-13 for the manufacture of medicaments for the treatment or prophylaxis of illnesses which are caused by the binding of adhesive proteins to blood platelets and by blood platelet aggregation and cell-cell adhesion, especially for the treatment or prophylaxis of blood platelet thrombi, thrombosis, stroke, cardiac infarct, inflammation, arteriosclerosis or osteoporosis, or as an antitumour agent or as an agent for wound healing.

**Claims for the following Contracting State : GR**

**1.** A process for the manufacture of N-Acyl-α-amino acid derivatives of the formula

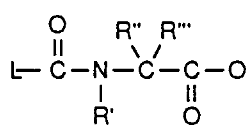

I

wherein

L is a group of the formula

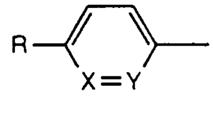

$L^1$

or

$R^o$-NH($CH_2$)$_t$ $L^2$

R is amidino or guanidino,

one of X and Y is CH and the other is CH or N,

- $R^o$ is hydrogen or amidino,
- t is a whole number of 2 to 6,
- R', R'' and R''' are hydrogen or N-substituents or side-chains usual in α-aminocarboxylic acids, whereby hydroxy or carboxy groups present in R', R'' and R''' can be etherified or, respectively, esterified or amidated and amino groups present in R', R'' and R''' can be $C_{1-6}$-alkanoylated or aroylated,
- Q is a group of the formula

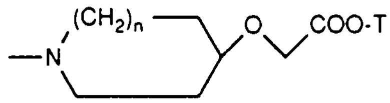

$Q^1$

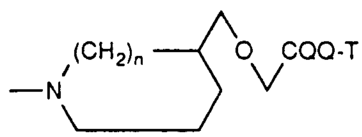

$Q^2$

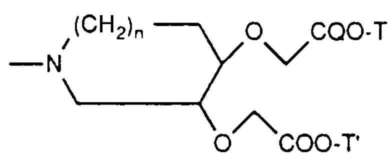

$Q^3$

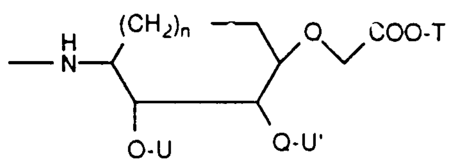

$Q^4$

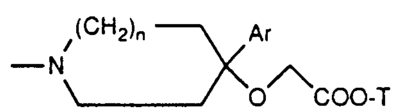

$Q^5$

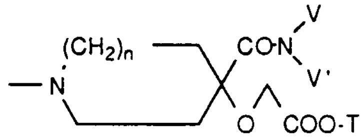

$O^6$

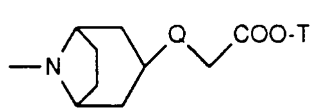

$Q^7$

or

$-N(V')(CH_2)v-C(V'',V''')CH_2OCH_2COO-T$ Q8

or, where R' and R'' together with the N atom and C atom to which they are attached form a ring, can also be a group of the formula

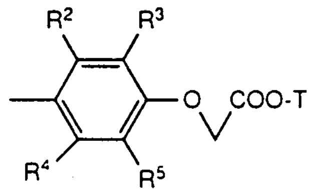

Q9

| | |
|---|---|
| n | is the number 0 or 1, |
| v | is a whole number of 0 to 3, |
| T and T' | are hydrogen or lower-alkyl or phenyl-lower-alkyl which is cleavable under physiological conditions, |
| V to V''' | are hydrogen or lower-alkyl, |
| U and U' | are hydrogen, $C_{1-6}$-alkanoyl or aroyl, |
| Ar | is aryl and |
| $R^2$ to $R^5$ | are hydrogen, lower-alkyl, lower-alkoxy, halogen or a group $-OCH_2COO-T'$ or |
| $R^2$ and $R^3$ | together with the phenyl group to which they are attached form a 1-naphthyl group, whereby "lower" denotes groups with 1-6 C atoms, |

as well as of hydrates or solvates and physiologically usable salts thereof, characterized by

a) cleaving an ether group or a protected amino, amidino or guanidino group or a carboxylic acid ester in a compound of the formula

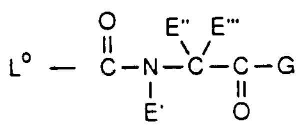

II

wherein

| | |
|---|---|
| $L^o$ | is a group of the formula |

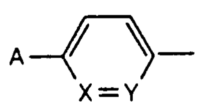

$L^{o1}$

or

$R^{o1}-(CH_2)_t$ $L^{o2}$

in which A is an optionally protected amidino or guanidino group,

| | |
|---|---|
| $R^{01}$ | is an optionally protected amino or amidino group, |
| E', E'', E''' and G | have the same significance as R', R'', R''' and, respectively, Q in formula I, whereby where $R^{01}$ is amino or where A is amidino or guanidino, at least one of E', E'', E''' and G contains at least one carboxylic acid ester group and/or ether group and/or protected amino group, |

or

b) converting the cyano group in a nitrile of the formula

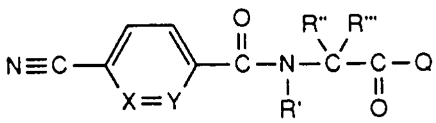

III

into the amidino group, or

c) reacting an amine of the formula

R'-NHC(R'',R'')CO-Q IV

with an acid of the formula $L^1$-COOH or a reactive derivative thereof, and

d) if desired, functionally modifying a reactive group present in a compound of formula I, and

e) if desired, converting a compound of formula I into a physiologically compatible salt or converting a salt of a compound of formula I into the free acid or base.

**2.** A process according to claim 1, wherein L is a group $L^1$,

R',R'' and R''' are hydrogen or N-substituents or side-chains usual in α-aminocarboxylic acids, whereby hydroxy or carboxy groups present can be etherified or, respectively, esterified or amidated, and

T in group Q is hydrogen or lower-alkyl which is cleavable under physiological conditions.

**3.** A process according to claim 1 or 2, wherein L is a group $L^1$, R is amidino, X is CH, Y is CH or N and Q is a group $Q^1$, $Q^2$, $Q^4$, $Q^5$ or $Q^9$.

**4.** A process according to claim 1, 2 or 3, wherein Q is a group $Q^1$, especially in which n = 1 and T is hydrogen or methyl and -N(R')C(R'',R''')CO- is one of the residues Gly, Ala, D-Ala, Val, Leu, Sar, Orn, Lys, Phg, 2-methyl-Pro, Phe, Tyr, 3-iodo-Tyr, 3,5-diiodo-Tyr, Ser(Ac), Ser, Asp, Glu, Pro, 4-benzyloxy-Pro, 4-hydroxy-Pro, 2-piperidylenecarbonyl, $NHCH(CH_2CH_2NH_2)CO$, Trp, Tyr(Me), Tyr(hexyl) and O,N-$(Me)_2$-Tyr.

**5.** A process according to claim 1, 2 or 3, wherein Q is a group $Q^2$, especially in which n = 1, T is hydrogen and -N(R')C(R'',R''')CO- is the Ala residue.

**6.** A process according to claim 1, 2 or 3, wherein Q is a group $Q^4$, especially those in which n = 1, T is hydrogen, U and U' are hydrogen or Ac and -N(R')C(R'',R''')CO- is the Ala residue.

**7.** A process according to claim 1, 2 or 3, wherein Q is a group $Q^5$, especially in which n = 1, T is hydrogen, Ar is α,α,α-trifluoro-m-tolyl and -N(R')C(R'',R''')CO- is the Ala residue.

**8.** A process according to claim 1, 2 or 3, wherein Q is a group $Q^9$, especially in which $R^2$ to $R^5$ are hydrogen or $R^2$ is the group $-OCH_2COO$(H or methyl), T is hydrogen or methyl and -N(R')C(R'',R''')CO- is the Pro residue.

**9.** A process according to claim 1 or 3 for the manufacture of a compound from the group of

[[1-N-(p-amidinobenzoyl)-L-alanyl]-4-piperidinyl]oxy]acetic acid,

[[1-[N-[(5-amidino-2-pyridyl)carbonyl]-L-alanyl]-4-piperidinyl]oxy]acetic acid,

[[1-N-(p-amidinobenzoyl)-3-(4-hydroxy-3-iodophenyl)-L-alanyl]-4-piperidinyl]oxy]acetic acid,

[[1-[3-acetoxy-N-(p-amidinobenzoyl)-L-alanyl]-4-piperidinyl]oxy]acetic acid,

[p-[[1-(p-amidinobenzoyl)-2-pyrrolidinyl]carbonyl]phenoxy]acetic acid,

[[1-[N-[(5-amidino-2-pyridyl)carbonyl]-L-tyrosyl]-4-piperidinyl]oxy]acetic acid and especially

[[1-N-(p-amidinobenzoyl)-L-tyrosyl]-4-piperidinyl]oxy]acetic acid.

**10.** A process according to claim 1, wherein Q is a group $Q^3$, especially in which n = 0 and T is hydrogen, or a group $Q^7$, especially in which T is hydrogen.

**11.** A process according to claim 1, wherein Q is a group $Q^8$, especially in which v = 1, T is hydrogen or butyl and V' to V''' are hydrogen.

**12.** Compounds according to claim 1, wherein -N(R')C(R'',R''')CO- is the N(methoxyethyl)Gly residue.

**13.** A process according to claim 1 for the manufacture of a compound from the group of:

(S)-1-[2-(5-Amidinopyridin-2-ylcarbonylamino)-3-(4-methoxyphenyl)propionyl]piperidin-4-yloxyacetic acid,

ethyl (S)-1-[2-(4-amidinobenzamido)-3-(4-methoxyphenyl)propionyl]piperidin-4-yloxyacetate,

(S)-1-[2-(4-amidinobenzamido)-3-(4-methoxyphenyl)propionyl]piperidin-4-yloxyacetic acid,

[1-[N-(4-amidinobenzoyl)-4'-hexyloxy-L-phenylalanyl]piperidin-4-yloxy]-acetic acid.

**14.** A process for the manufacture of pharmaceutical preparations, especially for the treatment or prophylaxis of illnesses which are caused by the binding of adhesive proteins to blood platelets and by blood platelet aggregation and cell-cell adhesion, containing a compound according to any one of claims 1-13 as the active ingredient.

**15.** The use of a compound according to any one of claims 1-13 for the manufacture of medicaments for the treatment or prophylaxis of illnesses which are caused by the binding of adhesive proteins to blood platelets and by blood platelet aggregation and cell-cell adhesion, especially for the treatment or prophylaxis of blood platelet thrombi, thrombosis, stroke, cardiac infarct, inflammation, arteriosclerosis or osteoporosis, or as an antitumour agent or as an agent for wound healing.

**16.** Compounds of the formula

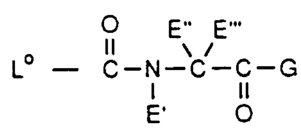

II

or

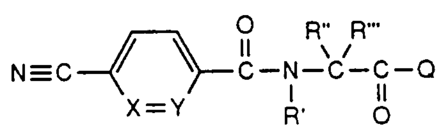

III

wherein

$L^o$ is a group of the formula

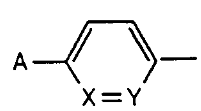

$L^{o1}$

or

$R^{o1}$-$(CH_2)_t$ $L^{o2}$

in which A is an optionally protected amidino or guanidino group,

$R^{01}$ is an optionally protected amino or guanidino group,

E', E'', E''' and G have the same significance as R', R'', R''' and, respectively, Q in formula I, whereby where $R^{01}$ is amino or guanidino or where A is amidino or guanidino, at least one of E', E'', E''' and G contains at least one carboxylic acid ester group and/or ether group and/or protected amino group.

**Revendications**

**Revendications pour les Etats contractants suivants : BE, CH, DE, DK, FR, GB, IT, LI, LU, MC, NL, PT, SE**

**1.** Dérivés de N-acyl-alpha-aminoacides de formule

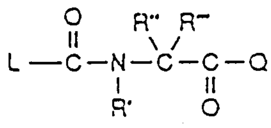

I

dans laquelle
L représente un groupe de formule

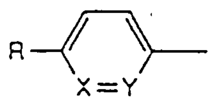

$L^1$

ou

$R^o$-NH$(CH_2)_t$ $L^2$

R représente un groupe amidino ou guanidino,
l'un des symboles X et Y représente CH et l'autre CH ou N,
$R^o$ représente l'hydrogène ou un groupe amidino,
t est un nombre entier allant de 2 à 6,
R' , R'' et R''' représentent l'hydrogène ou des substituants à l'azote ou chaînes latérales usuels dans les acides alpha-aminocarboxyliques, les groupes hydroxy et carboxy présents dans R', R'' et R''' pouvant être éthérifiés ou respectivement estérifiés ou amidés, et les groupes amino alcanoylés en C1-C6 ou aroylés,
Q représente un groupe de formule

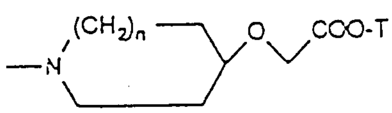

$Q^1$

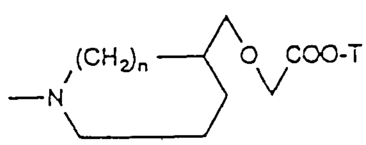

$Q^2$

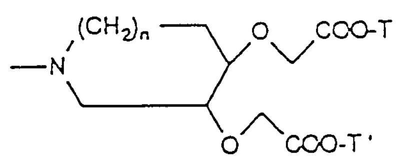

$Q^3$

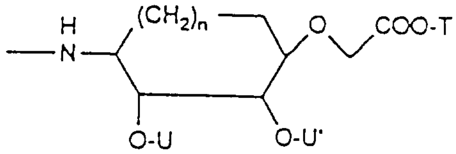

Q⁴

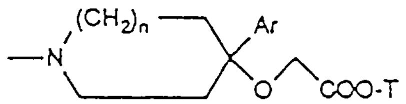

Q5

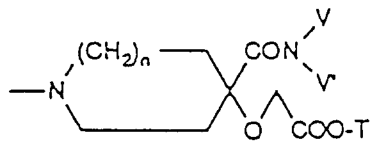

Q6

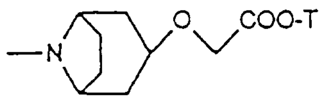

Q7

ou

-N(V')$(CH_2)_v$-C(V'',V''')$CH_2OCH_2COO$-T Q8

ou bien, lorsque R' et R'' forment ensemble et avec l'atome d'azote et l'atome de carbone auxquels ils sont reliés, un cycle, un groupe de formule

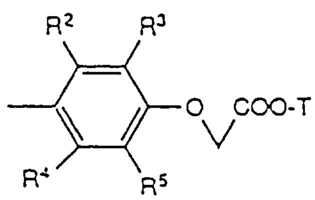

Q9

n est égal à 0 ou 1,
v est un nombre entier allant de 0 à 3,
T et T' représentent l'hydrogène ou des groupes alkyle inférieurs ou phényl-alkyle inférieurs scindables dans l'organisme,
V à V''' représentent l'hydrogène ou des groupes alkyle inférieurs,
U et U' représentent l'hydrogène, des groupes alcanoyle en C1-C6 ou aroyle,
Ar représente un groupe aryle et
$R^2$ à $R^5$ représentent l'hydrogène, des groupes alkyle inférieurs, alcoxy inférieurs, des halogènes ou un groupe -$OCH_2COO$-T', ou bien
$R^2$ et $R^3$ forment ensemble et avec le groupe phényle auquel ils sont reliés, un groupe 1-naphtyle,
l'expression "inférieur" s'appliquant à des groupes qui contiennent de 1 à 6 atomes de carbone,
ainsi que des hydrates ou solvates et sels acceptables pour l'usage pharmaceutique de ces composés.

**2.** Composés selon revendication 1, dans lesquels L représente un groupe $L^1$, R' , R'' et R''' représentent l'hydrogène ou des substituants à l'azote ou chaînes latérales usuels dans les acides alpha-aminocarboxyliques, les groupes hydroxy ou carboxy présents dans R', R'' et R''' pouvant être éthérifiés et respectivement estérifiés ou amidés, et T, dans le groupe Q, représente l'hydrogène ou un groupe alkyle inférieur scindable dans l'organisme.

3. Composés selon revendication 1 ou 2, dans lesquels L représente un groupe $L^1$, R représente un groupe amidino, X représente CH, Y représente CH ou N et Q représente un groupe $Q^1$, $Q^2$, $Q^4$, $Q^5$ ou $Q^9$.

4. Composés selon la revendication 1, 2 ou 3, dans lesquels Q représente un groupe $Q^1$, et plus spécialement dans lesquels n = 1 et T représente l'hydrogène ou un groupe méthyle, et -N(R')C-(R'',R''')CO- représente l'un des groupes Gly, Ala, D-Ala, Val, Leu, Sar, Orn, Lys, Phg, 2-méthyl-Pro, Phe, Tyr, 3-iodo-Tyr, 3,5-diiodo-Tyr, Ser(Ac), Ser, Asp, Glu, Pro, 4-benzyloxy-Pro, 4-hydroxy-Pro, 2-pipéridylène-carbonyle, $NHCH(CH_2CH_2NH_2)CO$, Trp, Tyr(Me), Tyr(hexyle) et $O,N(Me)_2$-Tyr.

5. Composés selon la revendication 1, 2 ou 3, dans lesquels Q représente un groupe $Q^2$ et plus spécialement dans lesquels n = 1, T représente l'hydrogène et -N(R')C(R'',R''')CO- représente le groupe Ala.

6. Composés selon la revendication 1, 2 ou 3, dans lesquels Q représente un groupe $Q^4$ et plus spécialement dans lesquels n = 1, T représente l'hydrogène, U et U' l'hydrogène ou Ac et - N(R')C-(R'',R''')CO- le groupe Ala.

7. Composés selon la revendication 1, 2 ou 3, dans lesquels Q représente un groupe $Q^5$, et plus spécialement dans lesquels n = 1, T représente l'hydrogène, Ar représente le groupe alpha,alpha,alpha-trifluoro-m-tolyle et -N(R')C(R'',R''')CO- le groupe Ala.

8. Composés selon la revendication 1, 2 ou 3, dans lesquels Q représente un groupe $Q^9$, et plus spécialement dans lesquels $R^2$ à $R^5$ représentent l'hydrogène, ou bien $R^2$ représente le groupe $-OCH_2COO$(H ou méthyle), T représente l'hydrogène ou un groupe méthyle et -N(R')C(R'',R''')CO- le groupe Pro.

9. Composés selon la revendication 1 ou 3, pris dans le groupe suivant :
l'acide [[1-[N-(p-amidinobenzoyl)-L-alanyl]-4-pipéridinyl]oxy]acétique,
l'acide [[1-[N-[(5-amidino-2-pyridyl)carbonyl]-L-alanyl]-4-pipéridinyl]oxy]acétique,
l'acide [[1-[N-(p-amidinobenzoyl)-3-(4-hydroxy-3-iodophényl)-L-alanyl]-4-pipéridinyl]oxy]acétique,
l'acide [[1-[3-acétoxy-N-(p-amidinobenzoyl)-L-alanyl]-4-pipéridinyl]oxy]acétique,
l'acide [p-[[1-(p-amidinobenzoyl)-2-pyrrolidinyl]carbonyl]phénoxy]acétique,
l'acide [[1-N-[(5-amidino-2-pyridyl)carbonyl]-L-tyrosyl]-4-pipéridinyl]oxy]acétique et plus spécialement
l'acide [[1-[N-(p-amidinobenzoyl)-L-tyrosyl]-4-pipéridinyl]oxy]acétique.

10. Composés selon revendication 1, dans lesquels Q représente un groupe $Q^3$, et plus spécialement dans lesquels n = 0 et T représente l'hydrogène, ou bien un groupe $Q^7$, et plus spécialement dans lesquels T représente l'hydrogène.

11. Composés selon revendication 1, dans lesquels Q représente un groupe $Q^8$, et plus spécialement dans lesquels v = 1, T représente l'hydrogène ou un groupe butyle et V' à V''' représentent l'hydrogène.

12. Composés selon revendication 1, dans lesquels -N(R')C(R'',R''')CO- représente le groupe N-(méthoxyéthyl)-Gly.

13. Composés selon revendication 1, pris dans le groupe suivant :
l'acide (S)-1-[2-(5-amidinopyridine-2-ylcarbonylamino)-3-(4-méthoxyphényl)-propionyl]pipéridine-4-yloxy acétique,
le (S)-1-[2-(4-amidinobenzamido)-3-(4-méthoxyphényl)propionyl]pipéridine-4-yloxy acétate d'éthyle,
l'acide (S)-1-[2-(4-amidinobenzamido)-3-(4-méthoxyphényl)propionyl]pipéridine-4-yloxy acétique,
l'acide [1-[N-(4-amidinobenzoyl)-4'-hexyloxy-L-phénylalanyl]pipéridine-4-yloxy]acétique.

**14.** Composés de formule

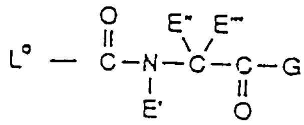

II

ou

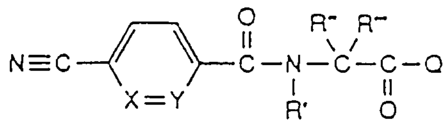

III

dans lesquels
$L^o$ représente un groupe de formule

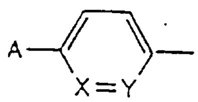

$L^{o1}$

ou

$R^{o1}$-$(CH_2)_t$     $L^{o2}$

A représentant un groupe amidino ou guanidino éventuellement protégé,
$R^{o1}$ un groupe amidino ou guanidino éventuellement protégé,
E', E'', E''' et G ont respectivement les mêmes significations que R', R'', R''' et Q dans la formule I, sous réserve que, lorsque $R^{o1}$ représente un groupe amidino ou guanidino, ou lorsque A représente un groupe amidino ou guanidino, au moins un des groupes E' E'', E''' et G contient au moins un groupe ester d'acide carboxylique et/ou un groupe éther et/ou un groupe amino protégé.

**15.** Composés selon l'une des revendications 1 à 13, pour l'utilisation en tant que substances actives pharmaceutiques.

**16.** Procédé de préparation des composés selon l'une des revendications 1 à 13, caractérisé en ce que
a) dans un composé de formule

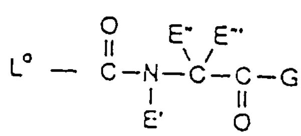

II

dans laquelle
$L^o$ représente un groupe de formule

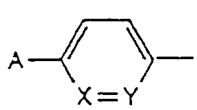

$L^{o1}$

ou

$R^{o1}$-$(CH_2)_t$     $L^{o2}$

A représentant un groupe amino ou guanidino éventuellement protégé,
$R^{o1}$ un groupe amidino ou guanidino éventuellement protégé,
E', E'', E''' et G ont respectivement les mêmes significations que R', R'', R''' et Q dans la formule I, sous réserve que, lorsque $R^{o1}$ représente un groupe amidino ou guanidino, ou bien lorsque A représente un groupe amidino ou guanidino, au moins un des groupes E', E'', E''' et G contient au moins un groupe ester d'acide carboxylique et/ou un groupe éther et/ou un groupe amino protégé,
on scinde un groupe éther ou un groupe amino, amidino ou guanidino protégé, ou un groupe ester d'acide carboxylique, ou bien
b) dans un nitrile de formule

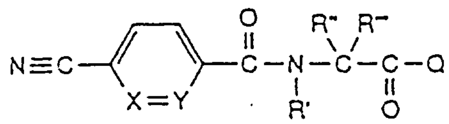

III

on convertit le groupe cyano en groupe amidino, ou bien
c) on fait réagir une amine de formule

R'-NHC(R'',R''')CO-Q IV

avec un acide de formule $L^1$-COOH ou l'un de ses dérivés réactifs, et
d) si on le désire, on soumet un groupe réactif contenu dans un composé de formule I à une modification fonctionnelle et
e) si on le désire, on convertit un composé de formule I en un sel acceptable pour l'usage pharmaceutique, ou bien un sel d'un composé de formule I en l'acide ou la base libre.

**17.** Compositions pharmaceutiques, servant en particulier au traitement ou à la prophylaxie de maladies causées par la fixation de protéines adhésives sur les plaquettes du sang, ou encore par l'agglomération des plaquettes du sang et l'adhérence cellule-cellule, contenant un composé selon l'une des revendications 1 à 13 en tant que substance active.

**18.** Utilisation d'un composé selon l'une des revendications 1 à 13 pour la préparation de médicaments servant au traitement ou à la prophylaxie de maladies causées par la fixation de protéines adhésives sur les plaquettes du sang ou par l'agglomération des plaquettes du sang et l'adhérence cellule-cellule, en particulier pour le traitement ou la prophylaxie des thrombus de plaquettes sanguines, des thomboses, de la congestion cérébrale, de l'infarctus cardiaque, des inflammations, de l'artériosclérose ou de l'ostéoporose ou en tant qu'agents anti-tumeurs ou agents de cicatrisation des blessures.

**Revendications pour l'Etat contractant suivant : ES**

**1.** Procédé de préparation de dérivés de N-acyl-alpha-aminoacides de formule

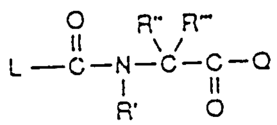

I

dans laquelle
L représente un groupe de formule

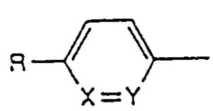

$L^1$

ou

$R^{\circ}$-$NH(CH_2)_t$ $L^2$

R représente un groupe amidino ou guanidino,

l'un des symboles X et Y représente CH et l'autre CH ou N,

$R^o$ représente l'hydrogène ou un groupe amidino,

t est un nombre entier allant de 2 à 6,

R', R'' et R''' représentent l'hydrogène ou des substituants à l'azote ou chaînes latérales usuels dans les acides alpha-aminocarboxyliques, les groupes hydroxy et carboxy présents dans R', R'', et R''' pouvant être éthérifiés ou respectivement estérifiés ou amidés, et les groupes amino alcanoylés en C1-C6 ou aroylés,

Q représente un groupe de formule

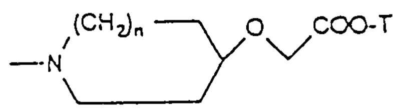

$Q^1$

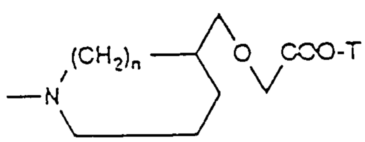

$Q^2$

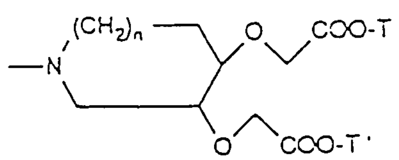

$Q^3$

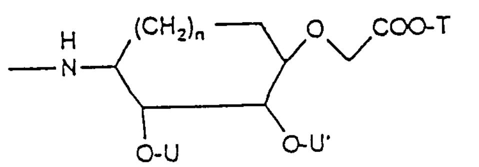

$Q^4$

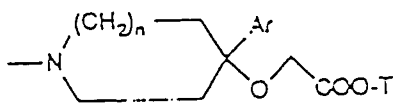

$Q^5$

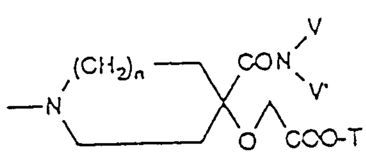

$Q^5$

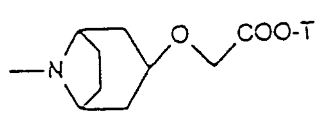

$Q^7$

ou

-$N(V')(CH_2)_v$-$C(V'',V''')CH_2OCH_2COO$-T $Q^8$

ou bien, lorsque R' et R'' forment ensemble et avec l'atome d'azote et l'atome de carbone auxquels ils sont reliés, un cycle, un groupe de formule

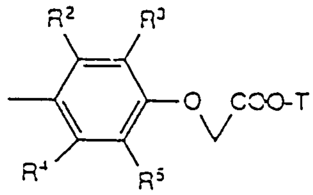

Q9

n est égal à 0 ou 1,

v est un nombre entier allant de 0 à 3,

T et T' représentent l'hydrogène ou des groupes alkyle inférieurs ou phényl-alkyle inférieurs scindables dans l'organisme,

V à V''' représentent l'hydrogène ou des groupes alkyle inférieurs,

U et U' représentent l'hydrogène, des groupes alcanoyle en C1-C6 ou aroyle,

Ar représente un groupe aryle et

$R^2$ à $R^5$ représentent l'hydrogène, des groupes alkyle inférieurs, alcoxy inférieurs, des halogènes ou un groupe $-OCH_2COO-T'$, ou bien

$R^2$ et $R^3$ forment ensemble et avec le groupe phényle auquel ils sont reliés, un groupe 1-naphtyle,

l'expression "inférieur" s'appliquant à des groupes qui contiennent de 1 à 6 atomes de carbone,

ainsi que des hydrates ou solvates et sels acceptables pour l'usage pharmaceutique de ces composés, caractérisé en ce que

a) dans un composé de formule

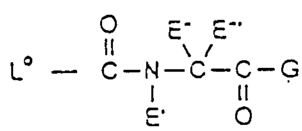

II

dans laquelle

$L^o$ représente un groupe de formule

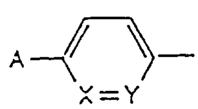

$L^{o1}$

ou

$R^{o1}-(CH_2)_t$ $L^{o2}$

A représentant un groupe amino ou guanidino éventuellement protégé,

$R^{o1}$ un groupe amidino ou guanidino éventuellement protégé,

E', E'', E''' et G ont respectivement les mêmes significations que R', R'', R''' et Q dans la formule I, sous réserve que, lorsque $R^{o1}$ représente un groupe amidino ou guanidino, ou bien lorsque A représente un groupe amidino ou guanidino, au moins un des groupes E', E'', E''' et G contient au moins un groupe ester d'acide carboxylique et/ou un groupe éther et/ou un groupe amino protégé, on scinde un groupe éther ou un groupe amino, amidino ou guanidino protégé, ou un groupe ester d'acide carboxylique, ou bien

b) dans un nitrile de formule

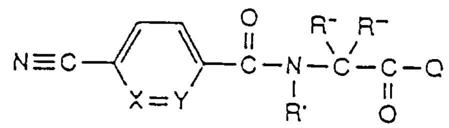

III

on convertit le groupe cyano en groupe amidino, ou bien

c) on fait réagir une amine de formule

R'-NHC(R'',R''')CO-Q IV

avec un acide de formule $L^1$-COOH ou l'un de ses dérivés réactifs, et

d) si on le désire, on soumet un groupe réactif contenu dans un composé de formule I à une modification fonctionnelle et

e) si on le désire, on convertit un composé de formule I en un sel acceptable pour l'usage pharmaceutique, ou bien un sel d'un composé de formule I en l'acide ou la base libre.

**2.** Procédé selon revendication 1, dans lequel L représente un groupe $L^1$, R', R'' et R''' représentent l'hydrogène ou des substituants à l'azote ou chaînes latérales usuels dans les acides alpha-aminocarboxyliques, les groupes hydroxy ou carboxy présents dans R', R'' et R''' pouvant être éthérifiés et respectivement estérifiés ou amidés, et T, dans le groupe Q, représente l'hydrogène ou un groupe alkyle inférieur scindable dans l'organisme.

**3.** Procédé selon revendication 1 ou 2, dans lequel L représente un groupe $L^1$, R représente un groupe amidino, X représente CH, Y représente CH ou N et Q représente un groupe $Q^1$, $Q^2$, $Q^4$, $Q^5$ ou $Q^9$.

**4.** Procédé selon revendication 1, 2 ou 3, dans lequel Q représente un groupe $Q^1$, et plus spécialement dans lequel n = 1 et T représente l'hydrogène ou un groupe méthyle, et -N(R')C(R'',R''')CO- représente l'un des groupes Gly, Ala, D-Ala, Val Leu, Sar, Orn, Lys, Phg, 2-méthyl-Pro, Phe, Tyr, 3-iodo-Tyr, 3,5-diiodo-Tyr, Ser(Ac), Ser, Asp, Glu, Pro, 4-benzyloxy-Pro, 4-hydroxy-Pro, 2-pipéridylène-carbonyle, $NHCH(CH_2CH_2NH_2)CO$, Trp, Tyr(Me), Tyr(hexyle) et $O,N(Me)_2$-Tyr.

**5.** Procédé selon revendication 1, 2 ou 3, dans lequel Q représente un groupe $Q^2$ et plus spécialement dans lequel n = 1, T représente l'hydrogène et - N(R')C(R'',R''')CO- représente le groupe Ala.

**6.** Procédé selon revendication 1, 2 ou 3, dans lequel Q représente un groupe $Q^4$ et plus spécialement dans lequel n = 1, T représente l'hydrogène, U et U' l'hydrogène ou Ac et -N(R')C(R'',R''')CO- le groupe Ala.

**7.** Procédé selon revendication 1, 2 ou 3, dans lequel Q représente un groupe $Q^5$, et plus spécialement dans lequel n = 1, T représente l'hydrogène, Ar représente le groupe alpha,alpha,alpha-trifluoro-m-tolyle et -N(R')C(R'',R''')CO- le groupe Ala.

**8.** Procédé selon revendication 1, 2 ou 3, dans lequel Q représente un groupe $Q^9$, et plus spécialement dans lequel $R^2$ à $R^5$ représentent l'hydrogène, ou bien $R^2$ représente le groupe $-OCH_2COO$(H ou méthyle), T représente l'hydrogène ou un groupe méthyle et N(R')C(R'',R''')CO- le groupe Pro.

**9.** Procédé selon revendication 1 ou 3, pour la préparation d'un composé pris dans le groupe suivant :
l'acide [[1-[N-(p-amidinobenzoyl)-L-alanyl]-4-pipéridinyl]oxy]acétique,
l'acide [[1-[N-[(5-amidino-2-pyridyl)carbonyl]-L-alanyl]-4-pipéridinyl]oxy]acétique,
l'acide [[1-[N-(p-amidinobenzoyl)-3-(4-hydroxy-3-iodophényl)-L-alanyl]-4-pipéridinyl]oxy]acétique,
l'acide [[1-[3-acétoxy-N-(p-amidinobenzoyl)-L-alanyl]-4-pipéridinyl]oxy]acétique,
l'acide [p-[[1-(p-amidinobenzoyl)-2-pyrrolidinyl]carbonyl]phénoxy]acétique,
l'acide [[1-[N-[(5-amidino-2-pyridyl)carbonyl]-L-tyrosyl]-4-pipéridinyl]oxy]acétique et plus spécialement
l'acide [[1-[N-(p-amidinobenzoyl)-L-tyrosyl]-4-pipéridinyl]oxy]acétique.

**10.** Procédé selon revendication 1, dans lequel Q représente un groupe $Q^3$, et plus spécialement dans lequel n = 0 et T représente l'hydrogène, ou bien un groupe $Q^7$, et plus spécialement dans lequel T représente l'hydrogène.

**11.** Procédé selon revendication 1, dans lequel Q représente un groupe $Q^8$, et plus spécialement dans lequel v = 1, T représente l'hydrogène ou un groupe butyle et V' à V''' représentent l'hydrogène.

**12.** Procédé selon revendication 1, dans lequel -N(R')C(R'',R''')CO- représente le groupe N-(méthoxyéthyl)-Gly.

**13.** Procédé selon revendication 1, pour la préparation d'un composé pris dans le groupe suivant :
l'acide (S)-1-[2-(5-amidinopyridine-2-ylcarbonylamino)-3-(4-méthoxyphényl)-propionyl]pipéridine-4-yloxy acétique,
le (S)-1-[2-(4-amidinobenzamido)-3-(4-méthoxyphényl)propionyl]pipéridine-4-yloxy acétate d'éthyle,
l'acide (S)-1-[2-(4-amidinobenzamido)-3-(4-méthoxyphényl)propionyl]pipéridine-4-yloxy acétique,
l'acide [1-[N-(4-amidinobenzoyl)-4'-hexyloxy-L-phénylalanyl]pipéridine-4-yloxy]acétique.

**14.** Utilisation d'un composé selon l'une des revendications 1 à 13, pour la préparation de médicaments servant au traitement ou à la prophylaxie de maladies causées par la fixation de protéines adhésives sur les plaquettes du sang ou par l'agglomération des plaquettes du sang et l'adhérence cellule-cellule, en particulier pour le traitement ou la prophylaxie des thrombus de plaquettes sanguines, des thromboses, de la congestion cérébrale, de l'infarctus cardiaque, des inflammations, de l'artériosclérose ou de l'ostéoporose ou en tant qu'agents anti-tumeurs ou agents de cicatrisation des blessures.

**Revendications pour l'Etat contractant suivant : GR**

**1.** Procédé de préparation de dérivés de N-acyl-alpha-aminoacides de formule

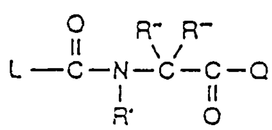

I

dans laquelle
L représente un groupe de formule

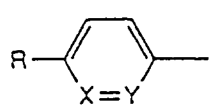

$L^1$

ou

$R^o$-NH$(CH_2)_t$ $L^2$

R représente un groupe amidino ou guanidino,
l'un des symboles X et Y représente CH et l'autre CH ou N,
$R^o$ représente l'hydrogène ou un groupe amidino,
t est un nombre entier allant de 2 à 6,
R', R'' et R''' représentent l'hydrogène ou des substituants à l'azote ou chaînes latérales usuels dans les acides alpha-aminocarboxyliques, les groupes hydroxy et carboxy présents dans R', R'' et R''' pouvant être éthérifiés ou respectivement estérifiés ou amidés, et les groupes amino alcanoylés en C1-C6 ou aroylés,
Q représente un groupe de formule

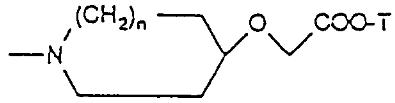

$Q^1$

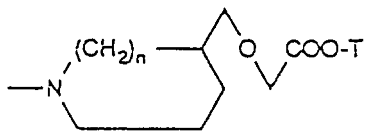

$Q^2$

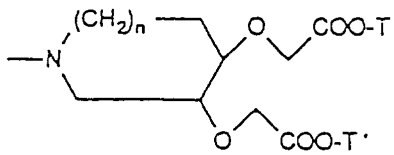

$Q^3$

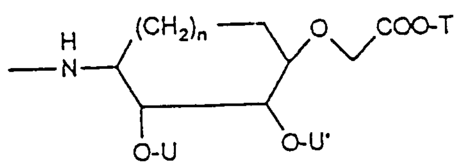

$Q^4$

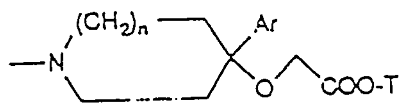

$Q^5$

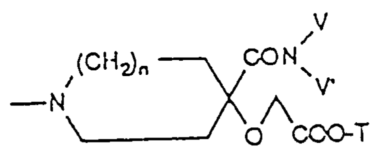

$Q^6$

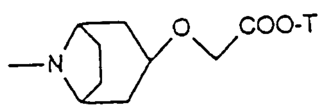

$Q^7$

ou

$-N(V')(CH_2)_v-C(V'',V''')CH_2OCH_2COO-T$ $Q^8$

ou bien, lorsque R' et R'' forment ensemble et avec l'atome d'azote et l'atome de carbone auxquels ils sont reliés, un cycle, un groupe de formule

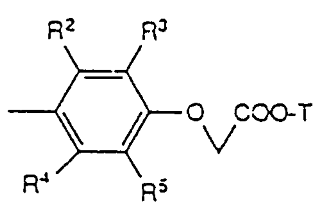

$Q^9$

n est égal à 0 ou 1,

v est un nombre entier allant de 0 à 3,
T et T' représentent l'hydrogène ou des groupes alkyle inférieurs ou phényl-alkyle inférieurs scindables dans l'organisme,
V à V''' représentent l'hydrogène ou des groupes alkyle inférieurs,
U et U' représentent l'hydrogène, des groupes alcanoyle en C1-C6 ou aroyle,
Ar représente un groupe aryle et
$R^2$ à $R^5$ représentent l'hydrogène, des groupes alkyle inférieurs, alcoxy inférieurs, des halogènes ou un groupe $-OCH_2COO-T'$, ou bien
$R^2$ et $R^3$ forment ensemble et avec le groupe phényle auquel ils sont reliés, un groupe 1-naphtyle,
l'expression "inférieur" s'appliquant à des groupes qui contiennent de 1 à 6 atomes de carbone,
ainsi que des hydrates ou solvates et sels acceptables pour l'usage pharmaceutique de ces composés,
caractérisé en ce que

a) dans un composé de formule

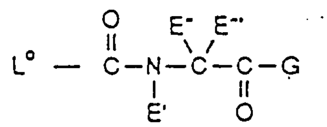

II

dans laquelle
$L^o$ représente un groupe de formule

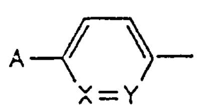

$L^{o1}$

ou

$R^{o1}-(CH_2)_t$ $L^{o2}$

A représentant un groupe amino ou guanidino éventuellement protégé,
$R^{o1}$ un groupe amidino ou guanidino éventuellement protégé,
E', E'', E''' et G ont respectivement les mêmes significations que R', R'', R''' et Q dans la formule I, sous réserve que, lorsque $R^{o1}$ représente un groupe amidino ou guanidino, ou bien lorsque A représente un groupe amidino ou guanidino, au moins un des groupes E', E'', E''' et G contient au moins un groupe ester d'acide carboxylique et/ou un groupe éther et/ou un groupe amino protégé,
on scinde un groupe éther ou un groupe amino, amidino ou guanidino protégé, ou un groupe ester d'acide carboxylique, ou bien

b) dans un nitrile de formule

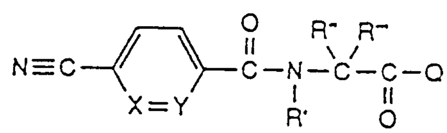

III

on convertit le groupe cyano en groupe amidino, ou bien

c) on fait réagir une amine de formule

R'-NHC(R'',R''')CO-Q IV

avec un acide de formule $L^1$-COOH ou l'un de ses dérivés réactifs, et

d) si on le désire, on soumet un groupe réactif contenu dans un composé de formule I à une modification fonctionnelle et

e) si on le désire, on convertit un composé de formule I en un sel acceptable pour l'usage pharmaceutique, ou bien un sel d'un composé de formule I en l'acide ou la base libre.

2. Procédé selon revendication 1, dans lequel L représente un groupe $L^1$, R', R'' et R''' représentent l'hydrogène ou des substituants à l'azote ou chaînes latérales usuels dans les acides alpha-aminocarboxyliques, les groupes hydroxy ou carboxy présents dans R', R'' et R''' pouvant être éthérifiés et respectivement estérifiés ou amidés, et T, dans le groupe Q, représente l'hydrogène ou un groupe alkyle inférieur scindable dans l'organisme.

3. Procédé selon revendication 1 ou 2, dans lequel L représente un groupe $L^1$, R représente un groupe amidino, X représente CH, Y représente CH ou N et Q représente un groupe $Q^1$, $Q^2$, $Q^4$, $Q^5$ ou $Q^9$.

4. Procédé selon revendication 1, 2 ou 3, dans lequel Q représente un groupe $Q^1$, et plus spécialement dans lequel n = 1 et T représente l'hydrogène ou un groupe méthyle, et -N(R')C(R'',R''')CO- représente l'un des groupes Gly, Ala, D-Ala, Val, Leu, Sar, Orn, Lys, Phg, 2-méthyl-Pro, Phe, Tyr, 3-iodo-Tyr, 3,5-diiodo-Tyr, Ser(Ac), Ser, Asp, Glu, Pro, 4-benzyloxy-Pro, 4-hydroxy-Pro, 2-pipéridylène-carbonyle, $NHCH(CH_2CH_2NH_2)CO$, Trp, Tyr(Me), Tyr(hexyle) et $O,N(Me)_2$-Tyr.

5. Procédé selon revendication 1, 2 ou 3, dans lequel Q représente un groupe $Q^2$ et plus spécialement dans lequel n = 1, T représente l'hydrogène et - N(R')C(R'',R''')CO- représente le groupe Ala.

6. Procédé selon revendication 1, 2 ou 3, dans lequel Q représente un groupe $Q^4$ et plus spécialement dans lequel n = 1, T représente l'hydrogène, U et U' l'hydrogène ou Ac et -N(R')C(R'',R''')CO- le groupe Ala.

7. Procédé selon revendication 1, 2 ou 3, dans lequel Q représente un groupe $Q^5$, et plus spécialement dans lequel n = 1, T représente l'hydrogène, Ar représente le groupe alpha,alpha,alpha-trifluoro-m-tolyle et -N(R')C(R'',R''')CO- le groupe Ala.

8. Procédé selon revendication 1, 2 ou 3, dans lequel Q représente un groupe $Q^9$, et plus spécialement dans lequel $R^2$ à $R^5$ représentent l'hydrogène, ou bien $R^2$ représente le groupe $-OCH_2COO$(H ou méthyle), T représente l'hydrogène ou un groupe méthyle et N(R')C(R'',R''')CO- le groupe Pro.

9. Procédé selon revendication 1 ou 3, pour la préparation d'un composé pris dans le groupe suivant
l'acide [[1-[N-(p-amidinobenzoyl)-L-alanyl]-4-pipéridinyl]oxy]acétique,
l'acide [[1-[N-[(5-amidino-2-pyridyl)carbonyl]-L-alanyl]-4-pipéridinyl]oxy]acétique,
l'acide [[1-[N-(p-amidinobenzoyl)-3-(4-hydroxy-3-iodophényl)-L-alanyl]-4-pipéridinyl]oxy]acétique,
l'acide [[1-[3-acétoxy-N-(p-amidinobenzoyl)-L-alanyl]-4-pipéridinyl]oxy]acétique,
l'acide [p-[[1-(p-amidinobenzoyl)-2-pyrrolidinyl]carbonyl]phénoxy]acétique,
l'acide [[1-[N-[(5-amidino-2-pyridyl)carbonyl]-L-tyrosyl]-4-pipéridinyl]oxy]acétique et plus spécialement
l'acide [[1-[N-(p-amidinobenzoyl)-L-tyrosyl]-4-pipéridinyl]oxy]acétique.

10. Procédé selon revendication 1, dans lequel Q représente un groupe $Q^3$, et plus spécialement dans lequel n = 0 et T représente l'hydrogène, ou bien un groupe $Q^7$, et plus spécialement dans lequel T représente l'hydrogène.

11. Procédé selon revendication 1, dans lequel Q représente un groupe $Q^8$, et plus spécialement dans lequel v = 1, T représente l'hydrogène ou un groupe butyle et V' à V''' représentent l'hydrogène.

12. Procédé selon revendication 1, dans lequel -N(R')C(R'',R''')CO- représente le groupe N-(méthoxyéthyl)-Gly.

13. Procédé selon revendication 1, pour la préparation d'un composé pris dans le groupe suivant :
l'acide (S)-1-[2-(5-amidinopyridine-2-ylcarbonylamino)-3-(4-méthoxyphényl)-propionyl]pipéridine-4-yloxy acétique,
le (S)-1-[2-(4-amidinobenzamido)-3-(4-méthoxyphényl)propionyl]pipéridine-4-yloxy acétate d'éthyle,
l'acide (S)-1-[2-(4-amidinobenzamido)-3-(4-méthoxyphényl)propionyl]pipéridine-4-yloxy acétique,
l'acide [1-[N-(4-amidinobenzoyl)-4'-hexyloxy-L-phénylalanyl]pipéridine-4-yloxy]acétique

14. Procédé de préparation de compositions pharmaceutiques, servant en particulier au traitement ou à la prophylaxie de maladies causées par la fixation de protéines adhésives sur les plaquettes du sang ou

par l'agglomération des plaquettes du sang et l'adhérence cellule-cellule, caractérisé en ce que l'on met un composé selon l'une des revendications 1 à 13, en tant que substance active, sous une forme galénique.

**15.** Utilisation d'un composé selon l'une des revendications 1 à 13, pour la préparation de médicaments servant au traitement ou à la prophylaxie de maladies causées par la fixation de protéines adhésives sur les plaquettes du sang ou par l'agglomération des plaquettes du sang et l'adhérence cellule-cellule, en particulier pour le traitement ou la prophylaxie des thrombus de plaquettes sanguines, des thromboses, de la congestion cérébrale, de l'infarctus cardiaque, des inflammations, de l'artériosclérose ou de l'ostéoporose ou en tant qu'agents anti-tumeurs ou agents de cicatrisation des blessures.

**16.** Composés de formule

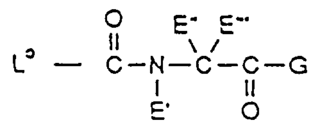

II

ou

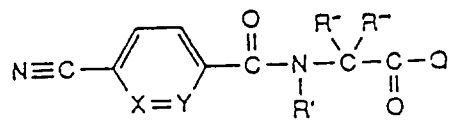

III

dans lesquels
$L^o$ représente un groupe de formule

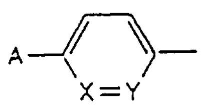

$L^{o1}$

ou

$R^{o1}$-$(CH_2)_t$ $L^{o2}$

A représentant un groupe amidino ou guanidino éventuellement protégé,
$R^{o1}$ un groupe amidino ou guanidino éventuellement protégé,
E', E'', E''' et G ont respectivement les mêmes significations que R', R'', R''' et Q dans la formule I, sous réserve que, lorsque $R^{o1}$ représente un groupe amidino ou guanidino, ou lorsque A représente un groupe amidino ou guanidino, au moins un des groupes E', E'', E''' et G contient au moins un groupe ester d'acide carboxylique et/ou un groupe éther et/ou un groupe amino protégé.